# EUROPÄISCHE PATENTANMELDUNG

(11) **EP 2 116 221 A1**
(43) Veröffentlichungstag der Anmeldung: **11.11.2009**
(21) Anmeldenummer: 09005861.1
(22) Anmeldetag: 28.04.2009
(51) Int. Cl.: A61K 8/31, A61Q 1/02, A61Q 17/04, A61Q 15/00

(54) **Kosmetische Zubereitungen enthaltend Kohlenwasserstoffe**

(30) Priorität: 07.05.2008 DE 102008022434
(71) Anmelder: Cognis IP Management GmbH, 40589 Düsseldorf (DE)
(72) Erfinder: Dierker, Markus, 40593 Düsseldorf (DE); Richter Joachim, SO45 3ZG Southampton (GB)

(57) **Zusammenfassung**

Die vorliegende Erfindung betrifft kosmetische und/oder pharmazeutische Zubereitungen, welche ausgewählte Kohlenwasserstoffe enthält.

## Beschreibung

Die vorliegende Erfindung betrifft kosmetische und/oder pharmazeutische Zubereitungen, enthaltend Kohlenwasserstoffe sowie die Verwendung von Kohlenwasserstoffen als Ölkörper und/oder Dispergiermittel.

### Stand der Technik

Sensorisch leichte Ölkörper, so genannte "light emollients", werden von der kosmetischen Industrie in einer Vielzahl von Formulierungen verwendet. Insbesondere für die dekorative Kosmetik bzw. in pflegenden Formulierungen werden so genannte "leichte" Komponenten eingesetzt. Bei diesen Komponenten kann es sich beispielsweise um flüchtige, cyclische Silikone (z.B. Cyclopentasiloxan oder Cyclomethicone) oder Kohlenwasserstoffe aus petrochemischen Prozessen handeln. Bei den zuletzt genannten Stoffen handelt es sich aufgrund ihrer Herstellung überwiegend um Gemische aus linearen, cyclischen und verzweigten Kohlenwasserstoffen, deren Flammpunkt durchaus unter 50 °C (wie z.B. beim Isododecan) liegen kann. Beispiele und anwendungstechnische Beschreibungen derartiger Formulierungen können in Standardwerken, wie zum Beispiel: **,**Handbook of Cosmetic Science and Technology', A. Barel, M. Paye, H. Maibach, Marcel Dekker Inc. 2001 nachgelesen werden. Aus toxikologischen, ökologischen bzw. sicherheitstechnischen Gründen besteht jedoch in Zukunft Bedarf nach alternativen Rohstoffen für derartige Formulierungsaufgaben.

Die Aufgabe der Erfindung bestand darin, alternative Rohstoffe zu finden, die ökologisch bzw. toxikologisch unbedenklich sind. Dabei war es insbesondere von Interesse Rohstoffe bereit zu stellen, welche ohne aufwendige Reinigungsschritte direkt in kosmetischen bzw. pharmazeutischen Zubereitungen eingesetzt werden können. Vorzugsweise sollten diese Rohstoffe auf Basis nachwachsender Rohstoffe erhältlich sein. Diese Rohstoffe sollten in typischen kosmetischen und/oder pharmazeutischen Formulierungen ohne anwendungstechnische Einschränkungen direkt eingesetzt werden können. Darüber hinaus sollten die Rohstoffe gegenüber den Kohlenwasserstoff Gemischen des Standes der Technik eine verbesserte Sensorik aufweisen, wünschenswert war auch, dass diese Rohstoffe eine bessere Hautverträglichheit aufweisen. Von besonderem Interesse war es, Rohstoffe bereit zu stellen, welche hinsichtlich ihrer sensorischen Einsatzmöglichkeiten mit Silikonölen, insbesondere mit niedrigviskosen Silikonölen, wie z.B. Dimethiconen vergleichbar sind. Wünschenswert war es insbesondere Rohstoffe zu Verfügung zu stellen, welche sich als Ersatzstoffe für Silikonöle eignen. Darüber hinaus war es von Interesse Rohstoffe bereit zu stellen, die gegenüber den Rohstoffen des Standes der Technik eine verbesserte CO₂-Bilan aufweisen.

Eine weitere Aufgabe bestand darin Rohstoffe zur Verfügung zu stellen, welche eine stabile Formulierung mit AP/Deo (=Antiperspirant/Desodorant) Wirkstoffen ermöglicht. Kosmetische Zubereitungen der Kategorie Antiperspirantien/Desodorantien, insbesondere in so genannten "Stick-Formulierungen" haben immer noch das Problem der unzureichenden Stabilität der kosmetischen Grundlage, insoweit daß sich geruchliche Veränderungen während der Lagerung ergeben. Eine weitere Aufgabe der Erfindung bestand daher darin, Rohstoffe zu Verfügung zu stellen, welche es ermöglichen antiperspirierende bzw. desodorierende Zubereitungen, insbesondere solche in "Stick-Formulierung" stabil bereit zu stellen. Diese Zubereitungen sollten insbesondere bei längerer Lagerung keine unerwünschten Geruchsentwicklungen zeigen. Eine weitere Aufgabe bestand darin Rohstoffe zu Verfügung zu stellen, welche einen sensorisch "leichten" Eindruck vermitteln, möglichst bei gleichzeigter verbesserter Hautverträglichkeit, insbesondere in Kombination mit UV-Lichtschutzfiltern sowie in Verbindung mit Selbstbräunern. Von besonderem Interesse ist die Bereitstellung von neuen Rohstoffen, die in Formulierungen der dekorativen Kosmetik einen sensorisch vorteilhaften Eindruck ermöglichen. An Formulierungen der dekorativen Kosmetik, wie beispielsweise Lippenstifte, Lidschatten, Mascara, Nagellack etc. werden aufgrund des Applikationsorts (hauptsächlich Gesicht und Hände) erhöhte Anforderungen an die Sensorik, insbesonderen die Flüchtigkeit gestellt, damit diese Produkte nicht den Eindruck von "Schwere" vermitteln. Desweiteren ist bei diesen Produkten eine gute Dispergierbarkeit von Pigmenten wünschenswert.

### Beschreibung der Erfindung

Ein Gegenstand der Erfindung betrifft kosmetische und/oder pharmazeutische Zubereitungen, enthaltend **0,1 bis 80 Gew.% eines Kohlenwasserstoffs**, ausgewählt aus der Gruppe bestehend aus C7, C9, C11, C13, C15, C17, C19, C21 und C23 Kohlenwasserstoffen.

Ein bevorzugter Gegenstand der Erfindung betrifft kosmetische und/oder pharmazeutische Zubereitungen, enthaltend **0,1 bis 80 Gew.% eines Kohlenwasserstoffs**, ausgewählt aus der Gruppe bestehend aus C11, C13, C15, C17, C19, C21 und C23 Kohlenwasserstoffen.

Die Gew.-% beziehen sich auf das Gesamtgewicht der kosmetischen und/oder pharmazeutischen Zubereitung.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

Der Begriff "CX-Kohlenwasserstoff" umfasst Kohlenwasserstoffe mit einer C-Zahl von X (wobei X eine ganze Zahl darstellt), so umfasst beispielsweise der Begriff C11-Kohlenwasserstoff alle Kohlenwasserstoffe mit einer C-Zahl von 11. Der Begriff "Kohlenstoff Zahl" oder "C-Zahl" umfasst alle im Kohlenwasserstoff vorhandenen C-Atome. Er beträgt somit z.B. für Undecan = 11 oder für Tridecan = 13.

Die Erfindung umfasst sowohl kosmetische und/oder pharmazeutische Zubereitungen, die nur einen der genannten Kohlenwasserstoffe enhält als auch beliebige Mischungen der genannten Kohlenwasserstoffe.

In einer bevorzugten Ausführungsform der Erfindung enthalten die kosmetischen und/oder pharmazeutischen Zubereitungen lineare Kohlenwasserstoffe, ausgewählt aus der Gruppe bestehend aus C7, C9, C11, C13, C15, C17, C19, C21 und C23 Kohlenwasserstoffe.

In einer bevorzugten Ausführungsform der Erfindung enthalten die kosmetischen und/oder pharmazeutischen Zubereitungen gesättige Kohlenwasserstoffe, ausgewählt aus der Gruppe bestehend aus C7, C9, C11, C13, C15, C17, C19, C21 und C23 Kohlenwasserstoffe.

In einer bevorzugten Ausführungsform der Erfindung enthalten die kosmetischen und/oder pharmazeutischen Zubereitungen gesättige, lineare Kohlenwasserstoffe, ausgewählt aus der Gruppe bestehend aus C7, C9, C11, C13, C15, C17, C19, C21 und C23 Kohlenwasserstoffe.

In einer bevorzugten Ausführungsform der Erfindung enthalten die kosmetischen und/oder pharmazeutischen Zubereitungen, Kohlenwasserstoffe, ausgewählt aus der Gruppe bestehend aus n-Heptan, n-Nonan, n-Undecan, n-Tridecan, n-Pentadecan, n-Heptadecan, n-Nonadecan, n-Henicosan und n-Tricosan.

In einer besonders bevorzugten Ausführungsform der Erfindung enthalten die kosmetischen und/oder pharmazeutischen Zubereitungen, Kohlenwasserstoffe, ausgewählt aus der Gruppe bestehend aus n-Undecan, n-Tridecan, n-Pentadecan, n-Heptadecan, n-Nonadecan, n-Henicosan und n-Tricosan.

Folgende Tabelle führt wichtige physikochemische Eigenschaften der bevorzugt zu verwendenden Kohlenwasserstoffe auf:

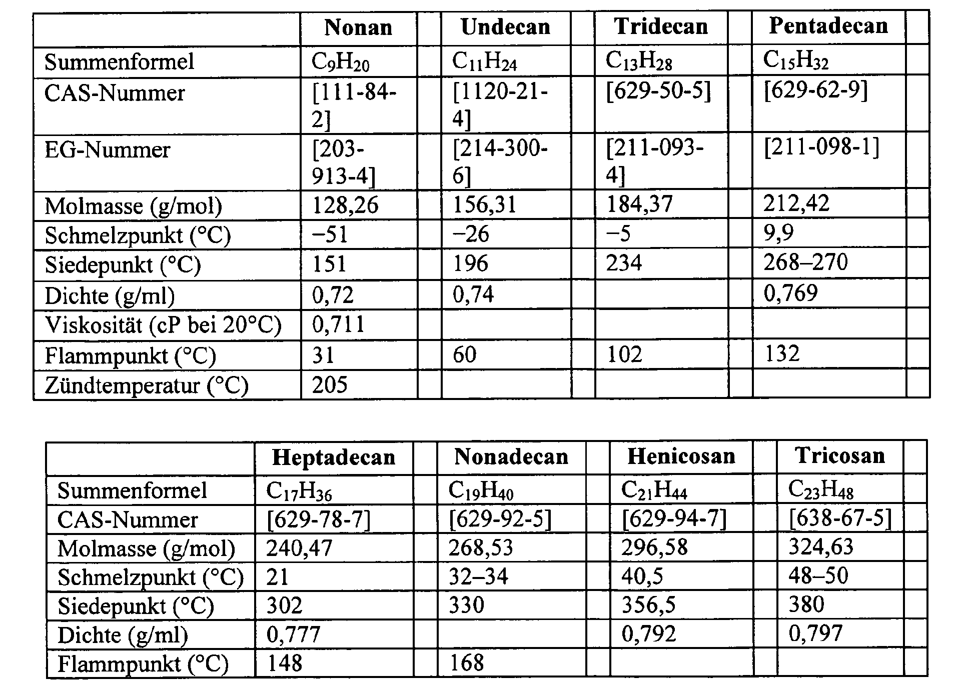

Die kosmetischen und/oder pharmazeutischen Zubereitungen können weitere Kohlenwasserstoffe, wie beispielsweise Paraffine enthalten. Bevorzugt ist es, dass die kosmetischen und/oder pharmazeutischen Zubereitungen mehr als 50 Gew.-%, insbesondere mehr als 60 Gew.-%, insbesondere mehr als 70 Gew.-%, insbesondere mehr als 80 Gew.-% insbesondere mehr als 85 Gew.-% insbesondere mehr als 90 Gew.-% insbesondere mehr als 95 Gew.-% Kohlenwasserstoffe, ausgewählt aus der Gruppe bestehend aus C7, C9, C11, C13, C15, C17, C19, C21 und C23 Kohlenwasserstoffe -bezogen auf die Summe der in der Zubereitung enthaltenen Kohlenwasserstoffe- enthalten.

Die Bezugsgröße "Summe der Kohlenwasserstoffe" umfasst alle in der kosmetischen und/oder pharmazeutischen Zubereitung enthaltenen Kohlenwasserstoffe, unabhängig von ihrer Kohlenstoffzahl.

Ein weiterer Gegenstand der Erfindung betrifft ein Verfahren zur Herstellung einer kosmetischen und/oder pharmazeutischen Zubereitung, wobei ein Kohlenwasserstoff, ausgewählt aus der Gruppe bestehend aus C7, C9, C11, C13, C15, C17, C19, C21 und C23 Kohlenwasserstoffen zu einem kosmetisch und/oder pharmazeutisch geeigneten Träger gegeben wird.

Ein weiterer Gegenstand der Erfindung betrifft die Verwendung von Kohlenwasserstoffen, ausgewählt aus der Gruppe bestehend aus C7, C9, C11, C13, C15, C17, C19, C21 und C23 Kohlenwasserstoffen in kosmetischen und/oder pharmazeutischen Zubereitungen, insbesondere als Ölkörper und/oder Dispergiermittel.

Ein weiterer Gegenstand der Erfindung betrifft die Verwendung von Kohlenwasserstoffen, ausgewählt aus der Gruppe bestehend aus C7, C9, C11, C13, C15, C17, C19, C21 und C23 Kohlenwasserstoffen in kosmetischen Zubereitungen zur Pflege von Haut und/oder Haaren. Ein weiterer Gegenstand der Erfindung betrifft die Verwendung von Kohlenwasserstoffen, ausgewählt aus der Gruppe bestehend aus C7, C9, C11, C13, C15, C17, C19, C21 und C23 Kohlenwasserstoffen in kosmetischen Zubereitungen zum Sonnenschutz.

Ein weiterer Gegenstand der Erfindung betrifft die Verwendung von Kohlenwasserstoffen, ausgewählt aus der Gruppe bestehend aus C7, C9, C11, C13, C15, C17, C19, C21 und C23 Kohlenwasserstoffen in Zubereitungen der dekorativen Kosmetik, wie beispielsweise Lippenstifte, Lip Gloss, Lidschatten, Wimperntuschen (Mascara), Lidstifte (Kajal), Nagellack sowie in Make-up Formulierungen jeder Art (Puder, Creme, Foundation, Abdeckstifte etc.).

Ein weiterer Gegenstand der Erfindung betrifft die Verwendung von Kohlenwasserstoffen, ausgewählt aus der Gruppe bestehend aus C7, C9, C11, C13, C15, C17, C19, C21 und C23 Kohlenwasserstoffen in Zubereitungen zur Reinigung von Haut und/oder Haaren, wie beispielsweise Shampoos, Duschgels, Badezusätze, Conditioner etc.

Ein weiterer Gegenstand der Erfindung betrifft die Verwendung von Kohlenwasserstoffen, ausgewählt aus der Gruppe bestehend aus C7, C9, C11, C13, C15, C17, C19, C21 und C23 Kohlenwasserstoffen zur Herstellung von feinteiligen Emulsionen, so z.B. Nanoemulsionen, Microemulsionen oder PIT Emulsionen. In solchen feinteiligen Emulsionen liegen die Öltröpfchen in der Regel im Bereich von 10 bis 1000 nm, vorzugsweise 100 bis 500 nm Durchmesser vor. Diese werden nach dem Fachmann bekannten Verfahren hergestellt, für PIT Emulsionen beispielsweise in Parfümerie und Kosmetik, 77. Jahrgang, Nr. 4/96, S. 250 - 254 von Wadle **et al.** beschrieben.

### Herstellung der Kohlenwasserstoffe

Die erfindungsgemäßen Kohlenwasserstoffe können beispielsweise durch reduktive Demethylierung nach dem Fachmann bekannten Methoden erhalten werden. Besonders geeignet zur Herstellung der erfindungsgemäßen Kohlenwasserstoffe ist das in der internationalen Anmeldung PCT/EP2006/011647 (Cognis) beschrieben Verfahren der reduktiven Dehydroxymethylierung ausgehend von Fettalkoholen pflanzlichen Ursprungs. Dabei können beispielsweise C12 oder C14 Fettalkohole dem beschriebenen Verfahren unterzogen werden und die so erhaltenen C11 oder C 13 Kohlenwasserstoffe zu den erfindungsgemäßen Zubereitungen gemischt werden.

Mit den erfindungsgemäßen Kohlenwasserstoffen werden leichte, stabile kosmetische und/oder pharmazeutische Zubereitungen erhalten, dies ist insbesondere dann der Fall, wenn die Kohlenwasserstoffe zusammen mit Antiperspirant-/Desodorant-Wirkstoffen eingesetzt werden.

Ein Gegenstand der Erfindung betrifft daher kosmetische und/oder pharmazeutische Zubereitungen, enthaltend **0,1 bis 80 Gew.% Kohlenwasserstoffe,** ausgewählt aus der Gruppe bestehend aus C7, C9, C11, C13, C15, C17, C19, C21 und C23 Kohlenwasserstoffen und mindestens einen Antiperspirant-/ Desodorant-Wirkstoff.

Erfindungsgemäß sind als **Antiperspirant/Desodorant Wirkstoff** alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.
Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakterizide bzw. bakteriostatische Wirkstoffe in Mengen von 0,01 bis 5 und vorzugsweise 0,1 bis 2 Gew.-% - bezogen auf das Gesamtgewicht der kosmetischen und/oder pharmazeutischen Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Mit den erfindungsgemäßen Kohlenwasserstoffen werden sensorisch leichte, kosmetische und/oder pharmazeutische Zubereitungen erhalten, dies ist insbesondere dann der Fall, wenn die Kohlenwasserstoffe zusammen mit UV-Lichtschutzfiltern eingesetzt werden.

Ein Gegenstand der Erfindung betrifft daher kosmetische und/oder pharmazeutische Zubereitungen, enthaltend **0,1 bis 80 Gew.% Kohlenwasserstoffe,** ausgewählt aus der Gruppe bestehend aus C7, C9, C11, C13, C15, C17, C19, C21 und C23 Kohlenwasserstoffen und mindestens einen **UV-Lichtschutzfilter.**

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- bornan-2-on-methylsulfat (Mexoryl SO)
➢ 3,3'-(1,4-Phenylendimethin)-bis (7,7-dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung **Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 **sowie** Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Eine bevorzugte Ausführungsform der Erfindung betrifft kosmetischen und/oder pharmazeutische Zubereitungen, enthaltend **0,1 bis 80 Gew.% Kohlenwasserstoffe,** ausgewählt aus der Gruppe bestehend aus C7, C9, C11, C13, C15, C17, C19, C21 und C23 Kohlenwasserstoffen und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzylidenbornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethylbenzalmalonate und ihren Mischungen.

Diese UV-Lichtschutzfilter sind beispielsweise unter den folgenden Handelsnamen kommerziell erhältlich:
NeoHeliopan®MBC (INCI: 4-Methylbenzylidene Camphor; Hersteller: Symrise); NeoHeliopan® BB (INCI: Benzophenone-3, Hersteller: Symrise); Parsol® 1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung - enthalten.

Ein Gegenstand der Erfindung betrifft kosmetische und/oder pharmazeutische Zubereitungen, enthaltend **0,1 bis 80 Gew.% Kohlenwasserstoffe**, ausgewählt aus der Gruppe bestehend aus C7, C9, C11, C13, C15, C17, C19, C21 und C23 Kohlenwasserstoffen und mindestens einen **Selbstbräuner.**

Als Selbstbräuner sind Substanzen zu verstehen, welche eine Bräunung der Haut verursachen. Beispielsweise seien Dihydroxyaceton, Erythrulose sowie alpha, beta-ungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose, Glycerolaldehyd, Alloxan, Hydroxymethylglyoxal, gamma-Dialdehyd, 6-Aldo-D-Fructose, Ninhydrin und meso-Weinsäuredialdehyd. **Als Selbstbräuner eignen sich insbesondere** Dihydroxyaceton und/oder Erythrulose.

Als besonders vorteilhaft haben sich Mischungen der o. g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen.

Die erfindungsgemäßen Zubereitungen enthalten die Selbstbräuner üblicherweise in Konzentrationen von 1 bis 10, insbesondere von 2 bis 5 Gew.-% -bezogen auf das Gesamtgewicht der kosmetischen und/oder pharmazeutischen Zubereitung.

Eine besonders bevorzugte Ausführungsform der Erfindung betrifft kosmetische und/oder pharmazeutische Zubereitungen, enthaltend **0,1 bis 80 Gew.% Kohlenwasserstoffe,** ausgewählt aus der Gruppe bestehend aus C7, C9, C11, C13, C15, C17, C19, C21 und C23 Kohlenwasserstoffen und mindestens einen **UV-Lichtschutzfilter** und mindestens einen **Selbstbräuner.**

Die erfindungsgemäßen kosmetischen und/oder pharmazeutischen Zubereitungen können beispielsweise als O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen kosmetischen und/oder pharmazeutischen Zubereitungen, eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich u.a. auch in Zubereitungen zur Haarpflege, Haarreinigung oder Haarfärbung einsetzen. Die erfindungsgemäßen kosmetischen und/oder pharmazeutischen Zubereitungen zeichnen sich durch positives sensorisches Verhalten bei Applikation aus.

Die erfindungsgemäßen kosmetischen und/oder pharmazeutischen Zubereitungen eignen sich als Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

Ein Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe bestehend aus C7, C9, C11, C13, C15, C17, C19, C21 und C23 Kohlenwasserstoffen und mindestens ein **Pigment und/oder einen Farbstoff.**

Der Begriff **Pigment** umfasst Partikel jeder Form, die weiß oder gefärbt, organisch oder anorganisch sind, in den Zubereitungen nicht löslich sind, und dem Zweck dienen die Zubereitung zu färben. In einer bevorzugten Ausführungsform werden anorganische Pigmente verwendet, besonders bevorzugt sind Metalloxide.

Als **anorganische Pigmente** seien exemplarisch genannt: Titandioxid, optional oberflächenbeschichtet, Zirkonium oder Ceriumoxide und Zink-, Eisen- (Schwarz, Gelb oder Rot) und Chrom-oxide, Manganviolett, Ultramarine Blau, Chromhydroate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugweise aus den Metalloxiden. In einer bevorzugten Ausführungsform ist das Pigment ausgewählt aus der Gruppe bestehend aus Titandioxid, Zinkoxid, Eisenoxid und Mischungen daraus.

Die Pigmente können sowohl einzeln als auch in Mischungen vorliegen.
Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen koennen. Unter den Farbpigmenten sind Eisenoxide besonders bevorzugt.
Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäss vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäss bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemässen Zubereitungen vorteilhaft auch **organische Farbpigmente** enthalten, d. h. organische Farbstoffe, welche in der Zubereitung praktisch unlöslich sind. Nach **DIN 55944: 1990-04** können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weisspigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.
Die erfindungsgemäßen Zubereitungen enthalten üblicherweise 0,1 bis 40 Gew.-% Pigmente - bezogen auf das Gesamtgewicht der kosmetischen und/oder pharmazeutischen Zubereitung.

Es ist ferner vorteilhaft im Sinne der vorliegenden Erfindung, wenn die erfindungsgemässe Zubereitung einen oder mehrere **Farbstoffe** enthält.

Die Farbstoffe können sowohl synthetischen als auch natürlichen Ursprungs sein. Eine Liste von geeigneten Farbstoffen findet sich in EP 1 371 359 A2, S.8, Z. 25-57, S.9 und S.10 sowie S.11, Z. 1 bis 54, auf welche hiermit explizit Bezug genommen wird.

Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen und/oder pharmazeutischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen und/oder pharmazeutischen Zubereitung.

Als Farbstoffe und Pigmente eignen sich insbesondere die gemäß Annex IV der Kommissions Direktive zugelassenen Farbstoffe und Pigmente (in der Fassung: **Commission Directive 2007/22/EC of 17 April 2007 amending Council Directive 76/768/EEC,** concerning cosmetic products, for the purposes of adapting Annexes IV and VI thereto to technical progress) zugelassen Stoffe, auf die hiermit explizit Bezug genommen wird.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die Kohlenwasserstoff Gemische lassen sich auch in tensidhaltigen Formulierungen wie z. B. Schaum- und Duschbädern, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Ein weiterer Gegenstand der Erfindung betrifft kosmetische und/oder pharmazeutische Zubereitungen, enthaltend **0,1 bis 80 Gew.-% Kohlenwasserstoffe** ausgewählt aus der Gruppe bestehend aus C7, C9, C11, C13, C15, C17, C19, C21 und C23 Kohlenwasserstoffen und mindestens einen Emulgator und/oder ein Tensid und/oder eine Wachskomponente und/oder ein Polymer und/oder einen weiteren Ölkörper.

### Emulgator

In einer Ausführungsform der Erfindung enthalten die erfindungsgemäßen Zubereitungen mindestens einen Emulgator.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe bestehend aus C7, C9, C11, C13, C15, C17, C19, C21 und C23 Kohlenwasserstoffen und mindestens einen Emulgator.

Die erfindungsgemäßen Zubereitungen enthalten den/die Emulgator(en) üblicherweise in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zubereitung.

Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Grösse und Stärke der hydrophilen und der lipophilen Gruppen eines Emulgators aus. Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Öiphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Lauryl Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/Polyhydroxystearate/Sebacate (Isolan^{®} GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913**,** aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 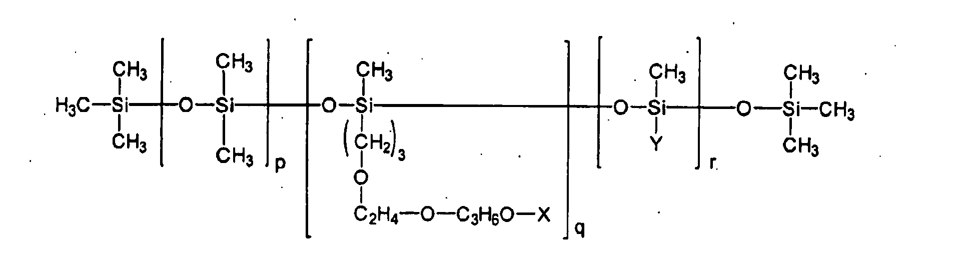 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden. Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird. Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Goldschmidt unter der Warenbezeichnung ABIL®EM 97 und ABIL®WE 09 verkauft wird. Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Ein weiterer vorteilhafter Silikonemulgator ist Octyl Dimethicon Ethoxy Glucosid der Firma Wacker.

Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL® EM 90 Evonik Goldschmidt), DC5200 Formulation Aid (Dow Corning)] sowie beliebige Mischungen aus beiden Emulgatoren.

### Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekuelteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächen aktive Substanzen verstanden, die einen HLB-Wert von größer 20 aufweisen.

Ein weiterer Gegenstand der Erfindung betrifft daher kosmetische und/oder pharmazeutische Zubereitungen, enthaltend **0,1 bis 80 Gew.-% Kohlenwasserstoffe**, ausgewählt aus der Gruppe bestehend aus C7, C9, C11, C13, C15, C17, C19, C21 und C23 Kohlenwasserstoffen und mindestens **ein Tensid.**

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zubereitungen enthalten den/die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-% bezogen auf das Gesamtgewicht der Zubereitung.

Typische Beispiele für **nichtionische Tenside** sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder - SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind **ampholytische Tenside**. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Wachskomponente

In einer Ausführungsform der Erfindung enthalten die erfindungsgemäßen Zubereitungen mindestens eine Wachskomponente.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe bestehend aus C7, C9, C11, C13, C15, C17, C19, C21 und C23 Kohlenwasserstoffen und mindestens **eine Wachskomponente.**

Die erfindungsgemäßen Zubereitungen enthalten den/die Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben.

Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C 12-C 18-Mono-, Di- und Triglyceriden), sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C 14-C22-Fettalkohole, die beispielsweise von der Cognis Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin **Perlglanzwachse**. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Polymere

In einer Ausführungsform der Erfindung enthalten die erfindungsgemäßen Zubereitungen mindestens ein Polymer.

Ein weiterer Gegenstand der Erfindung betrifft daher kosmetische und/oder pharmazeutische Zubereitungen, enthaltend **0,1 bis 80 Gew.-% Kohlenwasserstoffe**, ausgewählt aus der Gruppe bestehend aus C7, C9, C11, C13, C15, C17, C19, C21 und C23 Kohlenwasserstoffen und mindestens **ein Polymer.**

Die erfindungsgemäßen Zubereitungen enthalten das/die Polymere(n) üblicherweise in einer Menge von 0 bis 20 Gew.%, vorzugsweise 0,1 bis 15 Gew.-% und insbesondere 0,1 bis 10 Gew.-% bezogen auf das Gesamtgewicht der Zubereitung.

Geeignete **kationische Polymere** sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Ebenso geeignet sind sogenannte **quaternäre Polymere**, z.B. mit der INCI - Bezeichnung Polyquaternium-37, die der folgenden allgemeinen Formel entsprechen:

Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch Co-polymere mit anionischen, weiteren kationischen oder 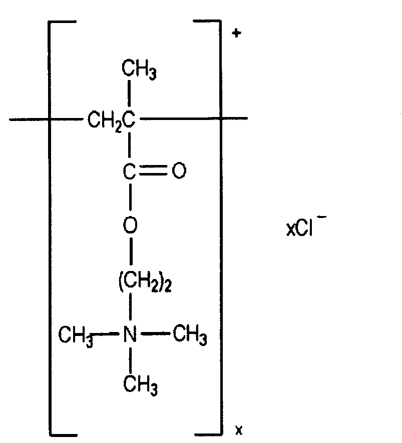 ungeladenen Monomeren erfindungsgemäß eingesetzt werden, insbesondere solche, die neben den genannten Alkylaminoalkyl (meth)acrylat oder -(meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten. Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

### Ölkörper

In einer Ausführungsform der Erfindung enthalten die erfindungsgemäßen Zubereitungen mindestens einen Ölkörper. Üblicherweise enthalten die erfindungsgemäßen Zubereitungen den oder die Kohlenwasserstoffe als Ölkörper. In der hier als bevorzugte genannten Ausführungsform enthalten die Zubereitungen somit einen von dem erfindungsgemäßen Kohlenstoffen verschiedenen Ölkörper, auch als "weiterer Ölkörper" bezeichnet.

Ein weiterer Gegenstand der Erfindung betrifft daher kosmetische und/oder pharmazeutische Zubereitungen, enthaltend **1 bis 80 Gew.-% Kohlenwasserstoffe,** ausgewählt aus der Gruppe bestehend aus C7, C9, C11, C13, C15, C17, C19, C21 und C23 Kohlenwasserstoffen mindestens **einen (weiteren) Ölkörper.**

Die Ölkörper (erfindungsgemäße Kohlenwasserstoffe plus weitere Ölkörper) sind üblicherweise in einer Gesamtmenge von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% bezogen auf das Gesamtgewicht der Zubereitung enthalten.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen..

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 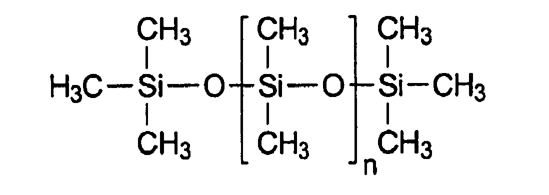 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo- trisiloxan, Polydimethylsiloxan, Poly (methylphenylsiloxan). Erfindungsgemäß besonders bevorzugte Silikone sind Dimethicon und Cyclomethicon.

Die erfindungsgemäßen Zubereitungen können weiterhin biogenen Wirkstoffe, Insekten-Repellentien, Tyrosinase Inhibitoren, Konservierungsmittel, Parfümöle, Überfettungsmittel, Stabilitsatoren und/oder Hydrotrope enthalten.

Ein weiterer Gegenstand der Erfindung betrifft daher kosmetische und/oder pharmazeutische Zubereitungen, enthaltend **0,1 bis 80 Gew.-% Kohlenwasserstoffe,** ausgewählt aus der Gruppe bestehend aus C7, C9, C11, C13, C15, C17, C19, C21 und C23 Kohlenwasserstoffen und **mindestens einen biogenen Wirkstoff, Insekten-Repellent, Tyrosinase Inhibitor, Konservierungsmittel, Parfümöl, Stabilisator und/oder Hydrotrop.**
Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Tyrosinhinbitoren,** die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe. Weiterhin eignen sich als Konservierungsmittel die in WO 07/048757 beschriebenen 1,2 Alkandiole mit 5 bis 8 C-Atomen.

Als Konservierungsmittel eignen sich insbesondere die gemäß Annex VI der Kommissions Direktive (in der Fassung Commission Directive 2007/22/EC of 17 April 2007 amending Council Directive 76/768/EEC, concerning cosmetic products, for the purposes of adapting Annexes IV and VI thereto to technical progress) zugelassen Stoffe, auf die hier explizit Bezug genommen wird.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### Herstellbeispiel 1:

### 1 a) Herstellung von Tridecan aus 1-Tetradecanol

1000 g 1-Tetradecanol (4,7 mol; Lorol C 14 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240 °C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Es ergab sich eine Auswaage von 845 g Reaktionsprodukt.

Eine GC-Analyse ergibt folgende Zusammensetzung: 89,0 % Tridecan, 2,1 % Tetradecan, 4,1 % 1-Tetradecanol, 4,2 % Dimere Reaktionsprodukte. Dieses Reaktionsprodukt wurde in einer Destillation zum reinen Tridecan fraktioniert und anschließend mit Stickstoff desodoriert. Man erhält ein farbloses, dünnflüssiges und geruchsarmes Produkt.

### 1b) Herstellung von Undecan aus 1-Dodecanol

1000 g 1-Dodecanol (5,4 mol; Lorol C 12 der Fa. Cognis) wurden in einem rührbaren Druckbehälter mit 10 g eines Nickelkatalysators (Ni-5249 P der Fa. Engelhard; Ni-Gehalt = 63 Gew%) vorgelegt und auf 240°C aufgeheizt. Anschließend wurde über einen Zeitraum von 8 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt und abgelassen und filtriert. Es ergab sich eine Auswaage von 835g Reaktionsprodukt.

Eine GC-Analyse ergibt folgende Zusammensetzung: 68,4 % Undecan, 0,6 % Dodecan, 21,7 % 1-Dodecanol, 7,2 % Dimere Reaktionsprodukte. Dieses Reaktionsprodukt wurde destilliert, um das Undecan rein zu erhalten. Dieses wurde anschließend mit Stickstoff desodoriert. Man erhält ein farbloses, dünnflüssiges und geruchsarmes Produkt.

### Herstellbeispiel 2:

### 2a) Herstellung von Pentadecan aus 1-Hexadecanol

1000 g 1-Hexadecanol (Lorol C 16 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240°C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Dieses Reaktionsprodukt wurde in einer Destillation zum reinen Pentadecan fraktioniert und anschließend mit Stickstoff desodoriert. Man erhält ein farbloses, flüssiges und geruchsarmes Produkt.

### 2b) Herstellung von Heptadecan aus 1-Octadecanol

1000 g 1-Octadecanol (Lorol C 18 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240°C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Dieses Reaktionsprodukt wurde in einer Destillation zum reinen Heptadecan fraktioniert und anschließend mit Stickstoff desodoriert. Man erhält ein farbloses, geruchsarmes Produkt, das bei 40°C flüssig ist.

### Rezepturbeispiele

Die nachfolgenden Rezepturbeispiele wurden jeweils erhalten, in dem der gemäß Herstellbeispiel 1a oder 1b (Undecan oder Tridecan) oder Herstellbeispiel 2a oder 2b erhaltene Kohlenwasserstoffs (Pentadecan oder Heptadecan) in die angegebenen kosmetischen Zubereitungen eingearbeitet wurde.

### Allzweck Creme (W/O)

| Phase | Komponente/ Handelsname | INCI | Gew.% |
|---|---|---|---|
| I. | DEHYMULS® E | Dicocoyl Pentaerhytithyl Distearyl Citrate (and) Sorbitan Sesquioleate (and) Cera Alba (Beeswax) (and) Aluminum Stearate | 3,00 |
| | DEHYMULS® PGPH | Polyglyceryl 2 Dipolyhydroxystearate | 2,00 |
| | CETIOL® OE | Dicaprylyl Ether | 5,00 |
| | CETIOL® 868 | Ethylhexyl Stearate | 5,00 |
| | MYRITOL® 331 | Cocoglycerides | 1,00 |
| | Pentadecan oder Heptadecan | | 6,00 |
| II. | Glycerin 86% | | 5,00 |
| | MgSO₄ x 7H₂O | | 1,00 |
| | Wasser, deionisiert | | 72,00 |
| III. | Konservierungsmittel | | q.s. |

### Herstellung:

Die Komponenten der Phase I wurden bei 80 bis 85 °C geschmolzen und zur Homogenität verrührt. Die Komponenten der Phase II wurden auf 80 bis 85 °C erhitzt und langsam, unter Rühren zur Phase I zugegeben. Es wurde für weitere 5 Minuten bei dieser Temperatur gerührt. Danach wurde die Emulsion unter Rühren abgekühlt und bei 65 bis 55°C homogenisiert. Sobald die Emulsion homogen erscheint, wurde unter Rühren weiter auf 30°C abgekühlt. Danach wurden Komponenten der Phase III zugegeben und erneut gerührt.

### Balsam zur Befeuchtung und zum Schutz der Lippen

| Phase | Komponente Handelsname | INCI | Gew.-% |
|---|---|---|---|
| I. | Cerilla Raffinée G* | Candelilla (Euphorbia Cerifera) Wax | 7,53 |
| | CUTINA® LM conc. | Polyglyceryl-2 Dipolyhydroxystearate and Octyldodecanol and Copernicia Cerifera (Carnauba) Wax and Euphorbia Cerifera (Candelilla) Wax and Beeswax and Cetearyl Glucoside and Cetearyl Alcohol | 6,57 |
| | Colophane claire type Y | Rosin | 1,89 |
| | Cerauba T1* | Carnauba(Copernica Cerifera)Wax | 1,86 |
| | Cerabeil blanche 1 * | Beeswax | 5.31 |
| | Pentadecan oder Heptadecan | | 15.57 |
| | EUTANOL® G | Octyldodecanol | 21,71 |
| | Crodamol ML(Croda) | Myristyl Lactate | 1,13 |
| | ELESTAB®366 | | 0.43 |
| II. | Castor oil | Castor Oil | 35.00 |
| III. | IRWINOL® LS 9319 | African wild mango butter | 3.00 |

erhältlich von Lambert- Riviere (France)

Herstellung: Phase I wurde bei 85°C geschmolzen, Phase II wurde zugefügt und die Temperatur auf 80°C gehalten. Phase III wurde kurz vor dem Einfüllen in die Gießform (welche mit Dimethicon 50 cts befeuchtet und auf 40 °C vorgeheizt war) zugegeben. Die Masse wurde in die Gießform gegeben und auf 40 °C abgekühlt. Die Gießformen wurde im Gefrierschrank auf nahe 0 °C abgekühlt.

### Styling Wachs

| Phase | Komponente Handelsname | INCI | Gew.-% |
|---|---|---|---|
| I. | CUTINA® MD | Glyceryl Stearate | 47,0 |
| | COMPERLAN® 100 | Cocamide MEA | 2,50 |
| | CUTINA® HR Powder | Hydrogenated Castor Oil | 2,50 |
| | PLANTACARE® 1200 UP | Lauryl Glucoside | 5,00 |
| | LANETTE® O | Cetearyl Alcohol | 7,00 |
| | CUTINA® CP | Cetyl Palmitate | 7,00 |
| | EUMULGIN® O 20 | Oleth-20 | 5,00 |
| | Pentadecan oder Heptadecan | | 23,5 |
| | Wacker Siliconoil AK 350 | Dimethicone | 0,50 |

Die Herstellung erfolgt durch Erhitzen aller Komponenten auf 80 °C und Homogenisierung.

### Feuchtigkeitsspendende Körpermilch

| Phase | Komponente Handelsname | INCI | Gew.-% |
|---|---|---|---|
| | EMULGADE® CM | Cetearyl Isononanoate (and) Ceteareth-20 (and) Cetearyl Alcohol (and)Glyceryl Stearate (and) Glycerin (and) Ceteareth | 5.0 |
| | EUMULGIN® VL 75 | Lauryl Glucoside (and) Polyglyceryl-2 Dipolyhydroxystearate (and) Glycerin | 2.0 |
| | CETIOL® OE | Dicaprylyl Ether | 4.0 |
| | CETIOL® J 600 | Oleyl Erucate | 1.0 |
| | ISOPROPYLMYRISTATE | Isopropyl Myristate | 7.0 |
| | Pentadecan oder Heptadecan | | 7.0 |
| II. | Wasser, deionisiert | | ad 100 |
| III. | Cosmedia SP | Sodium Polyacrylate | 0.4 |
| IV. | HISPAGEL® 200 | Glycerin (and) Glyceryl Polyacrylate | 20.0 |
| V. | Konservierungsmittel, Parfum | | q.s. |
| | pH 5.5 | | |

Die Herstellung erfolgte durch Mischen von Phase I und Wasser bei Raumtemperatur unter Rühren. Dann wurde Phase III zugefügt und solange gerührt bis eine homogene, gequollene Mischung vorlag. Dann wurde Phase IV zugefügt, gefolgt von Phase V, danach wurde der pH Wert eingestellt.

### O/W Soft Creme

| Phase | Komponente Handelsname | INCI | Gew.-% |
|---|---|---|---|
| I. | EMULGADE® SE-PF | Glyceryl Stearate (and) Ceteareth-20 (and) Ceteareth-12 (and) Stearyl Alcohol (and) Ceteareth-20 (and) Distearyl Ether | 6.0 |
| | LANETTE® O | Cetearyl Alcohol | 1.0 |
| | CUTINA® MD | Glyceryl Stearate | 2.0 |
| | CETIOL® MM | Myristyl Myristate | 2.0 |
| | Pentadecan oder Heptadecan | | 8.0 |
| | Jojoba Oil | Simmondsia Chinensis (jojoba) Seed Oil | 2.0 |
| | COPHEROL® 1250 | Tocopheryl Acetate | 0.5 |
| | | Dimethicone | 0.5 |
| | | Cyclomethicone | 3.0 |
| II. | Wasser | Aqua | ad 100 |
| | | Propylene Glycol | 3.0 |
| III. | HISPAGEL® 200 | Glycerin (and) Glyceryl Polyacrylate | 15.0 |
| IV. | Konservierungsmittel | | q.s. |
| | pH 5.5-6.5 | | |

Diese Creme wurde hergestellt, indem Phase I auf 80°C erhitzt wurde, Phase II wurde ebenfalls auf 80°C erhitzt und zu Phase I unter Rühren hinzugefügt. Diese Mischung wurde unter Rühren abgekühlt und bei ca. 55°C mit einem geeigneten Dispersionsgerät (z.B. Ultra Turrax) homogenisiert. Danach wurde Phase III unter kontinuierlichem Rühren hinzugefügt, Phase IV wurde zugegeben und der pH-Wert eingestellt.

### W/O Creme

| Phase | Komponente / Handelsname | INCI | Gew.-% |
|---|---|---|---|
| I. | MONOMULS® 90 O 18 | Glyceryl Oleate | 2,00 |
| | LAMEFORM® TGI | Polyglyceryl 3 Diisostearate | 4,00 |
| | CETIOL® A | Hexyl Laurate | 12,00 |
| | Pentadecan oder Heptadecan | | 12,00 |
| | SIPOL® C 16/18 OR | Cetearyl Alcohol | 1,00 |
| | Zinc stearate | Zinc Stearate | 2,00 |
| | Zinc Oxide | CI 77947 (or) Zinc Oxide | 15,00 |
| | Magnesium Sulphate | Magnesium Sulphate | 1,00 |
| | Glycerin | Glycerin | 3,00 |
| | Konservierungsmittel | | q.s. |
| | Benzyl Alcohol | Benzyl Alcohol | 0,40 |
| | HYDAGEN® B | Bisabolol | 0,50 |
| | Wasser | aqua | 100,00 |

Die ersten 7 Komponenten wurden bei 85°C geschmolzen. Magnesium Sulfate und Glycerin wurden im Wasser gelöst und diese Mischung wurde auf 85 °C erhitzt. Diese wässrige Phase wurde zur Ölphase gegeben und dispergiert. Unter kontinuierlichem Rühren wurde bis auf 40°C abgekühlt und dann wurden Benzyl Alkohol und Hydagen B gemischt und zu der Emulsion gegeben. Unter weiterem Rühren wurde bis auf 30°C abgekühlt und homogenisiert.

### "Body Wash" Reinigungsemulsion

| Phase | Komponente Handelsname | INCI | Gew.-% |
|---|---|---|---|
| I | Texapon ALS-IS | Ammonium Lauryl Sulfate | 30,00 |
| | TEXAPON® NSO | Sodium Laureth Sulfate | 18,00 |
| | Pentadecan oder Heptadecan | | 18,00 |
| | Plantacare® 1200 | Lauryl Glucoside | 8,00 |
| II | Jaguar HP 105 | Hydroxypopyl Guar | 2,00 |
| | Euxyl K400 | MethyldibromoGlutaronitrile and Phenoxyethanol | 0,10 |
| | Wasser | Aqua | 23,90 |
| | pH-value | 5,6 | |

**Tabelle 1: O/W-Sonnenschutzemulsionen**

| Die im Folgenden genannten Beispiele enthalten alle das gemäß Herstellbeispiel 1 erhaltene Pentadecan oder Heptadecan Gemisch. Alle Angaben sind in Gew.-%. | | | | | | | | | | | |
|---|---|---|---|---|---|---|---|---|---|---|---|
| **Komponente** | **1** | **2** | **3** | **4** | **5** | **6** | **7** | **8** | **9** | **10** | **11** |
| **L = Lotion, C = Creme,** | **L** | **C** | **S** | **L** | **C** | **L** | **L** | **C** | **L** | **C** | **L** |
| Eumulgin^{®} VL 75 | | | | | | 4 | 4 | 2 | | | |
| Eumulgin^{®} B2 | 2 | | | | | | | | | | |
| Tween^{®} 60 | | | | 1 | | | | | | | |
| Myri^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®}O | 1 | 6 | | | 5 | 2 | | 2 | | | |
| Antaron V 216 | | | 1 | | 2 | 2 | | | | 1 | |
| Lanolin, wasserfrei USP | | | | | | | 5 | | | | |
| Pentadecan oder Heptadecan | 2 | 2 | 4 | 1 | 2 | 2 | 2 | 1 | 2 | 2 | 1 |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Silikonöl Wacker AK^{®} 350 | | 2 | | | | | | | | | |
| Cetiol^{®} 868 | | | | | 2 | | 8 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |

**Fortsetzung Tabelle 1: O/W-Sonnenschutzemulsionen**

| **Komponente** | **1** | **2** | **3** | **4** | **5** | **6** | **7** | **8** | **9** | **10** | **11** |
|---|---|---|---|---|---|---|---|---|---|---|---|
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | 1 | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | | | 0.75 | | | | | 1 | 1 | | |
| Keltrol^{®} T | | | 0.25 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | Ad 100 | | | | | | | | | | |

**Tabelle 2: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme,** | **L** | **L** | **L** | **C** | **L** | **C** | **S** | **C** | **C** | **L** | **L** |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | 0.5 | |
| Lanette^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} O | | | | 2 | | | | | 1 | 1 | |
| Antaron V 220 | 1 | | | 2 | | | 0.5 | | | 2 | 0.5 |
| Pentadecan oder Heptadecan | 4 | 2 | 4 | 6 | 10 | 4 | 2 | 8 | 2 | 1 | 3 |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | 5 | | 3 | | | | | |
| Eutanol^{®} G 16 S | 10 | | | | | | | | | | |
| Cetiol^{®} PGL | | | | 5 | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz ) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} Hp 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 3: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| **L = Lotion; C = Creme** | **C** | **L** | **C** | **L** | **C** | **L** | **L** | **L** | **L** | **C** | **C** |
| Dehymuls^{®} PGPH | 4 | 2 | 1 | 3 | 3 | 1 | 1 | 2 | 2 | 4 | 1 |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Bienenwachs | 1 | | 2 | 1 | | 1 | | 3 | | 2 | 3 |
| Tego^{®} Care CG | | | | | 1 | | | | | | .5 |
| Prisorine^{®} 3505 | 1 | | 1 | 1 | | 1 | 1 | | | | 1 |
| Wollwachsalkohol, wasserfrei, USP | | | | | | | | | | | 1 |
| Antaron V 216 | 2 | | | | | | | | | | |
| Pentadecan oder Heptadecan | 3 | 4 | 2 | 1 | 10 | 2 | 2 | 6 | 3 | 12 | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 244 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |

**Fortsetzung Tabelle 3: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| Ceraphyl^{®} 45 | | | | 2 | | | | 2 | | 6 | |
| Cetiol^{®} B | | | 2 | 4 | | | | | | 3 | |
| Eutanol^{®} G | | | 3 | | 2 | | | 8 | | | |
| Cetiol^{®} PGL | | 11 | | | 2 | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 4: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion; C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 2 | 4 | 0.5 | 1 | 4 |
| Monomuls^{®} 90-O18 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Bienenwachs | | 1 | | 2 | 2 | | 3 | | 2 | 1 | 1 |
| Tego^{®} Care CG | | | | | | | | | | | |
| Isostearinsäure | 1 | 1 | | | | | 1 | 1 | | 1 | 1 |
| Wollwachsalkohol, wasserfrei | | | | | | | | | | | |
| Antaron V 220 | | 0.5 | 2 | 1 | 1 | 1 | | | | | |
| Pentadecan oder Heptadecan | 2 | 4 | 3 | 3 | 2 | 2 | 1 | 3 | 3 | 1 | 4 |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 244 | | 4 | | 2 | | | | | | | |
| Dow Corning DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Cetiol^{®} B | | | | | 3 | 3 | | 2 | 2 | | |
| Eutanol^{®} G | | | | 2 | | | 4 | | 5 | | |
| Cetiol^{®} PGL | | | | | | | 5 | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan^{®} MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote^{®} HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 5: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| **L = Lotion. C = Creme** | **C** | **L** | **C** | **L** | **C** | **L** | **L** | **L** | **C** | **C** | **C** |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Bienenwachs | 2 | | 3 | 1 | | 1 | | 1 | 3 | 2 | 1 |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Lanolin; anhydrous USP | | | 5 | | | | | | | 4 | |
| Pentadecan oder Heptadecan | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | 8 |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |

**Fortsetzung Tabelle 5: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| Cetiol^{®} 868 | | | | | | | | | | 2 | |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G 16 | | 1 | | | 1 | | | | | 3 | 7 |
| Eutanol^{®} G | | | 3 | | 1 | | | 8 | | | |
| Cetiol^{®} PGL | | | | | | 4 | | | 9 | | |
| Mandelöl | | | | | 3 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Aluminum chlorohydrate | | 8 | | | | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | | 5 | 3 | 3 | 5 | 3 | 2 | | 10 | 4 | |
| Wasser, Konservierungsmittel | ad 100, q.s. | | | | | | | | | | |

**Tabelle 6: W/O-Pflegeemulsionen**

| **Komponente** | **56** | **57** | **58** | **59** | **60** | **61** | **62** | **63** | **64** | **65** | **66** |
|---|---|---|---|---|---|---|---|---|---|---|---|
| **L = Lotion. C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 3 | 4 | 1 | 1 | 1 |
| Monomuls^{®} 90-O18 | | 1 | | 1 | | | | | | | |
| Lameform^{®} TGI | | | | | 4 | | | 1 | | 3 | |
| Abil^{®} EM 90 | | | | 3 | | | | | | 2 | |
| Isolan^{®} PDI | | 3 | | | | | | | | | 4 |
| Glucate^{®} DO | 1 | | | | | | | | | | |
| Arlacel^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Bienenwachs | | 4 | | 2 | 1 | 2 | 2 | 1 | 2 | 3 | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emery^{®} 1780 | | | | | | | | | | | |
| Lanolin anhydrous USP | | 7 | 3 | | | | | | | | |
| Pentadecan oder Heptadecan | 3 | 4 | 4 | 8 | 10 | 2 | 8 | 6 | 3 | 12 | 7 |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Corning ^{®} DC 244 | | 4 | | 2 | | 2 | | | | | |
| Dow Corning^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol^{®} J 600 | 4 | | | 3 | | | | | | | |
| Ceraphyl^{®} 45 | | | | 1 | | | | | 5 | 4 | |
| Cetiol^{®} B | | | | | 3 | 3 | | 2 | 2 | | |
| Eutanol^{®} G 16 | 1 | | | | | | 3 | | | | |
| Eutanol^{®} G | | | | 2 | | | 2 | | 5 | | |
| Cetiol^{®} PGL | | | 10 | | | | 4 | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 7: O/W-Pflegeemulsionen**

| **Komponente** | **67** | **68** | **69** | **70** | **71** | **72** | **73** | **74** | **75** | **76** | **77** |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| Eumulgin^{®} VL 75 | | | | | | 4 | | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E 24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®} AB | | 1 | | | | | | | | | 1 |
| Lanolin, wasserfrei, USP | | | | | | | 5 | | | | |
| Cetiol^{®} SB 45 | | | 1.5 | | | | 2 | | | | |
| Pentadecan oder Heptadecan | 3 | 4 | 2 | 1 | 10 | 2 | 2 | 6 | 3 | 12 | 1 |
| Cegesoft^{®} C 17 | | | | | | | | | | | |
| Myritol^{®} PC | | | | | 5 | | | | | | |
| Myritol^{®} 331 | 2 | 5 | | | | 6 | | 12 | | | |
| Finsolv^{®} TN | | | | | | 2 | | | 8 | | |
| Cetiol^{®} CC | 4 | 6 | | | | 4 | 4 | | | | 5 |
| Cetiol^{®} OE | | | 7 | | | | | | 4 | 3 | |
| Dow Corning DC^{®} 245 | | | 2 | | 5 | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Cetiol^{®} SN | | | 5 | | | | | | | | |
| Cetiol^{®} B | | | | | | | | | | 2 | |
| Eutanol^{®} G | | 2 | | 5 | | | | | | | |
| Cetiol^{®} PGL | | | | 7 | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | | 0.3 | | | |
| Carbopol^{®} Ultrez 10 | | | 0.3 | 0.2 | | 0.2 | | | 0.1 | 0.3 | |
| Cosmedia SP | | 0,3 | | | 0.2 | | | | | | 0.2 |
| Aluminum Chlorohydrate | | | 7 | | | | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s.,pH 6,5 - 7,5 | | | | | | | | | | |

**Tabelle 8: O/W-Pflegeemulsionen**

| **Komponente** | **78** | **79** | **80** | **81** | **82** | **83** | **84** | **85** | **86** | **87** | **88** |
|---|---|---|---|---|---|---|---|---|---|---|---|
| **L = Lotion, C = Creme** | **C** | **C** | **L** | **C** | **L** | **C** | **L** | **L** | **L** | **L** | **C** |
| Eumulgin^{®} VL 75 | 4 | 3 | | | | | 1 | | | | 2 |
| Generol^{®} R | | | | | | 2 | | | | | |
| Eumulgin^{®} B2 | | | | | | 2 | | | | 1 | |
| Tween^{®} 60 | | | | | | | | | | | |
| Cutina^{®} E 24 | | | | 2 | | | | | | | |
| Hostaphat^{®} KL 340 N | | | | | | | | | | | |
| Lanette^{®} E | 0.5 | | | | | | | | | | 1 |
| Amphisol^{®} K | 0.5 | 1 | | | | | | 1 | 1 | | |
| Natriumstearat | | | | | 1 | | | | | | |
| Emulgade^{®} PL 68/50 | | 6 | | | | | | 5 | | | 4 |
| Tego^{®} Care CG | | | | | | | | | | | |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{®} O | 2 | | | 2 | | 3 | 1 | | 1 | 1 | 6 |
| Novata^{®} AB | | | | | | | | | | | |
| Lanolin, wasserfrei, USP | | | | | | 4 | | | | | |
| Cetiol^{®} SB 45 | | | | | | | 2 | | | | |
| Pentadecan oder Heptadecan | 3 | 4 | 2 | 1 | 10 | 2 | 2 | 6 | 3 | 12 | 1 |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | | 5 | | | 3 | 3 | | 1 |
| Cetiol^{®} CC | | | | | | | | | | | 2 |
| Cetiol^{®} OE | | | | | 2 | | 2 | | 5 | | |
| Dow Corning DC^{®} 245 | | 2 | | | 1 | | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | 8 | | | | | | | | 10 |
| Butylenglykol | 5 | 2 | 3 | | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

**Tabelle 9: Sprayformulierungen**

| **Komponente** | **89** | **90** | **91** | **92** | **93** | **94** | **95** | **96** | **97** | **98** | **99** |
|---|---|---|---|---|---|---|---|---|---|---|---|
| **S = Körperspray, S* = Sonnenschutzspray** | **S** | **S** | **S** | **S** | **S** | **S*** | **S*** | **S*** | **S*** | **S*** | **S*** |
| Emulgade^{®} SE-PF | 8,9 | | 7,5 | 7.5 | 4,3 | 9.8 | 8.2 | 9.9 | | | |
| Eumulgin^{®} B2 | 3,1 | | 3 | | | | | 4.2 | | | |
| Eumulgin^{®} B3 | | | | | | 4.2 | 3.3 | | | | |
| Eumulgin^{®} HRE 40 | | | | | 4,7 | | | | | | |
| Cutina^{®} E 24 | | 5,9 | | 4 | | | | | | | |
| Amphisol^{®} K | | | | | | | | | 1 | 1 | 1 |
| Eumulgin^{®} VL 75 | | | | | | | | | | | 2 |
| Emulgade^{®} PL 68/50 | | 0.5 | | | | | | | 2.5 | 1 | |
| Cutina^{®} MD | | 3,1 | | | | | | | | | |
| Antaron V 220 | | | | | | 1 | 1 | 1 | | 1 | 1 |
| Pentadecan oder Heptadecan | 11 | 5 | 7 | 7 | 7 | 5 | 4 | 5 | 5 | 4 | 6 |
| Myritol^{®} PC | | | | | | | | | | | |
| Myritol^{®} 331 | | | 3 | 4 | 3 | 3 | 3 | 3 | | | |
| Finsolv^{®} TN | | 4 | | | | | | 8 | | | |
| Cetiol^{®} CC | 6 | | | 5 | 5 | 2 | 2 | 4 | | | |
| Cetiol^{®} OE | | 5 | 7 | | | 2 | | | | | |
| Dow Corning DC^{®} 244 | | 4 | 4 | 5 | | | | | | | |
| Cetiol^{®} 868 | 3 | | | | | | | | | | |
| Cetiol^{®} J 600 | | | | 2 | 2 | | | | | | |
| Cetiol^{®} B | | | | | | | 2 | | | | |
| Eutanol^{®} G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan^{®} MBC | | | | | | 2 | 2 | 2 | | 1 | 1 |
| Neo Heliopan^{®} OS | | | | | | 5 | | | | | |
| Neo Heliopan^{®} AV | | | | | | 6 | 6 | 2 | | 7.5 | 2 |
| Uvinul^{®} T 150 | | | | | | 1 | 1 | 1 | | 1 | |
| Parsol^{®} 1789 | | | | | | 1 | | 1 | | 1 | |
| Z-Cote^{®} HP 1 | | | | | | | | | | 2 | 2 |
| Eusolex^{®} T 2000 | | | | | | | | | | 2 | 2 |
| Veegum^{®} Ultra | | | | | | | | | | | 1.5 |
| Laponite^{®}XLG | | | | | | | | | | 1.5 | |
| Keltrol^{®} T | | | | | | | | | | | 0.5 |
| Pemulen^{®} TR 2 | | | | | | | | | 0.2 | | |
| Insect Repellent^{®} 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 100/ q.s./ q.s | | | | | | | | | | |

**Tabelle 10A: Antiperspirant Suspensionsstifte sowie Soft Solids - Mengenangaben in Gew.-% -**

| **Zusammensetzung** | **1** | **2** | **3** | **4** | **5** | **6** |
|---|---|---|---|---|---|---|
| Distearylether | - | 15 | - | - | - | - |
| Stearylalkohol | - | - | 15 | 10 | 14,7 | - |
| Guerbetalkohol C36 | 15 | - | - | - | - | - |
| Tribehenin | - | - | - | - | - | 20 |
| Hydrogenated Castor Oil | - | - | 4 | - | 3,7 | - |
| Pentadecan oder Heptadecan | 30 | 60 | 51 | 60 | 31,6 | 55 |
| Octyldodecanol | 5 | - | - | - | - | - |
| Dicaprylylether | 5 | - | - | - | - | - |
| Hexyldecanol + Hexyldecyl - Laurate | - | 10 | - | - | - | - |
| Cyclomethicone | 20 | - | - | - | 30 | - |
| Dry Flo Plus* | - | - | 5 | - | - | - |
| Silica | - | - | - | 2,5 | - | - |
| Talc | - | - | - | 2,5 | - | - |
| Aluminium Zirconium Tetrachlorohydrex GLY | - | - | 25 | 25 | - | 25 |
| Aluminium Chlorohydrate | 25 | 15 | - | - | 20 | - |

| | | | | | | |
|---|---|---|---|---|---|---|
| * National Starch | | | | | | |

**Tabelle 10B: Antiperspirant Suspensionsstifte sowie Soft Solids - Mengenangaben in Gew.-% -**

| **Zusammensetzung** | **7** | **8** |
|---|---|---|
| 12-Hydroxystearinsäure | 10 | 5 |
| Pentadecan oder Heptadecan | 65 | 65 |
| Dry Flo Plus* | - | 5 |
| Aluminium Zirconium Tetrachlorohydrex GLY | 25 | 25 |

| | | |
|---|---|---|
| * National Starch | | |

**Tabelle 11: Rezepturen 1 bis 13**

| **Komponenten INCI (Handelsname)** | **1** | **2** | **3** | **4** | **5** | **6** | **7** | **8** | **9** | **10** | **11** | **12** | **13** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | | | | | | | | | | | | 10.7 | 5.1 |
| Ceteareth-20 (Eumulgin^{®} B2) | | | | | | | | | | | | 5.8 | 3.4 |
| Cetearyl Glucoside, Cetearyl Alcohol (Emulgade^{®} PL 68/50) | 1 | | 1 | 1 | | 2 | 2 | | 2 | | 2 | | |
| Polyglyceryl-2 Dipolyhydroxystearate, Lauryl Glucoside, Glycerin (Eumulgin^{®} VL 75) | | 1 | | | 1 | | | 3 | | 2.5 | | | |
| Sodium Cetearyl Sulfate (Lanette^{®} E) | 1 | 1 | 1 | | 1 | | | | 1 | 1 | | | |
| Pentadecan oder Heptadecan | 5 | 4 | 8 | 3 | 5 | 8 | 4 | 2 | 4 | 3 | 5 | 10 | 2 |
| Dicaprylyl Carbonate (Cetiol^{®} CC) | 5 | 5 | 5 | | | | | 4 | | 5 | 3 | 4 | |
| Cocoglycerides (Myritol^{®} 331) | 3 | 4 | | 4 | 4 | | | | 5 | | | 3 | 3 |
| Dicaprylyl Ether (Cetiol^{®} OE) | | | | | 5 | | 3 | | 2 | | | | |
| Dibutyl Adipate (Cetiol^{®} B) | | | | 4 | | | | 4 | | 4 | | | |
| Hydrogenated Dimer Dilinoleyl/Dimethylcarbonate Copolymer (Cosmedia^{®} DC) | 1 | 1 | 1 | 1 | 1 | 1.5 | 1.5 | 2 | 3 | 2 | 1.5 | 2 | 2 |
| Ethyl Butylacetylaminopropionate | | | | | | | | | | | 5 | 5 | |
| Tocopherol | 1 | 1 | 1 | 1 | 1 | 1 | 1 | 1 | 1 | 1 | 1 | 1 | 1 |
| Zinc oxide, nanonisiert, gecoated | 5 | 5 | 5 | 5 | 5 | | 2 | | 5 | 3 | | | |
| Titanium dioxide, nanonisiert, Gecoated | | | | 5 | 5 | | 2 | 3 | 5 | 2 | | | |
| Ethyl hexyl Methoxycinnamate | 7.5 | 7.5 | 7.5 | | | | 3 | 1 | 3 | 5 | | 5 | 5 |
| Octocrylene | 9 | 9 | 9 | | | 2 | 1 | | | | 2 | | 1.5 |
| Butyl Methoxydibenzoylmethane | | | | | | 2 | 2 | | | 1 | 2 | 2 | |
| 4-Methylbenzylidene Camphor | | | | | | | 2 | | | | | | 2 |
| Ethylhexyl Triazone | | | | | | | 1 | 1 | 2 | | 1 | | |
| Diethylhexyl Butamido Triazone | | | | | | | 1 | 1 | 2 | | 1 | 2 | |
| Phenylbenzimidazole Sulfonic Acid als Na-Salz, 15% wäßrige Lsg. | | | | | | | | | | | | | 13.3 |
| Glycerin | 3 | 3 | 3 | 3 | 3 | 3 | 3 | 3 | 3 | 3 | 3 | 5 | 5 |
| Magnesium aluminium silicate (and) cellulose Gum | 0.75 | 0.75 | | 0.5 | 0.5 | | 0.5 | | | 0.35 | | | |
| Xanthan Gum | 0.25 | 0.25 | | 0.5 | 0.5 | | 0.5 | | | 0.35 | | | |
| Sodium Polyacrylate (Cosmedia^{®} SP) | | | 0.1 | | | 0.1 | 0.2 | | | | 0.1 | | |
| Acrylates/C10-30 Alkyl Acrylate Crosspolymer | | | | | | | | 0.2 | 0.1 | | | | |
| Wasser, Perfume, Preservatives | q.s. | q.s. | q.s. | q.s. | q.s. | q.s. | q.s. | q.s. | q.s. | q.s. | q.s. | q.s. | q.s. |

**Tabelle 12: Rezepturen 14 bis 26**

| **Komponenten INCI** **(Handelsname)** | **14** | **15** | **16** | **17** | **18** | **19** | **20** | **21** | **22** | **23** | **24** | **25** | **26** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | 3.7 | 3.7 | | | | | | | | | | 4.9 | 4.1 |
| Ceteareth-12 (Eumulgin^{®}B1) | 1.3 | 1.3 | | | | | | | | | | | |
| Ceteareth-20 (Eumulgin^{®}B2) | | | | | | | | | | | | 1.1 | 0.9 |
| Cetearyl Glucoside, Cetearyl Alcohol (Emulgade^{®} PL 68/50) | | | 5 | 1 | 1 | 1 | 1 | 3 | | | | | |
| Polyglyceryl-2 Dipolyhydroxystearate, Lauryl Glucoside, Glycerin (Eumulgin^{®} VL 75) | | | | | | | | | 3 | 5 | 5 | | |
| Sodium Cetearyl Sulfate (Lanette^{®} E) | | | | 0.25 | 0.25 | 0.25 | 0.25 | 0.25 | | | | | |
| Potassium Cetyl Phosphate | | | 0.5 | | | | | | | | | | |
| Pentadecan oder Heptadecan | 4 | 5 | 6 | 8 | 5 | 8 | 8 | 10 | 7 | 4 | 10 | 5 | 5 |
| Dicaprylyl Carbonate (Cetiol^{®} CC) | 5 | | 5 | | | | | | 2.5 | 4 | 4 | 5 | 5 |
| Coco-Caprylate/Caprate (Cetiol^{®} LC) | | | | 1 | 1 | 1 | 1 | 1 | | | | | |
| Caprylic/Capric Triglyceride | | | | 1 | 1 | 1 | 1 | 1 | | | | | |
| (Myritol^{®} 312) | | | | | | | | | | | | | |
| Cocoglycerides (Myritol^{®} 331) | | | | | | | | | | 4 | 4 | | |
| Cetearyl Isononanoate (Cetiol^{®} SN) | 3 | 3 | 3.5 | | | | | | | | | | |
| Octyldodecanol (Eutanol^{®} G) | | | | | | | | | 3.5 | 2 | 2 | | |
| Hexyldecanol (Eutanol^{®} G16) | | | | 1 | 1 | 1 | 1 | 1 | | | | | |
| Olus (Cegesoft^{®} PS6) | | 1.5 | 1.5 | | | | | | | | | | |
| Passiflora Incarnata (Cegesoft^{®} PFO) | 1.5 | | | | | | | | | | | | |
| Dimethicone | | | | 0.5 | 0.5 | 0.5 | 0.5 | 0.5 | | | | | |
| Dimer Distearyltricarbonate (Cosmedia^{®} DC) | 1 | | 1.5 | | | | | 1.5 | | 2,5 | 2,5 | | 0.5 |
| Triethyl Citrate (Hydagen^{®} C.A.T) | | | | | | | | | | | | 1.5 | |
| Tocopherol | | | | | | | | | 0.5 | 0.5 | 0.5 | | |
| Tocopheryl Acetate | 0.5 | 0.5 | | | | | | | | | | | |
| Ethanol | | | | | | | | | | | 5 | | |
| Aluminum Chlorhydrate (Locron L) | | | | | | | | | | | | | 40 |
| Chitosan (Hydagen^{®} DCMF) | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 0.3 | 0.5 | | |
| Glycerin, Glyceryl Polyacrylate (Hispagel^{®} 50) | | | | | | | | | | 10 | | | |
| Carbomer | | | | | | | 0.1 | | 0.2 | | 0.2 | | |
| Sodium Polyacrylate (Cosmedia^{®} SP) | | | | | 0.15 | | | | | | | | |
| Acrylates/C10-30 Alkyl Acrylate Crosspolymer | | | | | | 0.15 | | 0.05 | | | | | |
| Wasser, Perfume, Preservatives | q.s | q.s. | q.s. | q.s. | q.s. | q.s. | q.s. | q.s. | q.s. | q.s. | q.s. | q.s. | |

**Tabelle 13: Rezepturen 27 bis 33 (Formulierungen für AP/Deo)**

| **Komponenten INCI (Handelsname)** | **27** | **28** | **29** | **30** | **31** | **32** | **33** |
|---|---|---|---|---|---|---|---|
| Glyceryl Stearate, Ceteareth-20, Ceteareth-12, Cetearyl Alcohol, Cetyl Palmitate (Emulgade^{®} SE) | 6 | | 4,5 | | | 6 | |
| Ceteareth-20 (Eumulgin^{®}B2) | | | 1 | | | | |
| Cetearyl Isononanoate, Glyceryl Stearate, Ceteareth-20, Ceteareth-12, Cetearyl Alcohol, Cetyl Palmitate (Emulgade^{®} CM) | | | | | 20 | | |
| Polyglyceryl-3 Diisostearate Lameform TGI | | 3 | | | | | |
| Cocoglycerides (Novata^{®} AB) | | | | | | | 4 |
| Stearyl alcohol ( Lanette 18) | | | | 14,7 | | | |
| Hydrogenated Castor Oil (Cutina^{®} HR) | | | | 3,7 | | | 6,5 |
| Polyglyceryl-2 Dipolyhydroxystearate, (Dehymuls^{®} PGPH) | | 1 | | | | | |
| Sodium Cetearyl Sulfate (Lanette^{®} E) | 0,3 | | | | | 0,3 | |
| Behenyl Alcohol (Lanette^{®} 22) | 2 | | | | | 4 | |
| Pentadecan oder Heptadecan | 4 | 4 | 5 | 5 | 4 | 4 | 15 |
| Dicaprylyl Carbonate (Cetiol^{®} CC) | | 3 | | | | | |
| Dicaprylylether ( Cetiol^{®} OE) | 2 | | | 4 | | 3 | 9 |
| Cocoglycerides (Myritol^{®} 331) | | | | | | | |
| Cyclopentasiloxane | 3 | 5 | | 34 | | 2 | 14 |
| Cyclopentasiloxane and Dimethicone/Vinyldimethicone Crosspolymer SFE 839 (GE Bayer) | | 3 | | | | | |
| Dimethicone | 1 | | | | | | |
| Dimer Distearyltricarbonate (Cosmedia^{®} DC) | 1 | 1 | 1 | 1 | 1 | 1 | 1 |
| Triethyl Citrate (Hydagen^{®} C.A.T) | | | 2 | | | | |
| PEG-40 Hydrogenated Castor Oil | | | | | 1 | | |
| Tocopheryl Acetate | | | | 1 | | | |
| Aluminium Zirconium Tetrachlorohydrex GLY (Rezal 36) | 30 | 40 | | 22,9 | | 30 | 25 |
| Aluminum Chlorhydrate (Locron L) | | | 10 | | | | |
| Chitosan (Hydagen^{®} DCMF) | 0,05 | | | | | | |
| Glycolic Acid | 0,02 | | | | | | |
| Glycerin | | 5 | 5 | | | | |
| Propylene Carbonate ( Fluka) | | | | | | | 0,5 |
| Quaternium-18 Hectorite (Bentone 18) | | | | | | | 1 |
| Talc (Merck) | | | | | | 5 | 5 |
| MgSO4x 7H2O | | 1 | | | | | |
| Wasser Phase II | 46,7 | | 35 | | | | |
| Wasser, Perfume, Preservatives | q.s. | q.s. | q.s. | q.s. | q.s. | q.s. | q.s. |

| | | | | | | | |
|---|---|---|---|---|---|---|---|
| 27 - Antiperspirant / Deo Creme / 28 - Antiperspirant Creme (W/0) / 29 - Antiperspirant / Deo Spray 30 - Antiperspirant Stift mit Vitamin E / 31 - Deodorant Wipe - Formulierung / 32 - Antiperspirant Creme 33 - Antiperspirant Creme «Soft Solid » | | | | | | | |

In der Tabelle 14 werden Sonnenschutzformulierungen vom Typ O/W beschrieben, in der Tabelle 15 werden Pflegeemulsionen beschrieben. Durch den Einsatz des erfindungsgemäßen Kohlenwasserstoffs wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 14: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme, S = Spray** | L | C | L | C | L | C | S | C | C | L | L |
| Eumulgin^{®} VL 75 | 2 | | | | 3 | | | | 1 | | |
| Eumulgin^{®} B2 | | | | 2 | | | | | | 1 | |
| Tween^{®} 60 | | | | | | | | | | 1 | |
| Cutina^{®} E 24 | | | | 0.5 | | | | | | | |
| Hostaphat^{®} KL 340 N | | | | | | | | | | | 0.5 |
| Eumulgin^{®} SG | | | 0,5 | | | 0,5 | | 0,3 | 0,1 | | |
| Lanette^{®} E | | | | | | | 0.1 | | 0.5 | | |
| Amphisol^{®} K | 0.5 | | | | | | | | | | |
| Natriumstearat | | | | | 1 | | | | | | |
| Emulgade^{®} PL 68/50 | | 2 | 1 | | | 3 | | | | | |
| Tego^{®} Care 450 | | 2 | | | | | | | 2 | | |
| Cutina^{®} MD | | | | 2 | 1 | 3 | | | | | 1 |
| Lanette^{®} 14 | | 1 | | | | | | | | | |
| Lanette^{®} O | | | | 2 | | | | 2 | 1 | 1 | |
| Cutina^{®} PES | 1 | 1 | | 2 | | | | | | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Lanolin, wasserfrei, USP | | | | | | 1 | 1 | | | | |
| Pentadecan oder Heptadecan | 6 | 2 | 4 | 7 | 3 | 7 | 6 | 6 | 4 | 4 | 5 |
| Myritol^{®} PC | | | | | | | | | 5 | | |
| Myritol^{®} 331 | 6 | | 4 | | | 5 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | 2 | | | | | |
| Dow Corning DC^{®} 244 | | 2 | | | 1 | | | | | | |
| Dow Corning DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | | | 3 | | | | | |
| Eutanol^{®} G 16 S | 3 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E 1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | 2 | | | 2 | | 2 | | | | |
| Tinosorb^{®} S | | 1 | | | 2 | | 2 | | | | |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 15: O/W-Pflegeemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion, C = Creme** | C | C | L | C | L | C | L | L | L | L | C |
| Eumulgin^{®} VL 75 | | | 5 | | 4 | | | | | | 2 |
| Generol^{®} R | | | | | | 2 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Eumulgin^{®} SG | | | 0,1 | 0,5 | | 0,4 | | 0,2 | 0,1 | | |
| Lanette^{®} E | 0.5 | | | | | | | | | | |
| Amphisol^{®} K | 0,5 | 0.5 | | | | | | | | | |
| Natriumstearat | | | | | 1 | | | | | | |
| Emulgade^{®} PL 68/50 | | 2 | | 2 | | | | 3 | 4 | | |
| Tego^{®} Care 450 | | 1 | | | | | | | 1 | | |
| Cutina^{®} MD | 2 | 1 | 1 | 1 | | 5 | | | | 2 | |
| Lanette^{®} 14 | | | | | 1 | | | 2 | | 1 | |
| Lanette^{®} O | 2 | | | 2 | 1 | 3 | 1 | | 1 | 1 | 3 |
| Cutina^{®} PES | 1 | 2 | | 3 | 1 | | | | | 3 | 3 |
| Novata^{®} AB | | | | | | | | | 1 | 1 | |
| Lanolin, wasserfrei, USP | | | | | 4 | | | | | | |
| Cosmedia^{®} DC | | | 2 | | 1.5 | | | | 1 | 1 | |
| Cetiol^{®} SB 45 | | | | | 2 | | | | | | |
| Cegesoft^{®} C 17 | 2 | | | | | | | | | | |
| Pentadecan oder Heptadecan | 5 | 5 | 4 | 4 | 3 | 4 | 5 | 4 | 5 | 10 | 2 |
| Myritol^{®} PC | 6 | | | | 5 | | | | | | |
| Myritol^{®} 331 | 2 | | 5 | | 2 | | | | | | 3 |
| Finsolv^{®} TN | | | | 3 | 5 | | | 3 | 3 | | 1 |
| Cetiol^{®} CC | | | | 3 | 4 | | | 3 | | | |
| Cetiol^{®} OE | | | | | 2 | | 2 | | 5 | | |
| Dow Corning DC^{®} 245 | | 2 | | | 1 | 4 | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | 3 | | | | | | |
| Cetiol^{®} SN | | | | 5 | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.5 | | | | | 0.5 | 0.5 | 0.2 | | | 0.5 |
| Carbopol^{®} ETD 2001 | | 0.3 | | 0.3 | | | | | | | |
| Pemulen^{®} TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

### Formulierungen für den Sonnenschutz und die Hautpflege vom Typ Wasser in Öl

In Tabelle 16 werden Sonnenschutzformulierungen vom W/O Emulsionstyp, in Tabelle 17 werden Pflegeemulsionen beschrieben. Durch den Einsatz des Pentadecan oder Heptadecan wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 16: W/O - Sonnenschutzformulierungen**

| **Ingredient** | **1** | **2** | **3** | **4** | **5** | **6** | **7** | **8** | **9** | **10** | **11** |
|---|---|---|---|---|---|---|---|---|---|---|---|
| **L = Lotion; C = Creme** | C | L | C | L | C | L | L | L | L | C | C |
| Dehymuls^{®} PGPH | 4 | 2 | 1 | 3 | 3 | 1 | 1 | 2 | 2 | 4 | 1 |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego^{®} Care CG | | | | | 1 | | | | | | 0.5 |
| Prisorine^{®} 3505 | | | 1 | | | 1 | 1 | | | | 1 |
| Cosmedia DC | 3 | 4 | 2 | 1 | 1 | 2 | 2 | 2 | 3 | 1 | 1 |
| Pentadecan oder Heptadecan | 5 | 4 | 4 | 3 | 2 | 4 | 3 | 4 | 2 | 3 | 5 |
| Myritol^{®} 331 | 2 | | | | 3 | 6 | | | | | 3 |
| Finsolv^{®} TN | | | | 5 | | | 2 | | | | |
| Cetiol^{®} CC | 5 | | 2 | | 4 | 2 | | | 2 | 3 | 5 |
| Tegosoft DEC | | 4 | | 3 | | | 5 | 5 | | | |
| Cetiol^{®} OE | | | | | 4 | | 5 | | 4 | 2 | |
| Dow Corning^{®} DC 244 | | | 3 | | | | 2 | | 2 | 4 | |
| Dow Corning^{®} DC 2502 | 1 | | 1 | | 2 | 1 | | | | | 1 |
| Silikonöl Wacker AK 350 | | 1 | | 4 | | | | 3 | | | |
| Cetiol^{®} PGL | | 3 | | | | 2 | | | 4 | | |
| Cophero^{®} F 1300 | | | | | | 1 | | | | | |
| Magnesium sulfat x 7 H₂O | | | | | | 1 | | | | | |
| Neo Heliopan^{®} Hydro (Na-Salz) Neo Heliopan^{®} 303 | 2 | | 2.2 | | 3 | 3 | | | 1 | | 2 |
| | | 5 | | | | | | | 4 | | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A plus | | | | | 2 | | | | 3 | 3 | |
| Neo Heliopan^{®} AP (Na-Salz) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

**Tabelle 17: W/O-Pflegeemulsionen**

| **Komponente** | **55** | **56** | **57** | **58** | **59** | **60** | **61** | **62** | **63** | **64** | **65** |
|---|---|---|---|---|---|---|---|---|---|---|---|
| **L = Lotion. C = Creme** | C | L | C | L | C | L | L | L | C | C | C |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Lanolin; anhydrous USP | | | 5 | | | | | | | 4 | |
| Pentadecan oder Heptadecan | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} D SN | | 5 | | | | | | 3 | | | |
| Cetiol^{®} OE | 3 | | | | 4 | | 2 | | 4 | 2 | |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | Ad 100, q.s. | | | | | | | | | | |

**Tabelle 18**

| **Komponente** | **1** | **2** | **3** | **4** | **5** | **6** | **7** |
|---|---|---|---|---|---|---|---|
| Ethanol | | 4,00 | | 3,00 | | 4,50 | |
| Wasser dem. (add to) | 100 | 100 | 100 | 100 | 100 | 100 | 100 |
| Phenonip | 1,00 | 1,00 | 1,00 | 1,00 | 1,00 | 1,00 | 1,00 |
| Glycerin 99% | 3,00 | 3,00 | 3,00 | 3,00 | 3,00 | 3,00 | 3,00 |
| Ultragel^{®} 300 (Polyquaternium-37) | 0,8 | 1,00 | 1,50 | 0,90 | 2,00 | 1,50 | 0,75 |
| Eumulgin^{®} VL 75 (Lauryl Glucoside (and) Polyglyceryl-2 Dipolyhydroxystearate (and) Glycerin) | 1,80 | - | 0,80 | - | - | - | - |
| Emulgade^{®} PL 68/50 (Cetearyl Glucoside (and Cetearyl Alcohol) | - | - | - | - | - | 3,00 | - |
| Eumulgin^{®} SG (Sodium Stearylglutamate) | 0,10 | 0,12 | 0,30 | 0,10 | - | | 0,08 |
| Cutina^{®} PES (Pentaerythrityl Distearate) | 2,50 | 3,5 0 | 1,70 | 4,00 | 2,00 | 4,00 | 5,00 |
| Cetiol^{®} J600 (Oleyl Erucate) | 2,00 | - | 5,00 | - | - | 1,00 | 3,00 |
| Cetiol^{®} CC (Dicaprylyl Carbonate) | 3,50 | - | 4,00 | 4,00 | 2,50 | 3,00 | 4,00 |
| Cetiol^{®} PGL (Hexyldecanol (and) Hexyldecyl Laurate) | - | 2,00 | - | 4,00 | 5,00 | 5,00 | 1,00 |
| Tegosoft^{®} DEC (Diethylhexyl Carbonate) | - | - | - | 2,50 | - | - | 3,00 |
| DC^{®} 345 (Cyclopentasiloxane) | 4,00 | - | - | - | 2,00 | - | - |
| Cetiol^{®} B (Dibutyl Adipate) | 5,00 | 3,5 | 5,00 | | | 2,00 | |
| Myritol^{®} 331 (Cocoglycerides) | 3,00 | - | - | - | - | 4,00 | 4,50 |
| Pentadecan oder Heptadecan | 3,00- | 3,00 | 5,00 | 2,00 | 7,00 | 5,00 | 3,00- |
| DHA (Dihydroxyaceton) | 2,00 | 1,00 | 2,50 | 1,00 | 2,00 | 2,00 | 3,50 |
| Uvinul^{®} T 150 (Ethylhexyl Triazone) | - | | 1,00 | - | - | - | - |
| Tinosorb^{®} M (Methylene Bis-Benzotriazoyl Tetramethylbutylphenol) | - | 1,00 | 1,00 | - | - | - | - |
| Tinosorb^{®} S (Bis-Ethylhexyloxyphenol Methoxyphenyl Triazine) | - | 2,00 | - | - | 0,50 | - | - |
| Uvasorb^{®} HEB (Diethylhexyl Butamino Triazone) | - | - | - | - | 1,00 | - | - |
| Neo Heliopan^{®} AV (Ethylhexyl Methoxycinnamate | - | 5.00 | 5,00 | - | 3,00 | - | - |
| Parsol^{®} 1789 (Butyl Methoxydibenzoylmethane) | - | 3.00 | 3,00 | - | 2,00 | - | - |
| Neo Heliopan^{®} 303 (Octocrylene) | - | 3,50 | 2,00 | - | - | - | - |

**Sprühbare W/O Emulsion (SPF 20)**

| Phase | Bestandteil | INCI | Gew.-% |
|---|---|---|---|
| I | Dehymuls® LE | PEG-30 Dipolyhydroxystearate | 3.00 |
| | DC 245 | Cyclopentasiloxane | 5.00 |
| | Cetiol® A | Hexyl Laurate | 5.00 |
| | Cetiol® CC | Dicaprylyl Carbonate | 1,50 |
| | | Pentadecan oder Heptadecan | 1,50 |
| | Cosmedia ® Gel CC | Dicaprylyl Carbonate (and) Stearalkonium Hectorite (and) Propylene Carbonate | 4.00 |
| | Cetiol® Sun | Titanium Dioxide Dispersion | 20.00 |
| II | Deionized Water | Aqua | qs 100 |
| | Butylene Glycol | Butylene Glycol | 3.00 |
| | Ajidew N50 | Sodium PCA | 2.00 |
| | Magnesium Sulphate | Magnesium Sulphate | 0.50 |
| | EDTA 4 Na | Tetrasodium EDTA | 0.05 |
| | Preservative | | q.s. |
| III | Parfum | | q.s. |
| Viscosity, Brook. RVT, 20°C, Sp 4, 5 rpm 2700 mPas | | | |

**Sprühbare W/O Emulsion (SPF 20)**

| Phase | Bestandteil | INCI | Gew.-% |
|---|---|---|---|
| I | Dehymuls® LE | PEG-30 Dipolyhydroxystearate | 4.00 |
| | Dehymuls ® PGPH | Polyglyceryl-2 Dipolyhydroxystearate | 4.00 |
| | Cetiol® 868 | Ethylhexyl Stearate | 7.00 |
| | Cetiol® CC | Dicaprylyl Carbonate | 2,70 |
| | | Pentadecan oder Heptadecan | **2,70** |
| | Cosmedia ® Gel CC | Dicaprylyl Carbonate (and) Stearalkonium Hectorite (and) Propylene Carbonate | 3.00 |
| | Cetiol ® OE | Dicaprylyl Ether | 5.00 |
| | Zincum N 29 | Zinc Oxide | 2.00 |
| II | Cetiol® Sun | Titanium Dioxide Dispersion | 20.00 |
| III | Deionized Water | Aqua | qs 100.00 |
| | Glycerine | Glycerine | 5.00 |
| | Sodium Chloride | Sodium Chloride | 0.50 |
| | Magnesium Sulphate | Magnesium Sulphate | 0.50 |
| | Preservative | | q.s. |
| IV | Parfum | | q.s. |
| Viscosität: Brookfield. RVT, 20°C, Sp 5, 10 rpm 10400mPas | | | |

**Sonnenschutz Spray (SPF 6)**

| Phase | Bestandteil | INCI | Gew.-% |
|---|---|---|---|
| I | Deionized Water | Aqua | qs 100 |
| | Butylene Glycol | Butylene Glycol | 3.00 |
| II | Veegum Ultra | Magnesium Aluminum Silicate | 0.50 |
| | Keltrol T | Xanthan Gum | 0.30 |
| III | Eumulgin ® VL 75 | Lauryl Glucoside (and) Polyglyceryl-2-Dipolyhydroxystearate (and) Glycerin | 6.00 |
| | Cetiol® CC | Dicaprylyl Carbonate | 3,50 |
| | | Pentadecan oder Heptadecan | **3,50** |
| | Cetiol® AB | C12-15 Alkyl Benzoate | 7.00 |
| | Cetiol® Sun | Titanium Dioxide Dispersion | 10.00 |
| | Preservative | | q.s. |
| | Parfum | | q.s. |
| Viscosität: Brook. RVT, 20°C, Sp 4, 5 rpm 3500 mPas pH= 6.5 | | | |

**Sonnenlotion (SPF 20)**

| | Bestandteil | INCI | Gew.-% |
|---|---|---|---|
| I | Myritol® 331 | Cocoglycerides | 2.00 |
| | Cetiol® SB 45 | Shea Butter | 1.00 |
| | Cosmedia Gel CC | Dicaprylyl Carbonate (and) Stearalkonium Hectorite | 2.00 |
| | Uvasorb® HEB | Dioctyl Butamido Triazone | 3.00 |
| | | Pentadecan oder Heptadecan | 2,00 |
| | Neo Heliopan® AV | Ethylhexyl Methoxycinnamate | 4.00 |
| | Neo Heliopan® E1000 | Isoamyl p-Methoxycinnamate | 4.00 |
| | Neo Heliopan® 303 | Octocrylene | 3.00 |
| | Copherol® 1250 C | Tocopheryl Acetate | 0.25 |
| | Cosmedia® DC | Hydrogenated Dimer Dilinoleyl/Dimethylcarbonate Copolymer | 2.00 |
| II | Cetiol® Sun | Titanium Dioxide Dispersion | 6.00 |
| | Satiaxane CX 91 | Xanthan gum | 0,30 |
| | Eumulgin® VL75 | Lauryl Glucoside (and) Polyglyceryl-2-Dipolyhydroxystearate (and) Glycerin | 3.13 |
| III | Deionized Water | Water | 58.22 |
| | 2 NaEDTA | Disodium EDTA | 0.10 |
| | Liponic EG-1 | Glycereth-26 | 3.00 |
| IV | Tinosorb M | Methylene bis-benzotriazolyltetramethylbutylphenol | 3.00 |
| | Water | Water | 3.00 |
| | Preservative | | q.s. |
| | Parfum | | q.s |
| Viscosität Brook. RVT, 20°C, spindle 5, 10 rpm 2560 mPaspH= 6.70 | | | |

**Sonnenlotion (SPF 50+)**

| | Bestandteil | INCI | Gew.-% |
|---|---|---|---|
| I | Cetiol® B | Bibutyl Adipate | 2.00 |
| | Cosmedia® DC | Hydrogenated Dimer Dilinoleyl/Dimethylcarbonate Copolymer | 1.00 |
| | | Pentadecan oder Heptadecan | 2.00 |
| II | Neo Heliopan® OS | Ethylhexyl Salicylate | 3.00 |
| | Tinosorb® S | Bis-ethylhexyloxyphenol Methoxyphenyl Triazine | 2.50 |
| | Neo Heliopan® AV | Ethylhexyl Methoxycinnamate | 6.00 |
| | Neo Heliopan® 303 | Octocrylene | 8.00 |
| | Neo Heliopan® 357 | Butyl Methoxydibenzoylmethane | 2.50 |
| III | Cosmedia® Gel CC | Dicaprylyl Carbonate (and) Steralkonium Hectorite (and) Propylene Carbonate | 2.00 |
| IV | Emulgade® PL 6850 | Cetearyl Glucoside (and) Cetearyl Alcohol | 2.50 |
| | Eumulgin ® VL 75 | Lauryl Glucoside (and) Polyglyceryl-2-Dipolyhydroxystearate (and) Glycerin | 3.00 |
| V | Cetiol® Sun | Titanium Dioxide Dispersion | 10.00 |
| VI | Deionized Water | Aqua | qs 100 |
| | 2 NaEDTA | Disodium EDTA | 0.10 |
| | Preservative | | q.s. |
| | Liponic EG-1 | Glycereth-26 | 3.00 |
| | Keltrol® T | Xanthan gum | 0.60 |
| | Veegum Ultra | Magnesium Aluminum Silicate | 2.00 |
| VII | Dry Flo Plus | Aluminum Starch Octenylsuccinate | 2.00 |
| VIII | Tinosorb® M | Methylene Bis-Benzotriazolyl Tetramethylbutylphenol | 5.00 |
| IX | Parfum | | q.s. |
| Viskosität Brookfield Helipath 20°C, TB, 10 rpm 16200 mPas pH= 6.7 | | | |

**Anti-Ageing Sonnenschutz (SPF 30)**

| | Bestandteil | INCI | Gew.-% |
|---|---|---|---|
| I | Myritol® 331 | Cocoglycerides | 2.00 |
| | Cetiol® B | Bibutyl Adipate | 3.00 |
| | | Pentadecan oder Heptadecan | 2.00 |
| | Uvinul A plus | Diethylamino Hydroxybenzoyl Hexyl Benzoate | 5.00 |
| | Uvinul T 150 | Ethylhexyl Triazone | 2.50 |
| | Cosmedia® Gel CC | Dicaprylyl Carbonate (and) Stearalkonium Hectorite | 2.00 |
| | Cosmedia® DC | Hydrogenated Dimer Dilinoleyl/Dimethylcarbonate Copolymer | 2.00 |
| II | Cetiol® Sun | Titanium Dioxide Dispersion | 10.00 |
| | Satiaxane CX 91 | Xanthan gum | 0.40 |
| | Eumulgin® VL75 | Lauryl Glucoside (and) Polyglyceryl-2-Dipolyhydroxystearate (and) Glycerin | 3.50 |
| III | Deionized Water | Aqua | 49.60 |
| | 2NaEDTA | Disodium EDTA | 0.10 |
| | Glycerin | Glycerin | 3.00 |
| | Preservative | | q.s. |
| I | Photonyl® LS | Arginine (and) Disodium Adenosine | 2.00 |
| V | | Triphosphate (and) Mannitol (and) Pyridoxine HCl (and) RNA (and) Histidine HCl (and) Phenylalanine (and) Tyrosine | |
| | Water | | 12.00 |
| V | Copherol® 1250 C | Tocopheryl Acetate | 0.50 |
| | Parfum | | q.s. |
| Viskosität: Brook. RVT, 20°C, Spindel 5, 10 rpm 3560 mPaspH= 6.50 | | | |

**Anti-Falten Creme**

| | Bestandteil | INCI | Gew.-% |
|---|---|---|---|
| I | Eumulgin ® BA 25 | Beheneth-25 | 3.30 |
| | Lameform® TGI | Polyglyceryl-3 Diisostearate | 0.70 |
| | | Pentadecan oder Heptadecan | 2.00 |
| | Cutina ® PES | Pentaerythrityl Distearate | 2.00 |
| | Cetiol ®CC | Dicaprylyl Carbonate | 1.30 |
| | Cetiol ® AB | C 12-C 15 Alkyl Benzoate | 5.00 |
| | Cetiol ® LC | Coco-Caprylate/Caprate | 3.00 |
| | Cetiol® Sun | Titanium Dioxide Dispersion | 6.00 |
| | Phenonip | Phenoxyethanol (and) parabens | 0.80 |
| II | Deionized Water | Aqua | 61.85 |
| | 4NaEDTA | Tetrasodium EDTA | 0.05 |
| | Glycerin | Glycerin | 3.00 |
| | Butylene Glycol | Butylene Glycol | 2.00 |
| III | Veegum Ultra | Magnessium Aluminum Silicate | 3.00 |
| | Keltrol T | Xanthan Gum | 0.30 |
| IV | | Perfume Waterfresh | 0.50 |
| | Coviox ® T70 C | Tocopherol | 0.10 |
| V | Deionized Water | Aqua | 1.00 |
| | Plantactiv ® Aesculus | Escin | 0.10 |
| Viskosität, Brookfield, RVT, 20°C, Spindel 5, 5 rpm 11500 mPas pH= 6.2-6.7 | | | |

**Sonnenschutzlotion SPF 30**

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | | Pentadecan oder Heptadecan | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia ® DC | Hydrogenated Dimer Dilinoleyl/Dimethylcarbonate Copolymer | 1.00 |
| II | Cetiol® Sun | Titanium Dioxide Dispersion | 14.00 |
| | Satiaxane CX 91 | Xanthan gum | 0.10 |
| | Cosmedia ® SP | Sodium Polyacrylate | 0.40 |
| | Eumulgin® VL75 | Lauryl Glucoside (and) Polyglyceryl-2-Dipolyhydroxystearate (and) Glycerin | 3.75 |
| III | Deionized Water | Aqua | qs 100.00 |
| | Neo Heliopan® Hydro | Phenylbenzimidazole Sulfonic acid | 2.00 |
| | Trometamine | Trometamine | 1.60 |
| | Phenonip | | qs |
| Viscosität: Brookfield, RVT, 20°C, Spindel 5, 10 rpm 2240 mPas pH= 7.35 | | | |

**Sonnencreme SPF 50+**

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | | Pentadecan oder Heptadecan | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia® DC | Hydrogenated Dimer Dilinoleyl/Dimethylcarbonate Copolymer | 2.00 |
| | Emulgade® PL 6850 | Cetearyl Glucoside (and) Cetearyl Alcohol | 4.50 |
| | Lanette® E | Sodium Cetearyl Sulfate | 1.0 |
| II | Cetiol® Sun | Titanium Dioxide Dispersion | 14.00 |
| III | Deionized Water | Aqua | qs 100.00 |
| | Neo Heliopan® Hydro | Phenylbenzimidazole Sulfonic Acid | 2.00 |
| | NaOH 50% | Sodium Hydroxide | 0.6 |
| | | Preservative | q.s. |
| | | Parfum | q.s. |
| Viskosität: Brookfield, Helipath 20°C, TE, 4 rpm , 195000 mPas pH= 7.5 | | | |

**AP/Deo in Aerosol Form (Gew.-%)**

| Bestandteil | INCI | A | B |
|---|---|---|---|
| IPM | Isopropyl Myristate | 10,8 | 10,8 |
| Cetiol® CC | Dicaprylyl Carbonate | 5.00 | 5.00 |
| Cosmedia® Gel CC | Dicaprylyl Carbonate (and) Stearalkonium Hectorite (and) Propylene Carbonate | - | 2.00 |
| | Caprylic capric triglyceride(and)Stearalkonium Hectorite (and) Propylene Carbonate | 2.00 | - |
| | Pentadecan oder Heptadecan | 2.00 | 2.00 |
| Locron® P | Aluminium chlorhydrate | 3.00 | 3.00 |
| Hydagen® CAT | Triethyl Citrate | 1.00 | 1.00 |
| Perfume | Perfume | 1.20 | 1.20 |
| Propane | | 75.00 | 75.00 |

**Foundation oder Sonnenschutz enthaltend Titanium Dioxid**

| Ingredients | INCI Name | Gew.-% |
|---|---|---|
| Cetiol® CC | Dicaprylyl Carbonate | 95.00 |
| Cosmedia® Gel CC | Dicaprylyl Carbonate (and) Stearalkonium Hectorite (and) Propylene Carbonate | 1.00 |
| | Pentadecan oder Heptadecan | 1.00 |
| Titanium dioxide | | 5.00 |

**Sonnenschutz Spray SPF 40 (Gew.-%)**

| | Ingredient | INCI | A | B |
|---|---|---|---|---|
| I | Eumulgin ® VL 75 | Lauryl Glucoside (and) Polyglyceryl-2 Dipolyhydroxystearate (and) Glycerin | 6.0 | 6.0 |
| | Generol ® R | Brassica Campestris (Rapeseed) | 1.0 | 1.0 |
| | Cegesoft ® SH Cosmedia ® Gel CC | Sterols Shorea Stenoptera Dicaprylyl Carbonate(and) Stearalkonium Hectorite (and) Propylene Carbonate | 1.0 2.0 | 1.0 |
| | Eusolex 2292 | Ethylhexylmethoxycinnamate | 7.5 | 7.5 |
| | Tinosorb® S | Bis-ethylhexyloxyphenol methoxyphenyltriazine | 2.0 | 2.0 |
| | Cetiol ® CC | Dicaprylyl carbonate | 1.5 | |
| | | Pentadecan oder Heptadecan | 1.5 | 1.5 |
| | Cetiol ® AB | C12-15 Alkyl Benzoate | 4.0 | 4.0 |
| | Cosmedia ® DC | Hydrogenated Dimer Dilinoleyl / Dimethylcarbonate Copolymer | 1.00 | 1.00 |
| II | Water | | 61.45 | 63.45 |
| | EDTA 4Na (Prolabo) | Tetrasodium EDTA | 0,05 | 0.05 |
| | Elestab 50J | Chlorphenesin & Methylparaben | 0.60 | |
| III | Cosmedia®SP | Sodium Polyacrylate | 0.40 | 0.40 |
| I V | Tinsorb® M | Methylenebis benzotriazolyl tetramethylbutylphenol | 10.0 | 10.0 |
| Viskosität, Brookfield. RVT, 23° C, spindle 2,5 rpm, ph:6 3500mPas 700mPas | | | | |

**Spray SPF 40**

| | Bestandteil | INCI | A | B |
|---|---|---|---|---|
| I | Eumulgin® VL75 | Lauryl Glucoside (and) Polyglyceryl-2 Dipolyhydroxystearate (and) Glycerin | 6.0 | 6.0 |
| | Generol® | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 3.0 | - |
| | | Pentadecan oder Heptadecan | **2.0** | **2.0** |
| | Eusolex 2292 | Ethylhexylmethoxycinnamate | 7.5 | 7.5 |
| | Tinosorb S | Bis-ethylhexyloxyphenol methoxyphenyltriazine | 2.13 | 4.74 |
| | Cetiol® CC | Dicaprylyl Carbonate | 2.0 | 2.0 |
| | Cetiol® AB | C12-15 Alkyl Benzoate | 4.0 | 4.0 |
| II | Water | | 62.32 | 62.71 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.00 | 10.00 |
| Viskosität Book. RVT, 23° C, spindle 2,5 rpm, ph:6 A: 4020 mPas B: 730 mPas | | | | |

**Feuchtigkeitsspendende Sonnenschutz Lotion W/O SPF 20**

| | Bestandteil | INCI | A | B |
|---|---|---|---|---|
| I | Dehymuls® | PEG - 30 Dipolyhydroxystearate | 3.00 | 3.00 |
| | DC 245 | Cyclopentasiloxane | 5.00 | 5.00 |
| | Cetiol® OE | Dicaprylyl Ether | 4.00 | 4.00 |
| | Cetiol® A | Hexyl Laurate | 5.00 | 5.00 |
| | Cetiol® CC | Dicaprylyl Carbonate | 1.50 | 1.50 |
| | | Pentadecan oder Heptadecan | **1.50** | **1.50** |
| II | Cosmedia® Gel CC | Dicaprylyl Carbonate(and) Stearalkonium Hectorite (and) Propylene Carbonate | 4.00 | |
| III | Cetiol® SUN | Titanium dioxide (and) Alumina (and) Dimethicone and) Stearic acid ( and) Dicaprylyl Carbonate (and) Polyhydroxystearic acid | 20.00 | 20.00 |
| IV | Deionized water | Aqua | 49.55 | 53.55 |
| | Butylene Glycol | Butylene Glycol | 3.00 | 3.00 |
| | Ajidew N 50 | Sodium PCA | 2.00 | 2.00 |
| | Mg SO4 | Magnesium Sulfate | 0.50 | 0.50 |
| | EDTA, 4 Na | Tetrasodium EDTA | 0.05 | 0.05 |
| | Phenonip | Phenoxyethanol (and) Parabens | 0.50 | 0.50 |
| V | PerfumeSoleil/L27 8319/ C | Floressence | 0.40 | 0.40 |
| Viskosität, Brook. RVT, spindle 4, speed 5 [mPas] | | | 2000 | 5000 |

**Spray SPF 40 (Angaben in Gew.-%)**

| | Bestandteil | INCI | A | B |
|---|---|---|---|---|
| I | Eumulgin® VL 75 | Lauryl Glucoside (and) Polyglyceryl-2 Dipolyhydroxystearate (and) Glycerin | 6.0 | 6.0 |
| | Generol® R | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 2.0 | |
| | Eusolex 2292 | Ethylhexylmethoxycinnamate | 7.5 | 7.5 |
| | Tinosorb S | Bis-ethylhexyloxyphenol methoxyphenyltriazine | 2.0 | 2.0 |
| | Cetiol® CC | Dicaprylyl carbonate | 1.5 | 1.5 |
| | | Pentadecan oder Heptadecan | 1.5 | 1.5 |
| | Cetiol® AB | C12-15 Alkyl Benzoate | 4.0 | 4.0 |
| | Cosmedia ® DC | Hydrogenated Dimer Dilinoleyl/Dimethylcarbonate Copolymer | 1.0 | 1.00 |
| II | Water | | 61.45 | 63.45 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.0 | 10.0 |
| Viskosität, Brook. RVT, 23° C, spindle 2, 5 rpm, pH: 6 [mPas] A: 3500, B: 700 | | | | |

**Mat finish fluid foundation**

| | Bestandteil | INCI | Gew.-% |
|---|---|---|---|
| I | Lameform® TGI | Polyglyceryl-3 Diisostearate | 1.50 |
| | Eumulgin® BA25 | Beheneth-25 | 3.50 |
| | Cetiol® CC | Dicaprylyl Carbonate | 4.00 |
| | Myritol® 331 | Cocoglycerides | 2.00 |
| | | Pentadecan oder Heptadecan | 2.00 |
| | Fitoderm® | Squalane | 3.00 |
| | Cutina® PES | Pentaerythiryl Distearate | 1.00 |
| | Cegesoft® SH | Shorea Stenoptera | 1.00 |
| | Phenonip | Phenoxyethanol (and) Parabens | 0.80 |
| | KF 96, 100cs | Dimethicone | 3.00 |
| | Cetiol® OE | Dicaprylyl Ether | 3.00 |
| II | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33-8001 | Iron Oxide CI 77491 | 0.60 |
| | SunPuro Black Iron Oxide C33-7001 | Iron Oxide CI 77499 | 0.20 |
| III | Deionized Water | Aqua | 52.50 |
| | Glycerine | Glycerin | 3.00 |
| | Butylene Glycol | Butylene Glycol | 2.00 |
| IV | Veegum Ultra | Magnesium Aluminium Silicate | 1.00 |
| | Keltrol T | Xanthan Gum | 0.40 |
| V | Cosmedia® Gel CC | Dicaprylyl Carbonate (and) Stearalkonium Hectorite (and) Propylene Carbonate | 3.00 |
| VI | Cosmedia® PMMA V12 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosiät, Brook. RVT, spindle 3, speed 5, 8400mPas pH:6.7 | | | |

**Ultra protective spray SPF 40 0/W**

| | Bestandteil | INCI | Gew.-% |
|---|---|---|---|
| I | Eumulgin® VL 75 | Lauryl Glucoside (and) Polyglyceryl-2 Dipolyhydroxystearate (and) Glycerin | 7.00 |
| | Generol® R | Brassica Campestris (Rapeseed) Sterols | 1.00 |
| | Cosmedia® DC | Hydrogenated Dimer Dilinoleyl / Dimethylcarbonate Copolymer | 1.00 |
| | Eusolex 2292 | Ethylhexylmethoxycinnamate | 7.50 |
| | Tinosorb S | Bis-ethylhexyloxyphenol methoxyphenyltriazine | 2.00 |
| | Cetiol® CC | Dicaprylyl carbonate | 2.00 |
| | Cetiol® AB | C12-15 Alkyl Benzoate 4.00 | 4.00 |
| | | Pentadecan oder Heptadecan | 2.00 |
| | Cegesoft® VP | Olus & Hydrogenated Vegetable Oil & Candelilla Cera | 2.50 |
| | Myritol® 331 | Cocoglycerides | 2.00 |
| | Cosmedia® Gel CC | Sodium polyacrylate Carbonate(and) Stearalkonium Hectorite (and) Propylene Carbonate | 4.00 |
| II | Water | qsp100 | 55.15 |
| | EDTA 4Na | Tetrasodium EDTA 0.05 | 0.05 |
| | Elestab 50J | Chlorphenesin & Methylparaben | 0.60 |
| III | Keltrol T | Xanthan Gum | 0.20 |
| IV | Tinosorb M | Methylenebis benzotriazolyl tetramethylbutylphenol | 10.00 |
| Viscosity, Brook. RVT, 23° C, spindle 2,5 rpm, 2500mPaspH:6.4 | | | |

**Soft moisture Gel Creme**

| | Bestandteil | INCI | Gew.-% |
|---|---|---|---|
| I | Deionized Water | Aqua | 65.35 |
| | Glycerine | Glycerin | 3.00 |
| | Phenonip | Phenoxyethanol (and) Parabens | 0.60 |
| II | Cosmedia® SP | Sodium Polyacrylate | 1.20 |
| III | Cegesoft® PFO | Passiflora Incarnata | 2.00 |
| | Eusolex 2292 | Ethylhexyl Methoxycinnamate | 2.00 |
| | KF 96,100cs | Dimethicone 2.00 | 2.00 |
| IV | Cetiol® CC | Dicaprylyl Carbonate | 2.50 |
| | Eutanol® G | Octyldodecanol | 4.0 |
| | | Pentadecan oder Heptadecan | 2.50 |
| | Eusolex 4360 | Benzophenone-3 | 0.10 |
| | Cegesoft® SBE | Butyrospermum Parkii | 1.50 |
| V | Cosmedia® Gel CC | Dicaprylyl Carbonate (and) Stearalkonium Hectorite (and) Propylene Carbonate | 5.00 |
| VI | Deionized Water | Aqua | 5.00 |
| | Osmhydran® LS 8453 | Mannitol (and) Arginine (and) Serine (and) Sucrose (and) PCA (and) Citrulline (and) Glycogen (and) Histidine HCI (and) Alanine (and) Threonine (and) Glutamic Acid (and) Lysine HCI | 2.00 |
| VII | Vegeseryl® LP LS 9058 | Hydrolyzed Soy Protein | 1.00 |
| VIII | Coviox® T70C | Tocopherol | 0.10 |
| | Perfume Raphaelle | | 0.15 |
| Viscosity, Brook. RVT, Helipath TE, speed 4, 101 000mPas pH:5.5 | | | |

**Samtige Reinigungsmaske**

| | Bestandteil | INCI | Gew.-% |
|---|---|---|---|
| I | Emulgade® SE-PF | Glyceryl Stearate (and) Ceteareth-20 (and) Ceteareth-12 (and) Cetearyl Alcohol (and) Cetyl Palmitate | 8.00 |
| | Lanette® O | Cetearyl Alcohol | 2.00 |
| | Cegesoft® PS6 | Olus | 5.00 |
| | Cetiol® CC | Dicaprylyl Carbonate | 2.00 |
| | Cutina® PES | Pentaerythrityl Distearate | 3.00 |
| | | Pentadecan oder Heptadecan | 2.00 |
| II | Cosmedia® Gel CC | Dicaprylyl Carbonate (and) Stearalkonium Hectorite (and) Propylene Carbonate | 5.00 |
| III | Deionized Water | Aqua | 45.00 |
| | Glycerine | Glycerin | 10.00 |
| | EDTA,4Na | Tetrasodium EDTA | 0.10 |
| | Elestab® 50J | Chlorphenesin (and) Methylparaben | 0.40 |
| IV | Dry Flo AF | Corn Starch Modified0 | 1.00 |
| | Propylene Glycol | Propylene Glycol | 3.00 |
| V | Kaolin | Kaolin | 10.00 |
| VI | Perfume Concombre | | 0.20 |
| | Coviox® T70C | Tocopherol | 0.10 |
| VII | Purisoft ® LS 9602 | Glycerin (and) Moringa Pterygosperma | 2.00 |
| VIII | Green Covasol W7035 (a.s.0.1%) | CI 42090 (and) CI19140 | 3.20 |
| Viscosity, Brook. RVT, Helipath TE, Speed 4, 254 000mPas pH:6.3 | | | |

**Luxuriöse Körper Butter**

| | Bestandteil | INCI | Gew.-% |
|---|---|---|---|
| I | Deionized water | Aqua | 61.50 |
| | Glycerine | Glycerin | 5.00 |
| | Butylene Glycol | Butylene Glycol | 2.00 |
| | EDTA, 4 Na | Tetrasodium EDTA | 0.10 |
| | Phenonip | Phenoxyethanol (and) Parabens | 0.80 |
| II | Cosmedia® SP | Sodium Polyacrylate | 1.50 |
| III | Eumulgin® BA 25 | Beheneth-25 3.00 | 3.00 |
| | Lanette® O | Cetearyl Alcohol | 4.00 |
| | Cetiol® SN | Cetearyl Isononanoate | 3.00 |
| | Cetiol® SB45 | Butyrospermum Parkii | 5.00 |
| | Cetiol® CC | Dicaprylyl Carbonate | 0.50 |
| | | Pentadecan oder Heptadecan | 0.50 |
| | KF 96, 100cs | Dimethicone | 1.00 |
| | Generol® R | Brassica Campestris (Rapeseed) Sterols | 0.50 |
| IV | Cosmedia® Gel CC | Dicaprylyl Carbonate (and) Stearalkonium Hectorite (and) Propylene Carbonate | 5.00 |
| V | Cosmedia® PMMA V12 | Polymethylmethacrylate | 2.00 |
| | Deionized water | Aqua | 4.00 |
| VI | Coviox® T70C | Tocopherol | 0.10 |
| | Perfume Pétale 139012E | | 0.50 |
| Viscosity, Brook. RVT Helipath TE, speed4, 800 000mPas pH:7 | | | |

**Happy Deo Mist**

| Bestandteil | INCI | Gew.-% |
|---|---|---|
| IPM | Isopropyl Palmitate | 6.50 |
| Cetiol® Sensoft | Propylheptyl Caprylate | 6.50 |
| Cosmedia® Gel CC | Caprylic/Capric Triglyceride (and) Stearalkonium Hectorite (and) Propylene Carbonate | 2.00 |
| | Pentadecan oder Heptadecan | 2.00 |
| Locron P | Aluminium Clorohydrate | 5.00 |
| Perfume Deo Talco | Perfume | 0.30 |
| Propellant | | 77.7 |

| | | |
|---|---|---|
| Ratio propellant/other (3.34:1); Valve: RK 150P RA 634/5/8 (Vapor Phase); Button: V04.776 | | |

**Rich Hair conditioner / Haar Conditioner**

| Phase | Handelsname | INCI Bezeichnung | Gew.-% |
|---|---|---|---|
| I. | Dehyquart®L80 | Dicocoylethyl Hydroxyethylmonium Methosulfat (und) Propylene Glycol | 2 |
| | Lanette®O | Cetearyl Alcohol | 5 |
| | Cutina®GMS | Glyceryl Stearate | 1 |
| | Pentadecan oder Heptadecan | | **0,3** |
| | Cetiol®J 600 | Oleyl Erucate | 0,5 |
| | Plantacare®1200 UP | Lauryl Glucoside | 2 |
| II. | Glycerol | | 2 |
| | Wasser, entionisiert | | 50 |
| III. | Hydroxyethylcellulose | Hydroxyethylcellulose | 0,4 |
| | Wasser, deionisiert | | Ad 100 |
| IV. | Aloe vera Gel | | 0,1 |
| | Konservierungsmittel | | q.s. |
| | Parfum | | q.s. |
| >> | pH Wert | | 4 - 4,5 |
| >> | Viskosität mPas | | Ca. 8000 |

Herstellung: Phase I und II wurden auf 85°C erhitzt, Phase II wurde bei 85°C zugefügt und die Temperatur wurde beim Rühren gehalten bis zur vollständigen Homogenität. Die Emulsion wurde unter Rühren abgekühlt und das vorher in Wasser aufgelöste Polymer (Phase III) wurde zugefügt. Danach wurden die übrigen Additive (Phase IV) zugefügt und der pH-Wert eingestellt (z.B. mit Zitronensäure).

**Hair-Rinse & Colouring /Haar Spülung und Färbung**

| Phase | Handelsname | INCI Bezeichnung | Gew.-% |
|---|---|---|---|
| I. | Dehyquart® F75 | Distearoylethyl Hydroxyethylmonium Methosulfate (and) Cetearyl Alcohol | 2 |
| | Eumulgin® B2 | Ceteareth-20 | 0,5 |
| | Lanette® O | Cetearyl Alcohol | 4 |
| | Pentadecan oder Heptadecan | | **1,5** |
| II. | Wasser, deionisiert | | Ad 100 |
| III. | Sol. 5% Arianor Mahogany | Colour | 10 |
| IV. | Konservierungsmittel | | q.s. |
| >> | pH Wert | | 3,76 |

Herstellung: Schmelzen von Phase I bei 80-85°C. Erhitzen von Phase II auf 80-85°C and einrühren in Phase I. Rühren bei dieser Temperatur bis zur kompletten Homogenität. Kühlen unter Rühren auf 40°C. Hinzufügen von Konservierungsmittel and wenn nötig, von hitzeempfindlichen Additiven. Kühlen beim Rühren auf 30°C.Anpassen des pH Wertes, z.B. mit Zitronensäure. Hinzufügen der Arianor Lösung bei unter 40°C.

**Styling Gel Typ "Out of bed"**

| Phase | Handelsname | INCI Bezeichnung | Gew.-% |
|---|---|---|---|
| I. | Alcool 96% (Prolabo) | Alcohol 96% | 12 |
| | Luviskol® VA 64 (BASF) | PVP/VA | 4,5 |
| | Polyox® WSR-301 | PEG-90M | 0,25 |
| | Methocel®E4M Premium EP (Dow) | Hydroxypropyl Methylcellulose | 0,6 |
| | Dehyquart® L80 | Dicocoylethyl Hydroxyethylmonium Methosulfate (and) Propylene Glycol | 0,6 |
| II. | Eumulgin® HRE 40 | PEG-40 Hydrogenated Castor Oil | 1 |
| | Pentadecan oder Heptadecan | | **1,5** |
| III. | Wasser, entionisiert | | a.d. |
| IV | Hispagel® 200 | Glycerin (und) Glyceryl Polyacrylate | 36,7 |
| V | Konservierungsmittel, Parfum | | q.s. |
| >> | pH Wert | | 6,8 |
| >> | Viskosität (mPas) Brookfield RVT 23°C Spindel 5, 10rpm | | 220000 |

Herstellung: 1. Hinzufügen von Phase I bei Raumtemperatur nach und nach zum Alkohol. 2. Mischen von Phase II bis zur Homogenität und hinzufügen zu Phase I, 3. Hinzufügen von Phase III langsam zu Phase I und II, 4. Hinzufügen von Phase IV langsam zu Phase I-III, 5.wenn nötig hinzufügen von Phase V.

**Sprühbarer Hair-Care Conditioner mit Gluadin® WQTM**

| Phase | Handelsname | INCI | Gew.-% |
|---|---|---|---|
| I | Monomuls® 60-35C Powder | Hydrogenated Palm Glycerides | 1,24 |
| | Eumulgin® B1 | Ceteareth-12 | 2,76 |
| | Cetiol® OE | Dicaprylyl Ether | 5 |
| | Pentadecan oder Heptadecan | | 4 |
| | Dow Corning® 345 | Cyclomethicone | 2 |
| II. | Glycerin (86%) | | 2 |
| | Wasser | | 16 |
| III. | Gluadin® WQTM | Hydroxypropyltrimonium hydrolyzed wheat protein | 2,85 |
| | Plantacare® 2000 UP | Decyl Glucoside | 1 |
| | Wasser | | Ad 100 |
| | Konservierungsmittel | | q.s. |
| | pH Wert | | 5,5 |
| | Viskosität mPas | | <100 |

Herstellung: 1. Erhitzen der Komponenten von Phase I auf 85°C, 2. Erhitzen der Komponenten von Phase II ebenfalls auf 85°C und hinzufügen von Phase II durch Einrühren in Phase I, 3. Hinzufügen von Phase III kalt und rühren bis es abkühlt auf Raumtemperatur.

**Conditioner mit Dehyquart® C 4046**

| Phase | Handelsname | INCI | Gew.-% |
|---|---|---|---|
| I. | Dehyquart® C 4046 | Cetearyl Alcohol (und) Dipalmitoylethyl Hydroxyethylmonium Methosulfat (und) Ceteareth-20 | 4 |
| | Eutanol® G | Octyldodecanol | 1 |
| | Pentadecan oder Heptadecan | | **0,5** |
| II. | Wasser, entionisiert | | Ad 100 |
| | Parfum | | q.s. |
| | Konservierungsmittel | | q.s. |
| >> | pH Wert | | 3,5 |
| >> | Viskosität (mPas) Brookfield RVF, 23°C, Spindel 4, 10rpm | | 10300 |

Herstellung: Schmelzen von Phase I bei 80-85°C. Erhitzen von Phase II auf 80-85°C und Einrühren in Phase I. Fünf Minuten rühren bei dieser Temperatur. Abkühlen auf 40°C während des Rührens. Hinzufügen des Konservierungsmittels und wenn nötig, hitzeempfindlicher Additive. Abkühlen auf 30°C während des Rührens. Anpassen des pH Wertes, z.B. mit Zitronensäure.

**Conditioner mit Dehyquart® C 4046**

| Phase | Handelsname | INCI | Gew.-% |
|---|---|---|---|
| I. | Dehyquart® C 4046 | Cetearyl Alcohol (und) Dipalmitoylethyl Hydroxyethylmonium Methosulfat (und) Ceteareth-20 | 4 |
| | Pentadecan oder Heptadecan | | **1** |
| II. | Wasser, entionisiert | | Ad 100 |
| III. | Parfum | | q.s. |
| | Konservierungsmittel | | q.s. |
| >> | pH Wert | | 3,5 |
| >> | Viskosität (mPas) Brookfield RVF, 23°C, Spindel 4, 10rpm | | 2060 |

Herstellung: Schmelzen von Phase I bei 80-85°C. Erhitzen von Phase II auf 80-85°C und Einrühren in Phase I. Fünf Minuten Rühren bei dieser Temperatur. Abkühlen auf 40°C während des Rührens. Hinzufügen des Konservierungsmittels und wenn nötig, hitzeempfindlicher Additive. Abkühlen auf 30°C während des Rührens. Anpassen des pH Wertes, z.B. mit Zitronensäure.

**Hair Polish Glossing Spray - Japan Hair Oil sprühbar**

| Handelsname | INCI | Gew.-% |
|---|---|---|
| Dow Corning® 345 Fluid (Dow Corning) | Cyclomethicone | 85 |
| Cetiol® CC | Dicaprylyl Carbonate | 4 |
| Cetiol® ISL | Isostearyl Lactate | 3 |
| Pentadecan oder Heptadecan | | 5,2 |
| Neo Heliopan AV (Haarmann & Reimer) | Ethylhexyl Methoxycinnamate | 1,5 |
| Wacker PDM 1000 | Trimethylsiloxyphenyl Dimethicone | 0,5 |
| Konservierungsmittel | | q.s. |
| Parfum | | q.s. |
| Wasser, entionisiert | | Ad 100 |

Herstellung: Hinzufügen aller Zutaten in der Reihenfolge wie aufgeführt. Mischen bei Raumtemperatur.

**Hair gloss finishing serum**

| Handelsname INCI | | Gew.-% |
|---|---|---|
| DC®1501 (Dow Corning) | Cyclopentasiloxane (und) Dimethiconol | 70 |
| Pentadecan oder Heptadecan | | 19,7 |
| Cetiol® CC | Dicaprylyl Carbonat | 8 |
| Myritol® 318 | Caprylic/Capric Triglycerid | 0,5 |
| Neo Heliopan E 1000 (Haarmann & Reimer) | Isoamyl p-Methoxycinnamat | 1 |
| Phenonip (SIPCA) | Phenoxyethanol (und) Parabens | 0,5 |
| Parfum | | 0,3 |
| Farbstoff | | q.s. |

Herstellung: Hinzufügen der Zutaten wie gezeigt. Mischen bei Raumtemperatur. Bemerkung: Der Farbstoff und das Parfum, die benutzt werden, müssen öllöslich sein (das Parfum außerdem verträglich mit Dimethiconol, um Trübheit zu vermeiden)

**Hair gloss Gelatine für sehr geschädigtes und stumpfes Haar**

| Handelsname | INCI | Gew.-% |
|---|---|---|
| Cetiol® CC | Dicaprylyl Carbonate | 33,6 |
| Pentadecan oder Heptadecan | | 10 |
| Myritol® 318 | Caprylic/Capric Triglycerid | 43,6 |
| Wacker HDK H 20 (Wacker) | Silica Dimethyl Silylate | 12,5 |
| Parfum | | 0,3 |
| pH Wert | | N.A. |
| Viskosität bei 20°C, Helipath, spindel E, 5rpm (mPas) | | 500000 |

| | | |
|---|---|---|
| Herstellung: Vermischen der Zutaten in der angegebenen Reihenfolge. | | |

**Glossy light Hair Cream**

| Phase | Handelsname INCI | | Gew. % |
|---|---|---|---|
| I. | Dehyquart® F 75 | Distearoylethyl hydroxyethylmonium Methosulfat (und) Cetearyl Alkohol | 0,8 |
| | Dehyquart® L 80 | Dicocoylethyl Hydroxyethylmonium Methosulfat (und) Propylene Glycol | 1,8 |
| | Pentadecan oder Heptadecan | | 1 |
| | Lanette® O | Cetearyl Alcohol | 5 |
| | Cetiol® CC | Dicaprylyl Carbonate | 4 |
| II. | Wasser, entionisiert | | 87,4 |
| III. | Konservierungsmitt el | | q.s. |
| | Parfum | | q.s. |
| >> | pH Wert | | 3,3 |
| | Viskosität bei 20°C, Brookfield RVT, spindel 4, 10 rpm (mPas) | | 8600 |

Herstellung: 1. Erhitzen von Phase I auf 80-85°C, 2. Erhitzen von Phase II auf 80-85°C und hinzufügen über Phase I. Ca. 30 Minuten Rühren bei derselben Temperatur. 3. Abkühlen lassen bei leichtem Rühren. 4. Wenn die Temperatur unter 40°C ist, hinzufügen von Phase III in angegebener Reihenfolge, 5. Anpassen des pH Wertes wenn nötig.

**Tiefen Haar Pflege Creme mit Wildmango Butter**

| Phase | Handelsname | INCI | Gew.-% |
|---|---|---|---|
| I. | Strukture XL (National Starch) | Hydroxypropyl Starch Phospat | 5 |
| | Wasser, entionisiert | | 86,3 |
| II. | Dehyquart L 80 | Dicocoylethyl Hydroxyethylmonium Methosulfat (und) Propylene Glycol | 2,6 |
| | Pentadecan oder Heptadecan | | 1 |
| | Irwinol® LS 9319 | Octyldodecanol (und) Irvingia gabonensis (und) Hydrogenated Coco Glycerides | 0,1 |
| | Lamesoft®TM Benz | Glycol distearate (and) Coco Glucoside (and) | 4 |
| | | Glyceryl Oleate (and) Glyceryl Stearate | |
| | Gluadin ®WLM | Hydrolyzed Wheat Protein | 0,5 |
| | Nutrilan®MILK | Hydrolyzed Milk Protein | 0,5 |
| III. | Konservierungsmittel | | q.s. |
| | Parfum | | q.s. |
| >> | pH Wert | | 3,8 |
| >> | Viskosität (mPas), Brookfield RVT, 20°C, Spindel 4, 10 rpm | | 10000 |

Herstellung: 1.Auflösen von Struktur XL in Wasser und Rühren bis zur vollständigen Homogenität, 2. Anpassen des pH Wertes auf 3,5 - 4,0 mit Zitronensäure, verdünnt, 3. Hinzufügen der restlichen Zutaten in der aufgeführten Reihenfolge während des Rührens (Irwinol muss vor der Hinzugabe geschmolzen werden)

**Hair Cream gloss & Conditioning**

| Phase | Handelsname | INCI | Gew.-% |
|---|---|---|---|
| I. | Dehyquart® F 75 | Distearoylethyl Hydroxyethylmonium Methosulfat (und) Cetearyl Alcohol | 2 |
| | Lanette®O | Cetearyl Alcohol | 3 |
| | Pentadecan oder Heptadecan | | **1** |
| | Dehyquart® L 80 | Dicocoylethyl Hydroxyethylmonium Methosulfat (und) Propylene Glycol | 2 |
| | Cacao Butter (Nederland) | Theobroma cacao Seed Butter | 0,5 |
| | Structure XL (National Starch) | Hydroxypropyl Starch Phosphat | 5 |
| II. | Wasser, entionisiert | | 86,02 |
| | Glycerin | | 0,48 |
| III. | Konservierungsmittel | | q.s. |
| | Parfum | | q.s. |
| >> | pH Wert | | 4,3 |
| >> | Viskosität (mPas) Brookfield RVT, 20°C Spindel 5, 10 rpm | | 47600 |

Herstellung: 1. Schmelzen der Zutaten von Phase I bei 80-85°C, 2. Hinzufügen des Wassers bei derselben Temperatur während des Rührens. Diese Temperatur halten und für 30 Minuten rühren, 3. Abkühlen unter langsamen Rühren, 4. Anpassen des pH Wertes, wenn nötig mit Zitronensäure, 5. Hinzufügen des Konservierungsmittels und des Parfums bei unter 40°C.

**Deep penetrating Hair Reconstructor**

| Phase | Handelsname | INCI | Gew.- % |
|---|---|---|---|
| I. | Dehyquart® L 80 | Dicocoylethyl Hydroxyethylmonium Methosulfat (und) Propylene Glycol | 2 |
| | Pentadecan oder Heptadecan | | 1 |
| | DC 949 (Dow Corning) | Amodimethicone (und) Cetrimonium Chloride (und) Trideceth-12 | 1 |
| | Gluadin® WLM | Hydrolyzed Wheat Protein | 2 |
| | Parfum | | q.s. |
| | Konservierungsmittel | | q.s. |
| II. | Lamesoft® PW 45 | Cetyl palmitate (and) Beheneth-10 (and) Hydrogenated Castor Oil (and) Glyceryl Stearate | 4 |
| | Wasser, entionisiert | | 37,75 |
| III. | Rheocare®CTH(E) (Cosmetic Rheologies) | Polyquaternium-37 (und) Propylene Glycol Dicaprylate/Dicaprate (and) PPG-1 Trideceth-6 | 2,25 |
| | Wasser, entionisiert | | 50 |
| >> | pH Wert | | 4 |
| | Viskosität (mPas) Brookfield RVT, 20°C Spindel 4, 10 rpm | | 10500 |

Herstellung: 1. Mischen der Komponenten von Phase I bis zur vollständigen Homogenität, 2. Auflösen von Lamesoft® PW 45 in Wasser und hinzufügen über Phase 1, 3. Auflösen von Rheocare Polymer in entionisiertem Wasser bis man eine homogene Creme erhält, 4. Hinzufügen von Phase I + II über Phase III, 5. Anpassen des pH Wertes wenn nötig.

**Hair Conditioner Classic Care**

| Phase | Handelsname | INCI | Gew.- % |
|---|---|---|---|
| I. | Lanette® O | Cetearyl Alcohol | 4,5 |
| | Dehyquart® F 75 | Distearoylethyl Hydroxyethylmonium Methosulfat (und) Cetearyl Alcohol | 1,4 |
| | Cutina® GMS V | Glyceryl Stearate | 0,5 |
| | Pentadecan oder Heptadecan | | 0,2 |
| | DC 200-1000 (Dow Corning) | Dimethicone | 0,2 |
| II. | Dehyquart® A-CA | Cetrimonium Chloride | 2 |
| | Wasser, entionisiert | | 91,2 |
| III. | Konservierungsmittel | | q.s. |
| | Parfum | | q.s. |
| | pH Wert | | 3,9 |
| | Viskosität (mPas) Brookfield RVT, 20°C Spindel 4, 10 rpm | | 19700 |

Herstellung: 1. Mischen von Phase I bei 80-85°C bis zur Homogenität, 2. Hinzufügen von Phase II über Phase I bei derselben Temperatur (80-85°C) und Rühren. Weiterrühren während 30 Minuten, 3. Abkühlen bei langsamen Rühren und hinzufügen von Phase III bei einer Temperatur von weniger als 40°C, 4. Anpassen des pH Wertes wenn nötig (pH Wert ist = 3,5 - 4,5).

**Haar Glanz Nebel ("Mist")**

| Handelsname | INCI | Gew.-% |
|---|---|---|
| Pentadecan oder Heptadecan | | 80 |
| Cetiol® CC | Dicaprylyl Carbonat | 15 |
| Dow Corning® 200 Fluid (Dow Corning) | Dimethicone | 2,7 |
| Neo® Heliopan AV (Haarmann & Reimer) | Ethylhexyl Methoxycinnamate | 1,5 |
| Wacker PDM 1000 | Trimethylsiloxyphenyl Dimethicone | 0,5 |
| Parfum | | 0,3 |
| pH Wert | | N.A. |
| Viskosität bei 20 °C (mPas) | | < 100 |

| | | |
|---|---|---|
| Herstellung: Mischen der Zutaten in der angegebenen Reihenfolge | | |

**Sprühbarer Hair Conditioner, leave-on**

| Phase | Handelsname | INCI | Gew.-% |
|---|---|---|---|
| I. | Monomuls® 60 35 C | Hydrogenated Palm Glycerides | 1,24 |
| | Eumulgin® B1 | Ceteareth-12 | 2,76 |
| | Cetiol® S | Dioctylcyclohexane | 9 |
| | Cetiol® OE | Dicaprylyl Ether | 9 |
| | Pentadecan oder Heptadecan | | 2 |
| II. | Wasser | | 56,2 |
| | Gluadin® WQ | Laurdimonium Hydroxypropyl hydrolyzed wheat protein | 2,85 |
| | Plantacare® 1200 UP | Lauryl Glucoside | 1 |
| | Konservierungsmittel | | q.s. |
| | Parfum | | q.s. |
| | Viskosität mPas | | |

Herstellung: 1.Erhitzen von Phase I auf 85°C und Rühren bis es homogen ist, 2. Erhitzen von Phase II auf 85°C und langsam hinzufügen zu Phase I während des Rührens, 3. Hinzufügen von Phase III (kalt) bei 78°C zu PhaseI/II während des Rührens, 4. Der Emulsion erlauben Abzukühlen während des Rührens, welches die Emulsion ständig in Bewegung hält. Vermeiden von Lufteinschluss. Hinzufügen von Phase IV bei 30°C.

**Cream to Powder Foundation**

| | Komponente INCI Bezeichung (Handelsname, Hersteller) | Gew.% |
|---|---|---|
| A | Diisostearoyl malate (Corum 5015, Corum) | 7,7 |
| | Titanium dioxide (HD-0748, Lubrizol/Noveon) | 12,6 |
| | Iron oxides (HD-3170, Lubrizol/Noveon) | 1,25 |
| | Iron oxides (HD-3511, Lubrizol/Noveon) | 0,2 |
| B | Dimethicone (and) dimethicone crosspolymer (DC 9041 Sillcone Elastomer Blend, Dow Corning) | 8 |
| | Pentadecan oder Heptadecan | 5 |
| | Ethylhexyl Isononanoate (Corum 5021, Corum) | 6 |
| | Pentaerythryl tetraisostearate (Corum 5041, Corum) | 7 |
| | Polyisobutane (and) C₁₅₋₁₉ alkane (and) C₁₂₋₁₄ isoparaffin (Permethyl 284C, Presperse) | 3 |
| | Cholesteryl hydroxystearate (Corum 5-89, Corum) | 0,5 |
| | Carnauba Wachs | 4 |
| | Cetyl Palmitate (Corum 5000, Corum) | 0,6 |
| | Myristyl myristate (Corum 5028, Corum) | 0,6 |
| | Tribehenine (Corum 5094, Corum) | 0,5 |
| | Mikrocristallines Wachs | 1,5 |
| | Methyl methacrylate crosspolymer (Glanzpearl GMX 0610, ISP) | 5,5 |
| | Boron nilinde (Soltouch CC6059, Momantive) | 1 |
| | Talk (und) methicone (Corum 5901, Corum) | 11,67 |
| | Mica (und) methicone (und) Parffinium liquidum (mineral) oil (Sericite SL012P, Nikko) 10 | |
| | Lauroyl lysine (Corum 5106, Corum) | 6 |
| C | Pentaerythryl tetraisostearate (und) Paraffinium liquidum (mineral) oil (und) disteardimonium hectorite (und) propylene carbonate (und) palmitoyl tripeptide (Corum 9913, Corum) | 3 |
| | Pentaerythryl tetraisostearate (und) Paraffinium liquidum (mineral) oil (und) disteardimonium hectorite (und) propylene carbonate (und) palmitoyl oligopeptide (Corum 8616, Corum) | 3 |
| | Phenoxyethenol (und) methylparaben (und) butylparaben (und) ethylparaben (und) propylparaben (Paragon MEPB, Molmyre) | 1 |
| | Parfum | 0,06 |

| | | |
|---|---|---|
| Herstellung: Vormischung von A herstellen, B zu A geben und auf 90 °C erhitzen, gut mischen, Hitzezufuhr einstellen und C zugeben. | | |

**Makeup foundation**

| Komponente INCI Bezeichung (Handelsname, Hersteller) | Gew.-% |
|---|---|
| Wasser | 78,2 |
| Glycerin | 1,89 |
| Triethanolamine 99% | 0,75 |
| Methylparaben (Nipagin M. Nipa) | 0,19 |
| Xanthan gum (Keltrol SP, CP Kelco) | 0,28 |
| Titanium dioxid (Softex Titanium dioxide, C47-7756, Sun Chemical) | 8,75 |
| Brown iron oxid (SunChroma Brown iron oxide, C33-315 Sun Chroma, Sun Chemical) | 1,08 |
| Red iron oxide (SunChroma red iron oxide, C33-124 Sun Chroma, Sun Chemical) | 0,17 |
| Phenyl trimethicone (DC 556 cosmetic Fluid, Dow Corning) | 3,41 |
| Pentadecan oder Heptadecan | 1 |
| Stearic acid, 94% | 1,33 |
| Cetyl stearyl alcohol (Lanette O, Cognis) | 2,84 |
| Propylparaben (Nipasol, M. Nipa) | 0,1 |

**"Skin Corrector"**

| Komponente INCI Bezeichung (Handelsname, Hersteller) | Gew.-% |
|---|---|
| Synthetisches Wachs (Cirebelle 108, Cirebella) | 2,9 |
| Pentadecan oder Heptadecan | 19,51 |
| Synthetisches Wachs (Cirebelle 303, Cirebella) | 2,33 |
| Titanium dioxid Wachs dispersion (CWD-8906, Sun Chemical) | 65,12 |
| Yellow iron oxide wax dispersion (CWD-8942, Sun Chemical) | 4,18 |
| Red iron oxide wax dispersion (CWD-8801, Sun Chemical) | 1,91 |
| Black iron oxide wax dispersion (CWD-9921, Sun Chemical) | 0,05 |
| Alumina (und) titanium dioxid (SpectraFlex Focus White C88-1001, Sun Chemical) | 4 |

**Matte Antiaging Foundation**

| Phase | Komponente INCI Bezeichung (Handelsname, Hersteller) | Gew.-% |
|---|---|---|
| A | Cyclopentasiloxane (und) dimethicone copolyol (DC 5225C, Dow Corning) | 12 |
| | Pentadecan oder Heptadecan | 10 |
| | Titanium dioxid (BTD-401, Kobo) | 9 |
| | Yellow iron oxide (BYO-12, Kobo) | 0,8 |
| | Red iron oxide (BRO-12, Kobo) | 0,07 |
| | Black iron oxide (BRO-12, Kobo) | 0,034 |
| | Methylmethacrylate crosspolymer (Sepimat SB, Seppic) | 1 |
| | Methylmethacrylate crosspolymer (Sepimat HB V, Seppic) | 1 |
| | Phenoxyethanol (und) methylparaben (und) ethylparaben (und) propylparaben (und) butylparaben (Sepicide HB, Seppic) | 17,296 |
| | Parfum | 0,3 |
| B | Dipalmitoyl hydroxyproline (Sepilift DPHP, Seppic) | 0,5 |
| | Palmitoyl praline (und) magnesium palmitoyl-glutamate (und) sodium palmitoyl sarcosinate (Sepifeel One, Seppic) | 05, |
| | Undecylenoyl phenylalanine (Sepiwhite MSH, Seppic) | 0,1 |
| C | Sodium hydroxide, 48% Lösung | 0,2 |
| | Glycerin | 5 |
| | Sodium chloride | 2 |
| | Imidazolidinyl urea (Sepicide Cl, Seppic) | 0,2 |
| D | Wasser | 40 |

Herstellung: Phase A wird hergestellt durch Dispersion der Puder in den Silikonen und den n-Undecan/n-Tridecan unter Rühren, in Kugelmühle zerkleinern. Phasen B, C und D werden separat hergestellt und jeweils auf 80 °C erhitzt. Phase D wird zu Phase C hinzugefügt, dieses Gemisch wird dann zu B gegeben. Homogenisierung für einige Minuten, Abkühlen unter mäßigem Rühren. Diese Mischung wird dann unter heftigem Rühren zu Phase A gegeben und die entstehende Emulsion wird homogenisiert.

**Super Matte Foundation**

| Phase | Komponente INCI Bezeichung (Handelsname, Hersteller) | Gew.-% |
|---|---|---|
| A | Wasser | 54,72 |
| | Disodium EDTA | 0,1 |
| | Wasser (und) montmorillonite (und) cetyl alcohol (und) glycerin (und) pentylene glycol (und) sucrose stearate (und) aderoglucan (Matte Lite Concentrate 269, MMP) | 20 |
| B | Sucrose distearate (Sistearna SP30-C, MMP/Sisterna) | 0,75 |
| C | Titanium dioxide | 6 |
| | Red iron oxide | 0,5 |
| | Yellow iron oxide | 1,25 |
| | Black iron oxide | 0,13 |
| | Nylon 12 | 0,25 |
| D | Dimethicone (und) dimethicone/vinyl dimethicone crosspolymer (Clearocast 200, MMP) | 5 |
| | Pentadecan oder Heptadecan | 1 |
| | Isododecane (und) Isononyl Isononanoate (Clearocast 550, MMP) | 10 |
| E | Konservierungsmittel | 0,3 |

**"Chocolate Mousse"**

| Phase | Komponente INCI Bezeichung (Handelsname, Hersteller) | Gew.-% |
|---|---|---|
| A | Cl 77891 dimethicone (SAT-T-47051, US cosmetics) | 0,528 |
| | Cl 77491 dimethicone (SAT-R-33128, US cosmetics) | 0,456 |
| | Cl 77492 dimethicone (SAT-Y-33873, US cosmetics) | 0,732 |
| | Cl 77499 dimethicone (SAT-B-33134, US cosmetics) | 0,284 |
| B | Dimethicone/vinyl dimethicone crosspolymer (und) silica (Dow Corning 9701 Cosmetic Powder, Dow Corning) | 23 |
| | Zink oxid (und) dimethicone (Z-cote HP-1, BASF) | 6 |
| C | Wasser | 4 |
| | Wasser (und) propylene glycol (und) Helianthus annuus (Sonnnenblume) seed oil (und) Theobroma cacao extract (und) sclarolum gum (Cocoa Phytolat, Alban Muller) | 1 |
| | Dimethicone (Dow Corning 200 Fluid, 5cSt, Dow Corning) | 22,75 |
| | Pentadecan oder Heptadecan | 22,75 |
| | Cyclopentasiloxane (und) dimethiconol (Dow Corning 1501 Fluid, Dow Corning) | 5 |
| | Cyclopentasiloxane (und) dimethicone crosspolymer (und) dimethiconol (Dow Corning 9546 Silicone Elastomer Blend, Dow Corning) | 5 |
| | Theobroma cacao seed butter (Cocoa Butter, Alban Muller) | 1 |
| | Cocos nucifera (Kokosnuss) Öl (und) Gardenia tahitensis Blüten Extrakt (Monoi de Tahip Butter frangaced, Pacific Sud Cosmetique) | 1 |
| | Tocopherol acetate (di-alpha-Tocopherol Acetate, DSM) | 0,5 |
| | Polysilicone-15 (Parsol-SLX, DSM) | 5 |
| D | Parfum (Chocolat Creme 0310585, Expressions Perfumées) | 1 |

Herstellung: A wird vermahlen, und B wird zugefügt und gerührt. Phase C wird vorgelegt und unter Rühren und auf 45 °C erhitzt. Dann wird AB in kleinen Portionen zugefügt und zum Schluss wird D zugegeben.

**Fresh Foundation Emulsion Mousse**

| Phase | Komponente INCI Bezeichung (Handelsname, Hersteller) | Gew.-% |
|---|---|---|
| A | Ceteareth-25 (und) PEG-2 stearate (und) Paraffinium liquidum (mineral) oil (und) hydrogenated coconut oil (und) cetyl alcohol | 5 |
| | (und) sodium stearate (Base O/W 097, LCW) | |
| | Caprylic/capric triglyceride | 15 |
| | Pentadecan oder Heptadecan | 1 |
| B | Wasser | 59 |
| | Konservierungsmittel | 0,3 |
| | Glycerin | 2 |
| | Algas extract (und) sorbitol (Fucosorb, LCW) | 2 |
| C | Cl 77499 (und) silica (Unipure Black LC 989 EM, LCW) | 0,1 |
| | Cl 77492 (und) silica (Unipure Yellow LC 162 EM, LCW) | 0,75 |
| | Cl 77491 (und) silica (Unipure Red LC 361 EM, LCW) | 0,18 |
| | Cl 77891 (und) silica (Unipure white LC 981 EM, LCW) | 2,95 |
| | Mica (Submica FL, LCW) | 1 |
| | Methylmethacrylate crosspolymer (und) silica (Covabeed PMMA 2MUSL, LCW) | 1 |
| | Mica (Mica 8 R2041, LCW) | 4 |
| | Talk (Talc LCW, LCW) | 4 |
| | Sodium plyacrylate (Covacryl MV50, LCW) | 1 |
| | Silica dimethyl silyata (Covasilic 15, LCW) | 0,5 |

Herstellung: A und B werden getrennt hergestellt und jeweils auf 70°C erhitzt. B wird zu A unter Rühren zugefügt. Phase C wurde separate hergestellt und mit einem Pulvermischer gut durchmischt und danach unter Rühren zur Mischung aus A und B gegeben.

**Mattifying Fluid Foundation**

| Phase | Komponente INCI Bezeichung (Handelsname, Hersteller) | Gew.-% |
|---|---|---|
| A | Wasser | 20 |
| | Butylene glycol | 4 |
| | PEG-400 | 4 |
| | Dimethicone copolyol PEG-7 phosphate (Pecostil PS 100, Pheonix) | 1 |
| B | Titanium dioxide | 10 |
| | Talk | 2 |
| | Iron oxide yellow | 0,9 |
| | Iron oxide red | 0,3 |
| | Iron oxide black | 0,05 |
| | Titanium dioxide (Cl 77891) (und) iron oxide (Cl 77491) (und) mica (und) triethoxy caprylylsilane | 3 |
| C | C₂₀₋₂₂ alkyl phosphate (und) C₂₀₋₂₂ alcohols (Sensanov WR, Seppic) | 1 |
| | C₁₄₋₂₂ alcohol (und) C₁₂₋₂₀ alkylglucoside (Montanov L, Seppic) | 2 |
| | Isostearyl isostearat | 8 |
| | Pentadecan oder Heptadecan | 1 |
| | Ethylhexyl palmitate | 9 |
| | Vegetable oil (und) palm alcohol (und) lecithin (und) vegetable stearyl esters (Sensactive Veg. Chemyunion) | 2 |
| D | Wasser | q.s. to 100 |
| | Xanthan gum | 0,15 |
| E | Tromethamine | q.s. |
| | Tetrasodium EDTA | 0,05 |
| | Polymethyl methacrylate (Micropearl M100, Seppic) | 3 |
| F | Sodium acrylate/acryloyldimethyllaurate copolymer (und) isohexadecanle (und) polysorbane (Simulgel EG, Seppic) | 0,5 |
| G | Phenoxyethanol (und) methylparaben (und) ethylparaben (und) propylparaben (und) butylparaben (Chemynoi, Chemyunion) | 0,1 |
| | Parfum | qs |

**Ultra gloss Lippenstifte**

| Komponente INCI Bezeichung (Handelsname, Hersteller) | Gew.-% |
|---|---|
| Polyglyceryl-10 hydroxystearate/stearata/eicosadioate (und) dextrin palmitate (Nikkol Nikkowax LM, Nikko) | 5 |
| Copernicia cerifera (carnauba) Wachs | 10 |
| Cera alba (Bienenwachs) | 15 |
| Cetyl alcohol | 5 |
| Ricinus communis (castor) seed oil | 63 |
| Pentadecan oder Heptadecan | 2 |

| | |
|---|---|
| Herstellung: Mischung erhitzen und bei 80°C schmelzen und in Gießform füllen. | |

**Maximum shine Lippenstifte**

| Phase | Komponente INCI Bezeichung (Handelsname, Hersteller) | Gew.-% |
|---|---|---|
| A | D&C Red No. 6 Barium Lake (Red 6 lake, Emerald Hilton Davis) | 0,7 |
| | Iron oxides (CC33-5136 Russet Iron Oxide, Sun Chemical) | 2,5 |
| | Iron oxides (CC33-135 Black iron oxide, Sun Chemical) | 0,25 |
| | Ricinus communis (castor) seed oil (Crystal O, CasChem) | 3 |
| B | Ricinus communis (castor) seed oil (Crystal O, CasChem) | 35,75 |
| | Copernica cerifera (carnauba) Wachs (Carnauba No. 1, Strahl & Pitsch) | 1,1 |
| | Euphorbia cerifera (candelilla) Wachs (Candelilla Pure, Strahl & Pitsch) | 5 |
| | Ozokerite (Ozokerite Wax 1700, Frank B, Ross Company) | 2,5 |
| | Pentadecan oder Heptadecan | 1 |
| | Mikrocrystallines Wachs (Microwax SP 19, Strahl & Pitsch) | 2 |
| | Diisostearyl malata (Schercamol DISM Ester, Lubrizol/Noveon) | 24 |
| | Trisostearyl citrate (Schercomol TISC Ester, Lubrizol/Noveon) | 16,85 |
| | Methylparaben | 0,2 |
| | Propylparaben | 0,1 |
| C | Mica (und) iron oxides (Colorona Copper, EMD chemicals) | 5 |
| D | Ascorbyl palmitate | 0,05 |

**Ultra lasting lip color**

| Phase | Komponente INCI Bezeichung (Handelsname, Hersteller) | Gew.-% |
|---|---|---|
| A | D&C Red No. 7 Calcium Lake (31-DA-3707, Emerald Hilton Davis) | 0,6 |
| | D&C Red no. 6 Barium Lake (31-DA-3006, Emerald Hilton Davis) | 1,5 |
| | Iron oxides (CC33-5138 Russet iron oxide, Sun Chemical) | 4,5 |
| | Titanium dioxide | 0,5 |
| | Ricinus communis (castor) seed oil (Crystal O, CasChem) | 8,3 |
| B | Ricinus communis (castor) seed oil (Crystal O, CasChem) | 21,65 |
| | Copernica cerifera (carnauba) Wachs (Carnauba No. 1, Strahl & Pitsch) | 1,5 |
| | Euphorbia cerifera (candelilla) Wachs (Candelilla Pure, Strahl & Pitsch) | 6 |
| | Ozokerite (Ozokerite Wax 1700, Frank B. Ross Company) | 2,5 |
| | Mikrocrystallines Wachs (Microwax SP 19, Strahl & Pitsch) | 3,5 |
| | Triisostearyl polyglyceryl-3 dimer diinoleate (Schercemol PTTD Ester, Lubrizol/Noveon) | 10 |
| | Trisostearyl citrate (Schercomol TISC Ester, Lubrizol/Noveon) | 22 |
| | Isopropyl Isostearate (Schercamol 316 Ester, Lubrizol/Noveon) | 4 |
| | Pentadecan oder Heptadecan | 1 |
| | Methylparaben | 0,2 |
| | Propylparaben | 0,1 |
| C | Mica (und) Titanium dioxide (Flamenco Red, Engelhard) | 12 |
| D | Ascorbyl palmitate (Ascorbyl palmitate, DSM) | 0,05 |

**Long lasting Shine Lip Gloss**

| Phase | Komponente INCI Bezeichung (Handelsname, Hersteller) | Gew.-% |
|---|---|---|
| A | Octyldodecanol | 47,93 |
| B | Ethylcellulose (Ethocel 100FP, Amerchol) | 5 |
| C | Pentadecan oder Heptadecan | 18 |
| | bis-Biglyceryl polyacladipate-2 | 15 |
| | Octyl methoxycinnamate | 7,5 |
| | Polyethylene | 2 |
| D | Red No. 7 Aluminium lake (Sun Chemical) | 0,58 |
| | Iron oxide (Cl 77491, Sun Chemical) | 0,52 |
| | Iron oxide (Cl 77492, Sun Chemical) | 0,48 |
| | Iron oxide (Cl 77499, Sun Chemical) | 0,09 |
| E | Mica (und) titanium dioxide (Prepearis Shining Salin, Prespense) | 1,9 |
| | Dimethicone crosspolymer/silica (DC 7901 Powder, Dow Corning) | 1 |

**High-shine Lippenstift**

| | Komponente | Gew.-% |
|---|---|---|
| A | Hydrogenated Polydecene (und) ethylene propylene/styrene copolymer (und) butylene/ethylene/styrene copolymer (Versagel MG 750, Penreco) | 25,89 |
| | Petrolatum (und) ethylene/propylene/styrene copolymer (und) butylene/ethylene/styrene copolymer (Versagel P100, Penreco) | 10,38 |
| | Euphorbia cerifera (candelilla) wachs (Candelilla wax refined, Ross) | 8,8 |
| | Cera alba (Bienenwachs) (White Bleached Beeswax, Ross) | 5,18 |
| | Lanolin Wachs | 4,14 |
| | Lanolin Öl | 4,14 |
| | Octyldodecanol (Eutanol® G, Cognis) | 4,14 |
| | Pentadecan oder Heptadecan | 3,63 |
| | Copernicia cerifera (carnauba) Wachs (Carnauba wax, Ross) | 3,11 |
| | Octyldodecyl stearoyl stearate (Ceraphyl 647, ISP) | 3,11 |
| | Tridecyl trimellitate (Liponate TDTM, Lipo) | 3,11 |
| | Propylparaben | 0,1 |
| | Butylated hydroxytoluene | 0,1 |
| B | Butyrospermum parkii (shea butter) (Shea Butter, Ultra refined, Biochemica) | 5,18 |
| | Jojoba esters (Floraesters 30, Floratech) | 2,07 |
| | Theobroma grandiflorum seed butter (Cupuacu Butter, Beraca/Ross) | 2,07 |
| C | Iron oxides (und) synthetic fluorphlogopite (Sunshine Super Russet, Sun Chemical) | 8,29 |
| | Iron oxides (Soft-Tex Russet Iron Oxide, Sun Chemical) | 5,18 |
| | Red No. 7 Lake | 1,04 |
| | Yellow No. 6 Lake | 0,38 |

**Eleganter Lippenstift**

| Komponente INCI Bezeichnung (Hersteller) | Gew.-% |
|---|---|
| Dipentaerythrityl hexacaprylate/hexacaprate (und) tridecyl trimellitate (und) tri-decyl stearate (und) neopentyl glycol dicaprylate/dicaprate (Lipovol MOS-350, Lipo) | 72,2 |
| Synthetisches Bienenwachs (Lipobee 102, Lipo) | 8 |
| Copernicia cerifera (carnauba) Wachs | 4 |
| Euphorbia cerifera (candelilla) wachs | 4 |
| Pentadecan oder Heptadecan | 1 |
| Propylparaben | 0,2 |
| Mica (und) boron nitride (Lipomic 501 BN, Lipo) | 1 |
| Tocopherol (Vitamin E Acetate, Ruger Chemical) | 0,5 |
| Titanium dioxide (Cl 77891) (und) dipentaerythrityl hexacaprylate/hexacaprate (und) tridecyl trimellitate (und) tridecyl stearate (und) neopentyl glycol dicaprylate/dicaprate (Titanium dioxide, 60% in Lipovol MOS-350, Lipo) | 2,8 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Red, 60% in Lipovol MOS-350, Lipo) | 4,7 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Yellow, 60% in Lipovol MOS-350, Lipo) | 0,8 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Black, 60% in Lipovol MOS-350, Lipo) | 0,8 |

Herstellung: Alle Bestandteile werden unter Rühren auf 80-85 °C erhitzt. Der Ansatz wird auf 70-75°C abgekühlt und in eine Gießform gefüllt.

**Lip Gloss**

| | Komponente INCI Bezeichnung (Hersteller) | Gew.-% |
|---|---|---|
| A | Pentadecan oder Heptadecan | 2 |
| | C₄₋₂₄ alkyl dimethicone/divinyldimethicone crosspolymer dimethicone (NuLastic Silk MA, DM, Alzo) | 15 |
| | Methylheptyldiisostearate (Beantree, Alzo) | 10 |
| | C₄₋₂₄ alkyl dimethicone/divinyldimethicone crosspolymer dimethicone | q.s to |
| | (NuLastic Silk MA, DM-6, Alzo) | 100 |
| | Tocopherol (Vitamin E, BASF) | 0,2 |
| | Konservierungsmittel | qs |
| | Parfum (Peach Nectar 6104085, Bell Flavors & Fragances) | 0,2 |
| B | Calcium Sodium borosilicate (und) Iron oxides (Reflecks Dimensions Shiny Bronze G25000000, BASF) | 1 |
| | Mica (und) Titanium dioxide (Flamenco Winter Sparide 1300000 | 5 |
| | Hydrogenated polyisobutene (Luvitol Lite, BASF) | 10 |

| | | |
|---|---|---|
| Herstellung: Phasen A und B werden in getrennten Gefäßen jeweils homogenisiert, dann wird Phase B zu Phase A gefügt und gemischt, bis das Produkt homogen vorliegt. | | |

**Creamy Sheer Lip Colour**

| | Komponente INCI | Gew.-% |
|---|---|---|
| A | Sesamum indicum (Sesam) Samen Öl | 21,28 |
| | Octyldodecanol (Jarcol I-20, Jarchem) | 15,47 |
| | Euphorbia cerifera (candelilla) wachs (SP75, Strahl & Pitsch) | 12 |
| | Isohexyl Caprate | 10,07 |
| | Bis-Diglyceryl polyaryladipate-2 (Softisan 649, Sasol) | 9,95 |
| | Polyisobutene (Permethyl 104A, Presperse) | 9,67 |
| | Pentadecan oder Heptadecan | 2,89 |
| | Polyethylene (Performalene PL Polyethylene, New Phrase) | 2,5 |
| | Mikrocrystallines Wachs (Be Square 195, New Phrase) | 2,5 |
| B | Synthetic wax and titanium dioxide (und) Isopropyl Titanium triisostearate (SW65U, Kobo) | 4,24 |
| | Synthetic wax and iron oxide (und) Isopropyl Titanium triisostearate (SW60ER, Kobo) | 1,41 |
| | Synthetic wax and D&C Red No. 6 Barium Lake (und) Isopropyl Titanium triisostearate (SW40R6E, Kobo) | 1,41 |
| | Synthetic wax and Blue No.1 Lake (und) Isopropyl Titanium triisostearate (SW30B1A, Kobo) | 0,71 |
| | Mica (und) titanium dioxide (KTZ Classic White, Kobo) | 0,96 |
| C | Vitis vinifera (grape) seed oil | 4,84 |
| | Vitamin E acetata | 0,1 |

**Lip volume booster**

| | INCI Bezeichnung (Handelsname, Hersteller) | Gew.-% |
|---|---|---|
| A | Ricinus communis (castor) oil | 39,7 |
| | Hydrogenated coco-glycerides (Softisan 100, Sasol) | 8 |
| | Candelilla cera (und) Paraffin (Cenilla G, Barlocher) | 9 |
| | Bis-Diglyceryl polyacyladipate-1 (Softisan 645, Sasol) | 8 |
| | Cera alba (Cerabell White, Barlocher) | 3 |
| | Cera carnauba Wachs (Cernauba T1, Barlocher) | 2 |
| | Tricaprylin (Trivant OC-G, Trivant Chemical) | 20 |
| | Tocopheryl acetate (D-alpha-Tocopheryl Acetate, DSM/Roche) | 0,5 |
| | Pentadecan oder Heptadecan | 1 |
| | Propylparaben (Nipasol M. Nipa) | 0,1 |
| | BHT (Butylhydroxytoluol) | 0,05 |
| B | Ricinus communis (castor) oil (und) Cl 15850 (und) BHT (COD 8001, Sun Chemical) | 0,8 |
| | Calcium aluminum borosilicate (und) Cl 77891 (und) silica (und) tin oxide (Fionastar Noble Sparks, Merck) | 1 |
| | Mica (und) Cl 77891 (und) Cl 77491 (Timiron MP29 Sun Gold Sparkle, Merck) | 2 |
| | Mica (und) Cl 77891 (Timiron MP149 Diamond Cluster, Merck) | 2 |
| C | Glycerin (und) palmitoyl tripeptide-3 (Syn-Coll, Pentapharm) | 2,5 |
| | Alkyl methacrylate crosspolymer (Poly-Pore E200, Amcol HBS) | 0,35 |

Herstellung: alle Bestandteile der Phase A auf 80°C erhitzen, Phase B zu A hinzufügen, Phase C mischen und zu AB zugeben kurz vor der Homogenisierung.

**Plumping effect Lippenstift**

| | Komponenten INCI Bezeichnung (Handelsname, Hersteller) | Gew.-% |
|---|---|---|
| A | D&C Red No. 7 Ca Lake (Cl 15850:1) (HD-3007, Noveon) | 0,4 |
| | Red iron oxide (Cl 77491) (HD-3511, Noveon) | 0,4 |
| | Titanium dioxide Cl 77891 (HD-0748, Noveon) | 1,2 |
| | Paraffinum liquidum (mineral) oil (und) disteardimonium hectorite (und) propylene carbonate (Bentone Gel MO V, Elementis Specialities) | 1,5 |
| | Diisostearyl maleate (Corum 5015, Corum) | 10,5 |
| | Mica (und) titanium dioxide (und) tin oxide (Prestige Sparkling blue, Eckart) | 1,5 |
| B | Pentaerythrityl tetraisostearate (Corum 5041, Corum) | 15 |
| | Cholesteryl hydroxystearate (Corum 5069, corum) | 1 |
| | Hydrogenated polydecene (Nexbase 2006FG, Fortum) | 15 |
| | Pentadecan oder Heptadecan | 10 |
| | Isostearyl isostearate (corum 5088, Corum) | 19,9 |
| | Phenyltrimethicone (DC 556, Dow Corning) | 2 |
| | PEG-8 Bienenwachs (Corum 2680, Corum) | 2 |
| | Ceresin | 9 |
| | Euphorbia cerifera (candelilla) wachs | 2 |
| | Cera alba (Bienenwachs) | 2 |
| | Copernicia cerifera (carnauba) Wachs | 3 |
| | Jojoba ester (Floraester-70, Floratech) | 1 |
| | Pentaerythrityl tetraisostearate (und) Paraffinum liquidum (mineral) oil (und) disteardimonium hectorite (und) propyl carbonate (und) palmitoyl oligopeptide (Corum 8813, Corum) | 2 |
| C | Vanilyl butyl ether (Corum 9235, Corum) | 0,4 |
| | Flavor | 0,2 |

Herstellung: Phase A wird zur Homogenität gemischt, die Phase B wird hinzugefügt und auf 90°C erhitzt und gut gemischt. Die Hitzezufuhr wird gestoppt und Phase C hinzugefügt, danach gut gemischt.

**Lippenstift**

| | Komponenten INCI Bezeichnung (Handelsname, Hersteller) | Gew.-% |
|---|---|---|
| A | Propylene glycol dicaprylate/dicaprate (und) bis-diglyceryl polyacyladipate-1 (und) bis-diglyceryl polyadipate-2 (Softisan Gel, Sasol) | 9 |
| | Stearalkonium hectorite (und) propylene carbonate (Softisan 649, Sasol) | 6 |
| | Hydrogenated coco-glycerides (Softisan 100, Sasol) | 22,5 |
| | Hydrogenated palm oil (Softisan 154, Sasol) | 3 |
| | C₁₂₋₁₀ alkyl octanoate (Cosmacol EDI, Sasol) | 5 |
| | Petrolsturm (Merkur Vaseline773, Merkur) | 11 |
| | Cera alba (Bienenwachs) | 7 |
| | Cyclomethicone (Beisli CM 040, Wacker Chemie) | 3 |
| | Pentadecan oder Heptadecan | 4 |
| | Ricinus communis (castor) oil | 6 |
| | Paraffin | 14 |
| | Antioxidans | qs |
| B | Titanium dioxide (Cl 77891) (Timiron Splendid Red, Merck) | 3,5 |
| | Mica (und) Titanium dioxide (Cl 77891) (Timiron Starlusler MP-115, Merck) | 2,5 |
| | Mica (und) Titanium dioxide (Cl 77891) (Timiron Super Silver Fine, Merck) | 3 |
| C | Parfum | qs |
| | Tocopheryl acetate | 0,5 |

**Volumizing Mascara**

| | Komponenten INCI Bezeichnung (Handelsname, Hersteller) | Gew.-% |
|---|---|---|
| A | Cera alba (Bienenwachs) (White Beeswax SP422P, Strahl & Pitsch) | 2,76 |
| | Glyceryl stearate | 2,3 |
| | Copernicia cerifera (carnauba) Wachs (Carnauba wax SP63, Strahl & Pitsch) | 1,98 |
| | Stearic acid | 3 |
| | Ethylcellulose (Ethocel Standard 100FP Premium, Amerchol) | 3 |
| | Pentadecan oder Heptadecan | 1 |
| | Octyldodecanol | 7 |
| | Premised Chitosan PCA, 2,5% (Kytamer PCA Polymer, Amerchol) | 15 |
| B | Iron oxide (Black Iron Oxide, Sun Chemical) | 6 |
| | Wasser | 55,45 |
| C | Triethanolamine 99% | 1,5 |
| D | Propylene glycol (und) diazolidinyl ures (und) methylparaben (und) propylparaben (NiPaguard, Clariant) | 1 |

**Lidschatten Stift (Eye shadow stick)**

| | Komponenten INCI Bezeichnung (Handelsname, Hersteller) | Gew.-% |
|---|---|---|
| A | Pentadecan oder Heptadecan | 1 |
| | Ocryldodacyl neopentanoate (Elefac I-205, Alzo) | qs. to 100 |
| | Calcium sodium borosilicate (und) silica (und) titanium dioxide (Reflecks MultiDimensions Varying Violet G58000000, BASF) | 14 |
| | Mica (und) Titanium dioxide (und) iron oxides (Gemtone Moonstone G004, BASF) | 1 |
| B | Butylene glycol | 3,13 |
| | Acetylated glycol stearate (Unitwax, Guardian, ISP) | 22 |
| | Silica (Cab-o-Sil M5, Cabot) | 1,5 |
| C | Simmondsia chinensis (jojoba) seed oil (Lipovol J. Lipo) | 7,5 |
| | Stearic acid (Emersol 132, Cognis) | 3,6 |
| | Konservierungsmittel | qs |
| | Antioxidans | qs |

| | | |
|---|---|---|
| Phase B wird geschmolzen und gemischt, und dann zur vorgemischten Phase A gegeben. AB werden unter Rühren auf 80-05 °C erhitzt und dann wird langsam Phase C zugegeben. | | |

**Metallic Silver shine Mascara**

| | Komponenten INCI Bezeichnung (Handelsname, Hersteller) | Gew.-% |
|---|---|---|
| A | Wasser | 56,25 |
| | Xanthan gum (Keltrol T, CP Kelco) | 0,15 |
| | Magnesium aluminium silicate (Veegum HV, RT Vandervilt) | 0,55 |
| | Carbon black (und) wasser (MBD 201 20% Dispersion, Geotech) | 5 |
| B | Disodium EDTA (Edeta BD, BASF) | 0,05 |
| | Methylparaben (Methyl-4-hydroxybenzoate H 5501, Sigma Aldrich) | 0,3 |
| | Triethanolamine | 0,5 |
| | Propylene glycol | 2 |
| C | Aluminium powder (und) silica (Visionaire Bright Silver sea, Eckart) | 3 |
| D | Pentadecan oder Heptadecan | 5 |
| | Cyclopentasiloxane (und) PEG/PPG-18/18 dimethicone (Dow Corning 5225C Formulation Aid, Dow Corning) | 1 |
| | Cera alba (Bienenwachs) (Ewacers 12, Wagner) | 10 |
| | Stearic acid (Kortacid 1695, Akzo Nobel) | 2 |
| | Glyceryl stearate (Imwitor 960K, Sasol Wax) | 2 |
| | Stearyl alcohol (Lanette 18, Cognis) | 2 |
| | Polyisobutene (Permethyl-104A, Chesham Chemicals) | 1 |
| | Phenoxyethanol (und) methylparaben (und) butylparaben (und) ethylparaben (und) propylparaben (und) isobutylparaben (Phenonip, Clariant) | 1,2 |
| | PVP polyvinylpyrrolidone (Luviskol K30 Powder, BASF) | 4 |
| | Carnauba Wachs (Ewacera 34, Wagner) | 4 |

Herstellung: A wird gemischt, B wird gemischt und zu A zugefügt, C wird zu AB gegeben und auf 75°C erhitzt. D wird separat vorgemischt und auf 75°C erhitzt und zu ABC gegeben, während des Abkühlens wird gerührt.

**Creamy Shadow Stick**

| | Komponenten INCI Bezeichnung (Handelsname, Hersteller) | Gew.-% |
|---|---|---|
| A | Pentadecan oder Heptadecan | 38,5 |
| | PPG-3 myristyl ether (Varonic APM, Evonik, Degussa) | 7,0 |
| | Polyglyceryl-4- isostearate (and) cetyl PEG-PPG-10/1 dimethicone (and) hexyl laurate (Abil WE 09, Evonik, Degussa) | 1,0 |
| | Dimethicone, 10 mPas | 2,5 |
| | Cera alba (bees wax) | 4,5 |
| | Copernicia cerifera (carnauba) wax | 4,0 |
| | Ethylene/VA copolymer (A-C copolymer 400, Honeywell) | 2,5 |
| | Ozokerite | 5,8 |
| | C 16-C36 acid triglyceride | 2,0 |
| | Isobutylparaben (and) isopropylparaben (and) butylparaben (Liquipar Oil, From Nature with Love) | 0,5 |
| B | Nylon-12 (egolon 12-10, Evonik, Degussa | 2,0 |
| | Titanium dioxide | 5,0 |
| | Chromium oxide green | 10,00 |
| | CI 77491 (and) aluminium powder (and) silica | 5,0 |
| | CI 77891 (and) CI 77288 (and)mica | 10,0 |

**Wasserfeste Mascara**

| | Komponenten INCI Bezeichnung (Handelsname, Hersteller) | Gew.-% |
|---|---|---|
| A | Wasser | 32,84 |
| | Magnesium aluminium silicate (Veegum HV, RT Vanderbilt) | 0,10 |
| | Xanthan gum (Keltrol 1000, Kelco Corp.) | 0,20 |
| | Methylparaben | 0,25 |
| | Trisodium EDTA (Protacide Na3 EDTA, Protameen) | 0,01 |
| | Premixed sodium acrylates/acrylnitrogens copolymer (Hydrilien 9, 2,5% gel. Lio - siehe unten) | 15,00 |
| B | Glycerin (and)polyester 5 (Lipo PE Base G-55, Lipo) | 12,00 |
| | Talc (Rose Talc, Prespersa) | 3,00 |
| | Iron oxide (CI 77499) (Iron Oxide Black, Ultra Chemical) | 9,00 |
| C | Triethanolamine, 99% | 1,00 |
| D | Synthetic bees wax (Lipobee 102, Lipo) | 10,00 |
| | Copernicia cerifera (carnauba) wax | 4,50 |
| | Pentadecan oder Heptadecan | 5,50 |
| | C12-C16 alkyl benzoate (Liponate NEB, Lipo) | 1,00 |
| | Stearic acid (Lipo Stearic Acid, Lipo) | 3,00 |
| | Sorbitan sesquioleate (Liposorb SQO, Lipo) | 1,30 |
| | Propylparaben | 0,20 |
| E | Water (aqua) | 1,00 |
| | Imidazolidinyl urea (Leposerve IU, Lipo) | 0,10 |

Herstellung: Die Bestandteile der Phase A werden gemischt und auf 70 °C erhitzt, Phase A wird in einer Kolloidmühle gemahlen, die Bestandteile der Phase B werden in der angegebenen Reihenfolge zugefügt unter beständigem Mahlen, bis das Pigment gleichmäßig dispergiert ist, danach wird Phase C zugefügt. Die Bestandteile von C werden gemischt und bei 80-84°C gemischt (und dabei Entlüftet). Phase D wird zum Ansatz gegeben und emulgiert, der Ansatz wird auf 35 °C abgekühlt und die vorgemischte Phase E wird zugefügt. Bei 30°C wird der Ansatz in geeignete Gefäße abgefüllt.

Sodium acrylates/acrylnitrogens copolymer Premix: 97,50 Gew.- % Wasser und 2,5 Gew.- % Sodium acrylates/acrylnitrogens copolymer werden gemischt und auf 78-80°C erhitzt unter Rühren. Es wird für 15 bis 20 Minuten geführt bis die Lösung klar und homogen ist. Danach wird auf Raumtemperatur abgekühlt.

**Sebum resistenter Lidschatten**

| | Komponenten INCI Bezeichnung (Handelsname, Hersteller) | Gew.-% |
|---|---|---|
| A | Stearyl dimethicone (DC 2503 Cosmetic Wax, Dow Corning) | 10,00 |
| | C30-C45 alkyl methicone (and) C30-C45 olefin (DC AMS-C30 Cosmetic Wax, Dow Corning) | 5,00 |
| | Isododecane (and) arcylates/polytrimethylsiloxymethacrylate (DC FA 4002 ID Silicaone Acrylate, Dow Corning) | 10,00 |
| | Cyclopentasiloxane (and) triemthylsiloxymethacrylate (DC 749 Fluid, Dow Corning) | 51,00 |
| B | Cyclopentasiloxane (and) dimethicone crosspolymere (DC 9040 Silcone Elastomer Blend, Dow Corning) | 5,00 |
| | Pentadecan oder Heptadecan | 3 |
| C | Silica silylate (DC VM-2270 Aerogel Fine Particles, Dow Corning) | 1,00 |
| | Mica | 15,00 |

Herstellung: Die Bestandteile der Phase A werden gemischt und auf 80 °C erhitzt, unter Rühren wird abgekühlt. Die Phase B wird gemischt und unter Rühren zur Phase A gegeben, C wird unter langsamem Rühren zugegeben.

**Lippenstift**

| | Inhaltsstoff | INCI Bezeichnung | Gew.-% |
|---|---|---|---|
| I | Cerilla | Candellila Wax | 9.00 |
| | Cerewax | Microcristallina Wax | 3.50 |
| | Cerozo | Ozokerite Wax | 2.50 |
| | Cerauba | Carnauba Wax | 2.50 |
| | Cetiol MM | Myristyl Mysristate | 5.00 |
| | Eutanol® G | Octyldodecanol | 10.00 |
| | Myritol® 331 | Cocoglycerides | 5.00 |
| | Cetiol® CC | Dicaprylyl Carbonate | 4.00 |
| | | Pentadecan oder Heptadecan | 4.00 |
| | Cetiol® J600 | Oleyl Erucate | 1.00 |
| | Nipasol M | Propyl Paraben | 0.50 |
| | Lamesoft® TGI | Polyglyceryl-3 Diisostearate | 3.00 |
| | Cetiol® Sensoft | Propylheptyl Caprylate | 6.00 |
| II | COD 8008 | CI 77981 | 2.70 |
| | COD 8001 | CI 15850 | 12.90 |
| | COD 8010 | CI 15985 | 2.80 |
| | COD 8002 | CI 15850 | 1.40 |
| | COD 8007 | CI 42090 | 0.20 |
| | Ricinus Oil Codex | Ricinus Communis | 20.60 |
| III | | Fragance Astral 112 445 B | 0.40 |
| | Irwinol® LS 9319 | Octyldodecanol & Irvingia Gabonesis & Hydrogenated Coco-Glycerides | 2.50 |
| | Coviox® T70C | Tocopherol | 0.50 |

**Lippenpflege**

| | Inhaltsstoff | INCI Bezeichnung | Gew.-% |
|---|---|---|---|
| I | Polybutene M100 | Polybutene | 53.50 |
| | Cetiol® Sensoft | Propylheptyl Caprylate | 10.00 |
| | | Pentadecan oder Heptadecan | **5.00** |
| | Isopropyl palmitate | Isopropyl Palmitate | 20.00 |
| | Cosmedia® DC | Hydrogenated Dimer Dilinoleyl Dimethylcarbonate Copolymer | 5.00 |
| | Red 28 Lake | Red 28 Lake | 0.05 |
| | | Perfume Fruits Rouges | 0.30 |
| | Coviox® T70C | Tocopherol | 0.10 |
| | Nipasol M | Propylparaben | 0.50 |
| II | Aerosil R972 | Silica Dimethyl Silylate | 5.30 |
| III | Ronastar Noble Spars | Calcium Aluminium Borosilicate (and) Silica (and) Titanium Dioxide | 0.25 |
| Viskosität (mPa.s): Brookfield RVT, Spindle 5, Speed 10 11 000 | | | |

**O/W Flüssig Foundation**

| | Inhaltsstoff | INCI Bezeichnung | Gew.-% |
|---|---|---|---|
| I | Deionized Water | Aqua | 50.50 |
| | Butylene Glycol | Butylene Glycol | 2.00 |
| | Glycerine | Glycerin | 3.00 |
| II | Veegum Ultra | Magnesium Aluminium Silicate | 1.00 |
| | Keltrol T | Xanthan Gum | 0.40 |
| III | Lameform® TGI | Polyglyceryl-3 Diisostearate | 1.50 |
| | Eumulgin® B2 | Ceteareth-20 | 3.50 |
| | Cutina® PES | Pentaerythrityl Distearate | 1.00 |
| | Cegesoft® SH | Shorea Stenoptera | 1.00 |
| | Cetiol® CC | Dicaprylyl Carbonate | 4.00 |
| | | Pentadecan oder Heptadecan | **2.00** |
| | Myritol® 331 | Cocoglycerides | 4.00 |
| | Fitoderm® | Squalane | 3.00 |
| | Cetiol® Sensoft | Propylheptyl Caprylate | 5.00 |
| | Phenonip | Phenoxyethanol (and) Parabens | 0.80 |
| IV | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33-8001 | Iron Oxide CI 77491 | 0.60 |
| | SunPuro Black Iron Oxide C33-7001 | Iron Oxide CI 77499 | 0.20 |
| V | Cosmedia® Gel CC | Dicaprylyl Carbonate (and) Stearalkonium | 3.00 |
| | | Hectorite (and) PropyleneCarbonate | |
| VI | Micropearl M100 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosität (mPa.s): Brk. RVT spindle 5, speed 10 5 500, pH 7.3 | | | |

**Allzweck Creme (W/O)**

| Phase | Komponente/ Handelsname | INCI | Gew.% |
|---|---|---|---|
| I. | DEHYMULS® E | Dicocoyl Pentaerhytithyl Distearyl Citrate (and) Sorbitan Sesquioleate (and) Cera Alba (Beeswax) (and) Aluminum Stearate | 3,00 |
| | DEHYMULS® PGPH | Polyglyceryl 2 Dipolyhydroxystearate | 2,00 |
| | CETIOL® OE | Dicaprylyl Ether | 5,00 |
| | CETIOL® 868 | Ethylhexyl Stearate | 5,00 |
| | MYRITOL® 331 | Cocoglycerides | 1,00 |
| | Undecan oder Tridecan | | 6,00 |
| II. | Glycerin 86% | | 5,00 |
| | MgSO₄ x 7H₂O | | 1,00 |
| | Wasser, deionisiert | | 72,00 |
| III. | Konservierungsmittel | | q.s. |

### Herstellung:

Die Komponenten der Phase I wurden bei 80 bis 85 °C geschmolzen und zur Homogenität verrührt. Die Komponenten der Phase II wurden auf 80 bis 85 °C erhitzt und langsam, unter Rühren zur Phase I zugegeben. Es wurde für weitere 5 Minuten bei dieser Temperatur gerührt. Danach wurde die Emulsion unter Rühren abgekühlt und bei 65 bis 55°C homogenisiert. Sobald die Emulsion homogen erscheint, wurde unter Rühren weiter auf 30°C abgekühlt. Danach wurden Komponenten der Phase III zugegeben und erneut gerührt.

**Balsam zur Befeuchtung und zum Schutz der Lippen**

| Phase | Komponente Handelsname | INCI | Gew.-% |
|---|---|---|---|
| I. | Cerilla Raffinée G* | Candelilla (Euphorbia Cerifera) Wax | 7,53 |
| | CUTINA® LM conc. | Polyglyceryl-2 Dipolyhydroxystearate and Octyldodecanol and Copernicia Cerifera | 6,57 |
| | | (Carnauba) Wax and Euphorbia Cerifera (Candelilla) Wax and Beeswax and Cetearyl Glucoside and Cetearyl Alcohol | |
| | Colophane claire type Y | Rosin | 1,89 |
| | Cerauba T1* | Camauba(Copernica Cerifera)Wax | 1,86 |
| | Cerabeil blanche 1* | Beeswax | 5.31 |
| | Undecan oder Tridecan | | 15.57 |
| | EUTANOL® G | Octyldodecanol | 21,71 |
| | Crodamol ML(Croda) | Myristyl Lactate | 1,13 |
| | ELESTAB®366 | | 0.43 |
| II. | Castor oil | Castor Oil | 35.00 |
| III. | IRWINOL® LS 9319 African wild mango butter | | 3.00 |

| | | | |
|---|---|---|---|
| * erhältlich von Lambert- Riviere (France) | | | |

Herstellung: Phase I wurde bei 85°C geschmolzen, Phase II wurde zugefügt und die Temperatur auf 80°C gehalten. Phase III wurde kurz vor dem Einfüllen in die Gießform (welche mit Dimethicon 50 cts befeuchtet und auf 40 °C vorgeheizt war) zugegeben. Die Masse wurde in die Gießform gegeben und auf 40 °C abgekühlt. Die Gießformen wurde im Gefrierschrank auf nahe 0 °C abgekühlt.

**Styling Wachs**

| Phase | Komponente Handelsname | INCI | Gew.- % |
|---|---|---|---|
| I. | CUTINA® MD | Glyceryl Stearate | 47,0 |
| | COMPERLAN® 100 | Cocamide MEA | 2,50 |
| | CUTINA® HR Powder | Hydrogenated Castor Oil | 2,50 |
| | PLANTACARE® 1200 UP | Lauryl Glucoside | 5,00 |
| | LANETTE® O | Cetearyl Alcohol | 7,00 |
| | CUTINA® CP | Cetyl Palmitate | 7,00 |
| | EUMULGIN® O 20 | Oleth-20 | 5,00 |
| | Undecan oder Tridecan | | 23,5 |
| | Wacker Siliconoil AK 350 | Dimethicone | 0,50 |

Die Herstellung erfolgt durch Erhitzen aller Komponenten auf 80 °C und Homogenisierung.

**Feuchtigkeitsspendende Körpermilch**

| Phase | Komponente Handelsname | INCI | Gew.-% |
|---|---|---|---|
| | EMULGADE® CM | Cetearyl Isononanoate (and) Ceteareth-20 (and) Cetearyl Alcohol (and)Glyceryl Stearate (and) Glycerin (and) Ceteareth | 5.0 |
| | EUMULGIN® VL 75 | Lauryl Glucoside (and) Polyglyceryl-2 Dipolyhydroxystearate (and) Glycerin | 2.0 |
| | CETIOL® OE | Dicaprylyl Ether | 4.0 |
| | CETIOL® J 600 | Oleyl Erucate | 1.0 |
| | ISOPROPYLMYRISTATE | Isopropyl Myristate | 7.0 |
| | Undecan oder Tridecan | | 7.0 |
| II. | Wasser, deionisiert | | ad 100 |
| III. | Cosmedia SP | Sodium Polyacrylate | 0.4 |
| IV. | HISPAGEL® 200 | Glycerin (and) Glyceryl Polyacrylate | 20.0 |
| V. | Konservierungsmittel, Parfum | | q.s. |
| | pH 5.5 | | |

Die Herstellung erfolgte durch Mischen von Phase I und Wasser bei Raumtemperatur unter Rühren. Dann wurde Phase III zugefügt und solange gerührt bis eine homogene, gequollene Mischung vorlag. Dann wurde Phase IV zugefügt, gefolgt von Phase V, danach wurde der pH Wert eingestellt.

**O/W Soft Creme**

| Phase | Komponente Handelsname | INCI | Gew.-% |
|---|---|---|---|
| I. | EMULGADE® SE-PF | Glyceryl Stearate (and) Ceteareth-20 (and) Ceteareth-12 (and) Stearyl Alcohol (and) Ceteareth-20 (and) Distearyl Ether | 6.0 |
| | LANETTE® O | Cetearyl Alcohol | 1.0 |
| | CUTINA® MD | Glyceryl Stearate | 2.0 |
| | CETIOL® MM | Myristyl Myristate | 2.0 |
| | Undecan oder Tridecan | | 8.0 |
| | Jojoba Oil | Simmondsia Chinensis (jojoba) Seed Oil | 2.0 |
| | COPHEROL® 1250 | Tocopheryl Acetate | 0.5 |
| | | Dimethicone | 0.5 |
| | | Cyclomethicone | 3.0 |
| II. | Wasser | Aqua | ad 100 |
| | | Propylene Glycol | 3.0 |
| III. | HISPAGEL® 200 | Glycerin (and) Glyceryl Polyacrylate | 15.0 |
| IV. | Konservierungsmittel | | q.s. |
| | pH 5.5-6.5 | | |

Diese Creme wurde hergestellt, indem Phase I auf 80°C erhitzt wurde, Phase II wurde ebenfalls auf 80°C erhitzt und zu Phase I unter Rühren hinzugefügt. Diese Mischung wurde unter Rühren abgekühlt und bei ca. 55°C mit einem geeigneten Dispersionsgerät (z.B. Ultra Turrax) homogenisiert. Danach wurde Phase III unter kontinuierlichem Rühren hinzugefügt, Phase IV wurde zugegeben und der pH-Wert eingestellt.

**W/O Creme**

| Phase | Komponente / Handelsname | INCI | Gew.-% |
|---|---|---|---|
| I. | MONOMULS® 90 O 18 | Glyceryl Oleate | 2,00 |
| | LAMEFORM® TGI | Polyglyceryl 3 Diisostearate | 4,00 |
| | CETIOL® A | Hexyl Laurate | 12,00 |
| | Undecan oder Tridecan | | 12,00 |
| | SIPOL® C 16/18 OR | Cetearyl Alcohol | 1,00 |
| | Zinc stearate | Zinc Stearate | 2,00 |
| | Zinc Oxide | CI 77947 (or) Zinc Oxide | 15,00 |
| | Magnesium Sulphate | Magnesium Sulphate | 1,00 |
| | Glycerin | Glycerin | 3,00 |
| | Konservierungsmittel | | q.s. |
| | Benzyl Alcohol | Benzyl Alcohol | 0,40 |
| | HYDAGEN® B | Bisabolol | 0,50 |
| | Wasser | aqua | 100,00 |

Die ersten 7 Komponenten wurden bei 85°C geschmolzen. Magnesium Sulfate und Glycerin wurden im Wasser gelöst und diese Mischung wurde auf 85 °C erhitzt. Diese wässrige Phase wurde zur Ölphase gegeben und dispergiert. Unter kontinuierlichem Rühren wurde bis auf 40°C abgekühlt und dann wurden Benzyl Alkohol und Hydagen B gemischt und zu der Emulsion gegeben. Unter weiterem Rühren wurde bis auf 30°C abgekühlt und homogenisiert.

**"Body Wash" Reinigungsemulsion**

| Phase | Komponente Handelsname | INCI | Gew.-% |
|---|---|---|---|
| I | Texapon ALS-IS | Ammonium Lauryl Sulfate | 30,00 |
| | TEXAPON® NSO | Sodium Laureth Sulfate | 18,00 |
| | Undecan oder Tridecan | | 18,00 |
| | Plantacare® 1200 | Lauryl Glucoside | 8,00 |
| II | Jaguar HP 105 | Hydroxypopyl Guar | 2,00 |
| | Euxyl K400 | MethyldibromoGlutaronitrile and Phenoxyethanol | 0,10 |
| | Wasser | Aqua | 23,90 |
| | pH-value | 5,6 | |

### Tabelle 19: O/W-Sonnenschutzemulsionen

Die im Folgenden genannten Beispiele enthalten alle das gemäß Herstellbeispiel 1 erhaltene Undecan oder Tridecan Gemisch. Alle Angaben sind in Gew.-%.

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme,** | **L** | **C** | **S** | **L** | **C** | **L** | **L** | **C** | **L** | **C** | **L** |
| Eumulgin^{®} VL 75 | | | | | | 4 | 4 | 2 | | | |
| Eumulizin^{®} B2 | 2 | | | | | | | | | | |
| Tween^{®} 60 | | | | 1 | | | | | | | |
| Myrj^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®}O | 1 | 6 | | | 5 | 2 | | 2 | | | |
| Antaron V 216 | | | 1 | | 2 | 2 | | | | 1 | |
| Lanolin, wasserfrei USP | | | | | | | 5 | | | | |
| Undecan oder Tridecan | 2 | 2 | 4 | 1 | 2 | 2 | 2 | 1 | 2 | 2 | 1 |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Silikonöl Wacker AK^{®} 350 | | 2 | | | | | | | | | |
| Cetiol^{®} 868 | | | | | 2 | | 8 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | 1 | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra Keltrol^{®} T | | | 0.75 | | | | | 1 | 1 | | |
| | | | 0.25 | | | | | 0.5 | 0.5 | | |
| Carbopol^{®} 980 Ethanol | | 0.5 | | 0.2 | 0.2 | 0.2 | | 0.5 | 0.1 | 0.3 | 0.2 |
| | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | Ad 100 | | | | | | | | | | |

**Tabelle 20: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme,** | **L** | **L** | **L** | **C** | **L** | **C** | **S** | **C** | **C** | **L** | **L** |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | 0.5 | |
| Lanette^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} O | | | | 2 | | | | | 1 | 1 | |
| Antaron V 220 | 1 | | | 2 | | | 0.5 | | | 2 | 0.5 |
| Undecan oder Tridecan | 4 | 2 | 4 | 6 | 10 | 4 | 2 | 8 | 2 | 1 | 3 |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | 5 | | 3 | | | | | |
| Eutanol^{®} G 16 S | 10 | | | | | | | | | | |
| Cetiol^{®} PGL | | | | 5 | | | | | 2 | | |
| Photonyl^{®} LS | | | | | | | | | | 2 | |
| Panthenol | | | | | | 1 | | | | | |
| Bisabolol | | | | | | 0,2 | | | | | |
| Tocopherol / Tocopherylacetat | | | | | | 1 | | | | | |
| Neo Heliopan^{®} Hydro (Na-Salz) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 21: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| **L = Lotion; C = Creme** | **C** | **L** | **C** | **L** | **C** | **L** | **L** | **L** | **L** | **C** | **C** |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Bienenwachs | 1 | | 2 | 1 | | 1 | | 3 | | 2 | 3 |
| Tego^{®} Care CG | | | | | 1 | | | | | | 5 |
| Prisorine^{®} 3505 | 1 | | 1 | 1 | | 1 | 1 | | | | 1 |
| Wollwachsalkohol, wasserfrei, | | | | | | | | | | | 1 |
| Antaron V 216 | 2 | | | | | | | | | | |
| Undecan oder Tridecan | 3 | 4 | 2 | 1 | 10 | 2 | 2 | 6 | 3 | 12 | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 244 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G | | | 3 | | 2 | | | 8 | | | |
| Cetiol^{®} PGL | | 11 | | | 2 | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 22: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion; C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 2 | 4 | 0.5 | 1 | 4 |
| Monomuls^{®} 90-018 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Bienenwachs | | 1 | | 2 | 2 | | 3 | | 2 | 1 | 1 |
| Tego^{®} Care CG | | | | | | | | | | | |
| Isostearinsäure | 1 | 1 | | | | | 1 | 1 | | 1 | 1 |
| Wollwachsalkohol, wasserfrei | | | | | | | | | | | |
| Antaron V 220 | | 0.5 | 2 | 1 | 1 | 1 | | | | | |
| Undecan oder Tridecan | 2 | 4 | 3 | 3 | 2 | 2 | 1 | 3 | 3 | 1 | 4 |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 244 | | 4 | | 2 | | | | | | | |
| Dow Corning DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Cetiol^{®} B | | | | | 3 | 3 | | 2 | 2 | | |
| Eutanol^{®} G | | | | 2 | | | 4 | | 5 | | |
| Cetiol^{®} PGL | | | | | | | 5 | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan^{®} MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote^{®} HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 23: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| **L = Lotion. C = Creme** | **C** | **L** | **C** | **L** | **C** | **L** | **L** | **L** | **C** | **C** | **C** |
| Dehymuls^{®} PGPH | 1 | 3 | 1 | 2 | 3 | 1 | 1 | 2 | 1 | 1 | 1 |
| Monomuls^{®} 90-018 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Bienenwachs | 2 | | 3 | 1 | | 1 | | 1 | 3 | 2 | 1 |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Lanolin; anhydrous USP | | | 5 | | | | | | | 4 | |
| Undecan oder Tridecan | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | 8 |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |

**Fortsetzung Tabelle 23: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| Cetiol^{®} 868 | | | | | | | | | | 2 | |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G 16 | | 1 | | | 1 | | | | | 3 | 7 |
| Eutanol^{®} G | | | 3 | | 1 | | | 8 | | | |
| Cetiol^{®} PGL | | | | | | 4 | | | 9 | | |
| Mandelöl | | | | | 3 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Aluminum chlorohydrate | | 8 | | | | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 24: W/O-Pflegeemulsionen**

| **Komponente** | **56** | **57** | **58** | **59** | **60** | **61** | **62** | **63** | **64** | **65** | **66** |
|---|---|---|---|---|---|---|---|---|---|---|---|
| **L = Lotion. C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 3 | 4 | 1 | 1 | 1 |
| Monomuls^{®} 90-018 | | 1 | | 1 | | | | | | | |
| Lameform^{®} TGI | | | | | 4 | | | 1 | | 3 | |
| Abil^{®} EM90 | | | | 3 | | | | | | 2 | |
| Isolan^{®} PDI | | 3 | | | | | | | | | 4 |
| Glucate^{®} DO | 1 | | | | | | | | | | |
| Arlacel^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Bienenwachs | | 4 | | 2 | 1 | 2 | 2 | 1 | 2 | 3 | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emeryl^{®} 1780 | | | | | | | | | | | |
| Lanolin anhydrous USP | | 7 | 3 | | | | | | | | |
| Undecan oder Tridecan | 3 | 4 | 4 | 8 | 10 | 2 | 8 | 6 | 3 | 12 | 7 |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Corning ^{®} DC 244 | | 4 | | 2 | | 2 | | | | | |
| Dow Corning^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol^{®} J 600 | 4 | | | 3 | | | | | | | |
| Ceraphyl^{®} 45 | | | | 1 | | | | | 5 | 4 | |
| Cetiol^{®} B | | | | | 3 | 3 | | 2 | 2 | | |
| Eutanol^{®} G 16 | 1 | | | | | | 3 | | | | |
| Eutanol^{®} G | | | | 2 | | | 2 | | 5 | | |
| Cetiol^{®} PGL | | | 10 | | | | 4 | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 25: O/W-Pflegeemulsionen**

| **Komponente** | **67** | **68** | **69** | **70** | **71** | **72** | **73** | **74** | **75** | **76** | **77** |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| Eumulgin^{®} VL 75 | | | | | | 4 | | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E 24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®} AB | | 1 | | | | | | | | | 1 |
| Lanolin, wasserfrei, USP | | | | | | | 5 | | | | |
| Cetiol^{®} SB 45 | | | 1.5 | | | | 2 | | | | |
| Undecan oder Tridecan | 3 | 4 | 2 | 1 | 10 | 2 | 2 | 6 | 3 | 12 | 1 |
| Cegesoft^{®} C 17 | | | | | | | | | | | |
| Myritol^{®} PC | | | | | 5 | | | | | | |
| Myritol^{®} 331 | 2 | 5 | | | | 6 | | 12 | | | |
| Finsolv^{®} TN | | | | | | 2 | | | 8 | | |
| Cetiol^{®} CC | 4 | 6 | | | | 4 | 4 | | | | 5 |
| Cetiol^{®} OE | | | 7 | | | | | | 4 | 3 | |
| Dow Corning DC^{®} 245 | | | 2 | | 5 | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |

**Fortsetzung Tabelle 25: O/W-Pflegeemulsionen**

| Komponente | 67 | 68 | 69 | 70 | 71 | 72 | 73 | 74 | 75 | 76 | 77 |
|---|---|---|---|---|---|---|---|---|---|---|---|
| Ceraphyl^{®} 45 | | | | | | | 3 | | | | |
| Cetiol^{®} SN | | | 5 | | | | | | | | |
| Cetiol^{®} B | | | | | | | | | | 2 | |
| Eutanol^{®} G | | 2 | | 5 | | | | | | | |
| Cetiol^{®} PGL | | | | 7 | | | | | 5 | 5 | |
| Dry Flo ^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | | 0.3 | | | |
| Carbopol^{®} Ultrez 10 | | | 0.3 | 0.2 | | 0.2 | | | 0.1 | 0.3 | |
| Cosmedia SP | | 0,3 | | | 0.2 | | | | | | 0.2 |
| Aluminum Chlorohydrate | | | 7 | | | | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s.,pH 6,5 - 7,5 | | | | | | | | | | |

**Tabelle 26: O/W-Pflegeemulsionen**

| **Komponente** | **78** | **79** | **80** | **81** | **82** | **83** | **84** | **85** | **86** | **87** | **88** |
|---|---|---|---|---|---|---|---|---|---|---|---|
| **L = Lotion, C = Creme** | **C** | **C** | **L** | **C** | **L** | **C** | **L** | **L** | **L** | **L** | **C** |
| Eumulgin^{®} VL 75 | 4 | 3 | | | | | 1 | | | | 2 |
| Generol^{®} R | | | | | | 2 | | | | | |
| Eumulgin^{®} B2 | | | | | | 2 | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | | |
| Lanette^{®} E | 0.5 | | | | | | | | | | 1 |
| Amphisol^{®} K | 0.5 | 1 | | | | | | 1 | 1 | | |
| Natriumstearat | | | | | 1 | | | | | | |
| Emulgade^{®} PL 68/50 | | 6 | | | | | | 5 | | | 4 |
| Tego^{®} Care CG | | | | | | | | | | | |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{®} O | 2 | | | 2 | | 3 | 1 | | 1 | 1 | 6 |
| Novata^{®} AB | | | | | | | | | | | |
| Lanolin, wasserfrei, USP | | | | | | 4 | | | | | |
| Cetiol^{®} SB 45 | | | | | | | 2 | | | | |
| Undecan oder Tridecan | 3 | 4 | 2 | 1 | 10 | 2 | 2 | 6 | 3 | 12 | 1 |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | | 5 | | | 3 | 3 | | 1 |
| Cetiol^{®} CC | | | | | | | | | | | 2 |
| Cetiol^{®} OE | | | | | 2 | | 2 | | 5 | | |
| Dow Corning DC^{®} 245 | | 2 | | | 1 | | | | | 8 | 2 |
| Dow Corning DC^{®} 2502 | | 1 | | | 1 | | | | | | 3 |

**Fortsetzung Tabelle 26: O/W-Pflegeemulsionen**

| **Komponente** | **78** | **79** | **80** | **81** | **82** | **83** | **84** | **85** | **86** | **87** | **88** |
|---|---|---|---|---|---|---|---|---|---|---|---|
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

**Tabelle 27: Sprayformulierungen**

| **Komponente** | **89** | **90** | **91** | **92** | **93** | **94** | **95** | **96** | **97** | **98** | **99** |
|---|---|---|---|---|---|---|---|---|---|---|---|
| **S = Körperspray, S* =** Sonnenschutzspray | **S** | **S** | **S** | **S** | **S** | **S*** | **S*** | **S*** | **S*** | **S*** | **S*** |
| Emulgade^{®} SE-PF | 8,9 | | 7,5 | 7.5 | 4,3 | 9.8 | 8.2 | 9.9 | | | |
| Eumulgin^{®} B2 | 3,1 | | 3 | | | | | 4.2 | | | |
| Eumulgin^{®} B3 | | | | | | 4.2 | 3.3 | | | | |
| Eumulgin^{®} HRE 40 | | | | | 4,7 | | | | | | |
| Cutina^{®} E 24 | | 5,9 | | 4 | | | | | | | |
| Amphisol^{®} K | | | | | | | | | 1 | 1 | 1 |
| Eumulgin^{®} VL 75 | | | | | | | | | | | 2 |
| Emulgade^{®} PL 68/50 | | 0.5 | | | | | | | 2.5 | 1 | |
| Cutina^{®} MD | | 3,1 | | | | | | | | | |
| Antaron V 220 | | | | | | 1 | 1 | 1 | | 1 | 1 |
| Undecan oder Tridecan | 11 | 5 | 7 | 7 | 7 | 5 | 4 | 5 | 5 | 4 | 6 |
| Myritol^{®} PC | | | | | | | | | | | |
| Myritol^{®} 331 | | | 3 | 4 | 3 | 3 | 3 | 3 | | | |
| Finsolv^{®} TN | | 4 | | | | | | 8 | | | |
| Cetiol^{®} CC | 6 | | | 5 | 5 | 2 | 2 | 4 | | | |
| Cetiol^{®} OE | | 5 | 7 | | | 2 | | | | | |
| Dow Corning DC^{®} 244 | | 4 | 4 | 5 | | | | | | | |
| Cetiol^{®} 868 | 3 | | | | | | | | | | |
| Cetiol^{®} J 600 | | | | 2 | 2 | | | | | | |
| Cetiol^{®} B | | | | | | | 2 | | | | |
| Eutanol^{®} G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan^{®} MBC | | | | | | 2 | 2 | 2 | | 1 | 1 |
| Neo Heliopan^{®} OS | | | | | | 5 | | | | | |
| Neo Heliopan^{®} AV | | | | | | 6 | 6 | 2 | | 7.5 | 2 |
| Uvinul^{®} T 150 | | | | | | 1 | 1 | 1 | | 1 | |
| Parsol^{®} 1789 | | | | | | 1 | | 1 | | 1 | |
| Z-Cote^{®} HP 1 | | | | | | | | | | 2 | 2 |
| Eusolex^{®} T 2000 | | | | | | | | | | 2 | 2 |
| Veegum^{®} Ultra | | | | | | | | | | | 1.5 |
| Laponite^{®}XLG | | | | | | | | | | 1.5 | |
| Keltrol^{®} T | | | | | | | | | | | 0.5 |
| Pemulen^{®} TR 2 | | | | | | | | | 0.2 | | |
| Insect Repellent^{®} 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 100/ q.s./ q.s | | | | | | | | | | |

**Tabelle 28A: Antiperspirant Suspensionsstifte sowie Soft Solids - Mengenangaben in Gew.-% -**

| **Zusammensetzung** | **1** | **2** | **3** | **4** | **5** | **6** |
|---|---|---|---|---|---|---|
| Distearylether | - | 15 | - | - | - | - |
| Stearylalkohol | - | - | 15 | 10 | 14,7 | - |
| Guerbetalkohol C36 | 15 | - | - | - | - | - |
| Tribehenin | - | - | - | - | - | 20 |
| Hydrogenated Castor Oil | - | - | 4 | - | 3,7 | - |
| Undecan oder Tridecan | 30 | 60 | 51 | 60 | 31,6 | 55 |
| Octyldodecanol | 5 | - | - | - | - | - |
| Dicaprylylether | 5 | - | - | - | - | - |
| Hexyldecanol + Hexyldecyl - Laurate | | 10 | - | - | - | - |
| Cyclomethicone | 20 | - | - | - | 30 | - |
| Dry Flo Plus* | - | - | 5 | - | - | - |
| Silica | - | - | - | 2,5 | - | - |
| Talc | - | - | - | 2,5 | - | - |
| Aluminium Zirconium Tetrachlorohydrex GLY | - | - | 25 | 25 | - | 25 |
| Aluminium Chlorohydrate | 25 | 15 | - | - | 20 | - |

| | | | | | | |
|---|---|---|---|---|---|---|
| * National Starch | | | | | | |

**Tabelle 28B: Antiperspirant Suspensionsstifte sowie Soft Solids - Mengenangaben in Gew.-%**

| **Zusammensetzung** | **7** | **8** |
|---|---|---|
| 12-Hydroxystearinsäure | 10 | 5 |
| Undecan oder Tridecan | 65 | 65 |
| Dry Flo Plus* | - | 5 |
| Aluminium Zirconium Tetrachlorohydrex GLY | 25 | 25 |

| | | |
|---|---|---|
| * National Starch | | |

**Tabelle 29: Rezepturen 1 bis 13**

| **Komponenten INCI** **(Handelsname)** | **1** | **2** | **3** | **4** | **5** | **6** | **7** | **8** | **9** | **10** | **11** | **12** | **13** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | | | | | | | | | | | | 10.7 | 5.1 |
| Ceteareth-20 (Eumulgin^{®} B2) | | | | | | | | | | | | 5.8 | 3.4 |
| Cetearyl Glucoside, Cetearyl Alcohol (Emulgade^{®} PL 68/50) | 1 | | 1 | 1 | | 2 | 2 | | 2 | | 2 | | |
| Polyglyceryl-2 Dipolyhydroxystearate, Lauryl Glucoside, Glycerin (Eumulgin^{®} VL 75) | | 1 | | | 1 | | | 3 | | 2.5 | | | |
| Sodium Cetearyl Sulfate (Lanette^{®} E) | 1 | 1 | 1 | | 1 | | | | 1 | 1 | | | |
| Undecan oder Tridecan | 5 | 4 | 8 | 3 | 5 | 8 | 4 | 2 | 4 | 3 | 5 | 10 | 2 |
| Dicaprylyl Carbonate (Cetiol^{®} CC) | 5 | 5 | 5 | | | | | 4 | | 5 | 3 | 4 | |
| Cocoglycerides (Myritol^{®} 331) | 3 | 4 | | 4 | 4 | | | | 5 | | | 3 | 3 |
| Dicaprylyl Ether (Cetiol^{®} OE) | | | | | 5 | | 3 | | 2 | | | | |
| Dibutyl Adipate (Cetiol^{®} B) | | | | 4 | | | | 4 | | 4 | | | |
| Hydrogenated Dimer Dilinoleyl/Dimethylcarbonat e Copolymer (Cosmedia^{®} DC) | 1 | 1 | 1 | 1 | 1 | 1.5 | 1.5 | 2 | 3 | 2 | 1.5 | 2 | 2 |
| Ethyl Butylacetylaminopropionate | | | | | | | | | | | 5 | 5 | |
| Tocopherol | 1 | 1 | 1 | 1 | 1 | 1 | 1 | 1 | 1 | 1 | 1 | 1 | 1 |
| Zinc oxide, nanonisiert, gecoated | 5 | 5 | 5 | 5 | 5 | | 2 | | 5 | 3 | | | |
| Titanium dioxide, nanonisiert, Gecoated | | | | 5 | 5 | | 2 | 3 | 5 | 2 | | | |
| Ethyl hexyl Methoxycinnamate | 7.5 | 7.5 | 7.5 | | | | 3 | 1 | 3 | 5 | | 5 | 5 |
| Octocrylene | 9 | 9 | 9 | | | 2 | 1 | | | | 2 | | 1.5 5 |
| Butyl Methoxydibenzoylmethane | | | | | | 2 | 2 | | | 1 | 2 | 2 | |
| 4-Methylbenzylidene Camphor | | | | | | | 2 | | | | | | 2 |
| Ethylhexyl Triazone | | | | | | 1 | 1 | 2 | | | 1 | | |
| Diethylhexyl Butamido Triazone | | | | | | 1 | 1 | 2 | | | 1 | 2 | |
| Phenylbenzimidazole Sulfonic Acid als Na-Salz, 15% wäßrige Lsg. | | | | | | | | | | | | | 13.3 |
| Glycerin | 3 | 3 | 3 | 3 | 3 | 3 | 3 | 3 | 3 | 3 | 3 | 5 | 5 |
| Magnesium aluminium silicate (and) cellulose Gum | 0.75 | 0.75 | | 0.5 | 0.5 | | 0.5 | | | 0.35 | | | |
| Xanthan Gum | 0.25 | 0.25 | | 0.5 | 0.5 | | 0.5 | | | 0.35 | | | |
| Sodium Polyacrylate (Cosmedia^{®} SP) | | | 0.1 | | | 0.1 | 0.2 | | | | 0.1 | | |
| Acrylates/C10-30 Alkyl Acrylate Crosspolymer | | | | | | | | 0.2 | 0.1 | | | | |
| Wasser, Perfume, Preservatives | q.s. | q.s. | q.s. | q.s. | q.s. | q.s. | q.s. | q.s. | q.s. | q.s. | q.s. | q.s. | q.s. |

**Tabelle 30: Rezepturen 14 bis 26**

| **Komponenten INCI** **(Handelsname)** | **14** | **15** | **16** | **17** | **18** | **19** | **21** | **21** | **22** | **23** | **24** | **25** | **26** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | 3.7 | 3.7 | | | | | | | | | | 4.9 | 4.1 |
| Ceteareth-12 (Eumulgin^{®}B1) | 1.3 | 1.3 | | | | | | | | | | | |
| Ceteareth-20 (Eumulgin^{®}B2) | | | | | | | | | | | | 1.1 | 0.9 |
| Cetearyl Glucoside, Cetearyl Alcohol (Emulgade^{®} PL 68/50) | | | 5 | 1 | 1 | 1 | 1 | 3 | | | | | |
| Polyglyceryl-2 Dipolyhydroxystearate, Lauryl Glucoside, Glycerin (Eumulgin^{®} VL 75) | | | | | | | | | 3 | 5 | 5 | | |
| Sodium Cetearyl Sulfate (Lanette^{®} E) | | | | 0.25 | 0.25 | 0.25 | 0.25 | 0.25 | | | | | |
| Potassium Cetyl Phosphate | | | 0.5 | | | | | | | | | | |
| Undecan oder Tridecan | 4 | 5 | 6 | 8 | 5 | 8 | 8 | 10 | 7 | 4 | 10 | 5 | 5 |
| Dicaprylyl Carbonate (Cetiol^{®} CC) | 5 | | 5 | | | | | | 2.5 | 4 | 4 | 5 | 5 |
| Coco-Caprylate/Caprate (Cetiol^{®} LC) | | | | 1 | 1 | 1 | 1 | 1 | | | | | |
| Caprylic/Capric Triglyceride (Myritol^{®} 312) | | | | 1 | 1 | 1 | 1 | 1 | | | | | |
| Cocoglycerides (Myritol^{®} 331) | | | | | | | | | | 4 | 4 | | |
| Cetearyl Isononanoate (Cetiol^{®} SN) | 3 | 3 | 3.5 | | | | | | | | | | |
| Octyldodecanol (Eutanol^{®} G) | | | | | | | | | 3.5 | 2 | 2 | | |
| Hexyldecanol (Eutanol^{®} G16) | | | | 1 | 1 | 1 | 1 | 1 | | | | | |
| Olus (Cegesoft^{®} PS6) | | 1.5 | 1.5 | | | | | | | | | | |
| Passiflora Incarnata (Cegesoft^{®} PFO) | 1.5 | | | | | | | | | | | | |
| Dimethicone | | | | 0.5 | 0.5 | 0.5 | 0.5 | 0.5 | | | | | |
| Dimer Distearyltricarbonate (Cosmedia^{®} DC) | 1 | | 1.5 | | | | | 1.5 | | 2,5 | 2,5 | | 0.5 |
| Triethyl Citrate (Hydagen^{®} C.A.T) | | | | | | | | | | | | 1.5 | |
| Tocopherol | | | | | | | | | 0.5 | 0.5 | 0.5 | | |
| Tocopheryl Acetate | 0.5 | 0.5 | | | | | | | | | | | |
| Ethanol | | | | | | | | | | | 5 | | |
| Aluminum Chlorhydrate (Locron L) | | | | | | | | | | | | | 40 |
| Chitosan (Hydagen^{®} DCMF) | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 0.3 | 0.5 | | |
| Glycerin, Glyceryl Polyacrylate (Hispagel^{®} 50) | | | | | | | | | | 10 | | | |
| Carbomer | | | | | | | 0.1 | | 0.2 | | 0.2 | | |
| Sodium Polyacrylate (Cosmedia^{®} SP) | | | | 0.15 | | | | | | | | | |
| Acrylates/C10-30 Alkyl Acrylate Crosspolymer | | | | | | 0.15 | | 0.05 | | | | | |
| Wasser, Perfume, Preservatives | q.s | q.s. | q.s. | q.s. | q.s. | q.s. | q.s. | q.s. | q.s. | q.s. | q.s. | q.s. | |

**Tabelle 31: Rezepturen 27 bis 33 (Formulierungen für AP/Deo)**

| **Komponenten INCI (Handelsname)** | **27** | **28** | **29** | **30** | **31** | **32** | **33** |
|---|---|---|---|---|---|---|---|
| Glyceryl Stearate, Ceteareth-20, Ceteareth-12, Cetearyl Alcohol, Cetyl Palmitate (Emulgade^{®} SE) | 6 | | 4,5 | | | 6 | |
| Ceteareth-20 (Eumulgin^{®}B2) | | | 1 | | | | |
| Cetearyl Isononanoate, Glyceryl Stearate, Ceteareth-20, Ceteareth-12, Cetearyl Alcohol, Cetyl Palmitate (Emulgade^{®} CM) | | | | | 20 | | |
| Polyglyceryl-3 Diisostearate Lameform TGI | | 3 | | | | | |
| Cocoglycerides (Novata^{®} AB) | | | | | | | 4 |
| Stearyl alcohol ( Lanette 18) | | | | 14,7 | | | |
| Hydrogenated Castor Oil (Cutin^{®} HR) | | | | 3,7 | | | 6,5 |
| Polyglyceryl-2 Dipolyhydroxystearate, (Dehymuls^{®} PGPH) | | 1 | | | | | |
| Sodium Cetearyl Sulfate (Lanette^{®} E) | 0,3 | | | | | 0,3 | |
| Behenyl Alcohol (Lanette^{®} 22) | 2 | | | | | 4 | |
| Undecan oder Tridecan | 4 | 4 | 5 | 5 | 4 | 4 | 15 |
| Dicaprylyl Carbonate (Cetiol^{®} CC) | | 3 | | | | | |
| Dicaprylylether ( Cetiol^{®} OE) | 2 | | | 4 | | 3 | 9 |
| Cocoglycerides (Myritol^{®} 331) | | | | | | | |
| Cyclopentasiloxane | 3 | 5 | | 34 | | 2 | 14 |
| Cyclopentasiloxane and Dimethicone/Vinyldimethicone Crosspolymer SFE 839 (GE Bayer) | | 3 | | | | | |
| Dimethicone | 1 | | | | | | |
| Dimer Distearyltricarbonate (Cosmedia^{®} DC) | 1 | 1 | 1 | 1 | 1 | 1 | 1 |
| Triethyl Citrate (Hydagen^{®} C.A.T) | | | 2 | | | | |
| PEG-40 Hydrogenated Castor Oil | | | | | 1 | | |
| Tocopheryl Acetate | | | | 1 | | | |
| Aluminium Zirconium Tetrachlorohydrex GLY (Rezal 36) | 30 | 40 | | 22,9 | | 30 | 25 |
| Aluminum Chlorhydrate (Locron L) | | | 10 | | | | |
| Chitosan (Hydagen^{®} DCMF) | 0,05 | | | | | | |
| Glycolic Acid | 0,02 | | | | | | |
| Glycerin | | 5 | 5 | | | | |
| Propylene Carbonate ( Fluka) | | | | | | | 0,5 |
| Quaternium-18 Hectorite (Bentone 18) | | | | | | | 1 |
| Talc (Merck) | | | | | | 5 | 5 |
| MgSO4x 7H2O | | 1 | | | | | |
| Wasser Phase II | 46,7 | | 35 | | | | |
| Wasser, Perfume, Preservatives | q.s. | q.s. | q.s. | q.s. | q.s. | q.s. | q.s. |

| | | | | | | | |
|---|---|---|---|---|---|---|---|
| 27 - Antiperspirant / Deo Creme / 28 - Antiperspirant Creme (W/0) / 29 - Antiperspirant / Deo Spray 30 - Antiperspirant Stift mit Vitamin E / 31 - Deodorant Wipe - Formulierung / 32 - Antiperspirant Creme 33 - Antiperspirant Creme «Soft Solid » | | | | | | | |

In der Tabelle 32 werden Sonnenschutzformulierungen vom Typ O/W beschrieben, in der Tabelle 33 werden Pflegeemulsionen beschrieben. Durch den Einsatz des erfindungsgemäßen Kohlenwasserstoffs wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 32: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme, S = Spray** | L | C | L | C | L | C | S | C | C | L | L |
| Eumulgin^{®} VL 75 | 2 | | | | 3 | | | | 1 | | |
| Eumulgin^{®} B2 | | | | 2 | | | | | | 1 | |
| Tween^{®} 60 | | | | | | | | | | 1 | |
| Cutina^{®} E 24 | | | | 0.5 | | | | | | | |
| Hostaphat^{®} KL 340 N | | | | | | | | | | | 0.5 |
| Eumulgin^{®} SG | | | 0,5 | | | 0,5 | | 0,3 | 0,1 | | |
| Lanette^{®} E | | | | | | | 0.1 | | 0.5 | | |
| Amphisol^{®} K | 0.5 | | | | | | 1 | | | | |
| Natriumstearat | | | | | 1 | | | | | | |
| Emulgade^{®} PL 68/50 | | 2 | 1 | | | 3 | | | | | |
| Tego^{®} Care 450 | | 2 | | | | | | | 2 | | |
| Cutina^{®} MD | | | | 2 | 1 | 3 | | | | | 1 |
| Lanette^{®} 14 | | 1 | | | | | | | | | |
| Lanette^{®} O | | | | 2 | | | | 2 | 1 | 1 | |
| Cutina^{®} PES | 1 | 1 | | 2 | | | | | | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Lanolin, wasserfrei, USP | | | | | | 1 | 1 | | | | |
| Undecan oder Tridecan | 6 | 2 | 4 | 7 | 3 | 7 | 6 | 6 | 4 | 4 | 5 |
| Myritol^{®} PC | | | | | | | | | 5 | | |
| Myritol^{®} 331 | 6 | | 4 | | | 5 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | | | 3 | | | | | |
| Eutanol^{®} G 16 S | 3 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | 2 | | | 2 | | 2 | | | | |
| Tinosorb^{®} S | | 1 | | | 2 | | 2 | | | | |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 33: O/W-Pflegeemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion, C = Creme** | C | C | L | C | L | C | L | L | L | L | C |
| Eumulgin^{®} VL 75 | | | 5 | | 4 | | | | | | 2 |
| Generol^{®} R | | | | | | 2 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Eumulgin^{®} SG | | | 0,1 | 0,5 | | 0,4 | | 0,2 | 0,1 | | |
| Lanette^{®} E | 0.5 | | | | | | | | | | |
| Amphisol^{®} K | 0,5 | 0.5 | | | | | | | | | |
| Natriumstearat | | | | | 1 | | | | | | |
| Emulgade^{®} PL 68/50 | | 2 | | 2 | | | | 3 | 4 | | |
| Tego^{®} Care 450 | | 1 | | | | | | | 1 | | |
| Cutina^{®} MD | 2 | 1 | 1 | 1 | | 5 | | | | 2 | |
| Lanette^{®} 14 | | | | | 1 | | | 2 | | 1 | |
| Lanette^{®} O | 2 | | | 2 | 1 | 3 | 1 | | 1 | 1 | 3 |
| Cutina^{®} PES | 1 | 2 | | 3 | 1 | | | | | | 3 |
| Novata^{®} AB | | | | | | | | | 1 | 1 | |
| Lanolin, wasserfrei, USP | | | | | | 4 | | | | | |
| Cosmedia^{®} DC | | | 2 | | | 1.5 | | | 1 | 1 | |
| Cetiol^{®} SB 45 | | | | | | | 2 | | | | |
| Cegesoft^{®} C 17 | 2 | | | | | | | | | | |
| Undecan oder Tridecan | 5 | 5 | 4 | 4 | 3 | 4 | 5 | 4 | 5 | 10 | 2 |
| Myritol^{®} PC | 6 | | | | | 5 | | | | | |
| Myritol^{®} 331 | 2 | | 5 | | | | 2 | | | | 3 |
| Finsolv^{®} TN | | | | 3 | 5 | | | 3 | 3 | | 1 |
| Cetiol^{®} CC | | | | 3 | | | 4 | 3 | | | |
| Cetiol^{®} OE | | | | | 2 | | 2 | | 5 | | |
| Dow Corning DC^{®} 245 | | 2 | | | 1 | 4 | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.5 | | | | | 0.5 | 0.5 | 0.2 | | | 0.5 |
| Carbopol^{®} ETD 2001 | | 0.3 | | 0.3 | | | | | | | |
| Pemulen^{®} TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

### Formulierungen für den Sonnenschutz und die Hautpflege vom Typ Wasser in Öl

In Tabelle 34 werden Sonnenschutzformulierungen vom W/O Emulsionstyp, in Tabelle 17 werden Pflegeemulsionen beschrieben. Durch den Einsatz des Undecan oder Tridecan wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 34: W/O - Sonnenschutzformulierungen**

| **Ingredient** | **1** | **2** | **3** | **4** | **5** | **6** | **7** | **8** | **9** | **10** | **11** |
|---|---|---|---|---|---|---|---|---|---|---|---|
| **L = Lotion; C = Creme** | C | L | C | L | C | L | L | L | L | C | C |
| Dehymuls^{®} PGPH | 4 | 2 | 1 | 3 | 3 | 1 | 1 | 2 | 2 | 4 | 1 |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego^{®} Care CG | | | | | 1 | | | | | | 0.5 |
| Prisorine^{®} 3505 | | | 1 | | | 1 | 1 | | | | 1 |
| Cosmedia DC | 3 | 4 | 2 | 1 | 1 | 2 | 2 | 2 | 3 | 1 | 1 |
| Undecan oder Tridecan | 5 | 4 | 4 | 3 | 2 | 4 | 3 | 4 | 2 | 3 | 5 |
| Myritol^{®} 331 | 2 | | | | 3 | 6 | | | | | 3 |
| Finsolv^{®} TN | | | | 5 | | | 2 | | | | |
| Cetiol^{®} CC | 5 | | 2 | | 4 | 2 | | | 2 | 3 | 5 |
| Tegosoft DEC | | 4 | | 3 | | | 5 | 5 | | | |
| Cetiol^{®} OE | | | | | 4 | | 5 | | 4 | 2 | |
| Dow Corning^{®} DC 244 | | | 3 | | | | 2 | | 2 | 4 | |
| Dow Corning^{®} DC 2502 | 1 | | 1 | | 2 | 1 | | | | | 1 |
| Silikonöl Wacker AK 350 | | 1 | | 4 | | | | 3 | | | |
| Cetiofl^{®} PGL | | 3 | | | | 2 | | | 4 | | |
| Cophero^{®} F 1300 | | | | | | 1 | | | | | |
| Magnesium sulfat x 7 H₂O Neo Heliopan^{®} Hydro (Na-Salz) Neo Heliopan^{®} 303 | | | | | | 1 | | | | | |
| | 2 | | 2.2 | | 3 | 3 | | | 1 | | 2 |
| | | 5 | | | | | | | 4 | | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A plus | | | | | 2 | | | | 3 | 3 | |
| Neo Heliopan^{®} AP (Na-Salz) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

**Tabelle 35: W/O-Pflegeemulsionen**

| **Komponente** | **55** | **56** | **57** | **58** | **59** | **60** | **61** | **62** | **63** | **64** | **65** |
|---|---|---|---|---|---|---|---|---|---|---|---|
| **L = Lotion. C = Creme** | C | L | C | L | C | L | L | L | C | C | C |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Lanolin; anhydrous USP | | | 5 | | | | | | | 4 | |
| Undecan oder Tridecan | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | Ad 100, q.s. | | | | | | | | | | |

**Tabelle 36**

| **Komponente** | **1** | **2** | **3** | **4** | **5** | **6** | **7** |
|---|---|---|---|---|---|---|---|
| Ethanol | | 4,00 | | 3,00 | | 4,50 | |
| Wasser dem. (add to) | 100 | 100 | 100 | 100 | 100 | 100 | 100 |
| Phenonip | 1,00 | 1,00 | 1,00 | 1,00 | 1,00 | 1,00 | 1,00 |
| Glycerin 99% | 3,00 | 3,00 | 3,00 | 3,00 | 3,00 | 3,00 | 3,00 |
| Ultragel^{®} 300 (Polyquaternium-37) | 0,8 | 1,00 | 1,50 | 0,90 | 2,00 | 1,50 | 0,75 |
| Eumulgin^{®} VL 75 (Lauryl Glucoside (and) Polyglyceryl-2 Dipolyhydroxystearate (and) Glycerin) | 1,80 | - | 0,80 | - | - | - | - |
| Emulgade^{®} PL 68/50 (Cetearyl Glucoside (and Cetearyl Alcohol) | - | - | - | - | - | 3,00 | - |
| Eumulgin^{®} SG (Sodium Stearylglutamate) | 0,10 | 0,12 | 0,30 | 0,10 | - | | 0,08 |
| Cutina^{®} PES (Pentaerythrityl Distearate) | 2,50 | 3,5 0 | 1,70 | 4,00 | 2,00 | 4,00 | 5,00 |
| Cetiol^{®} J600 (Oleyl Erucate) | 2,00 | - | 5,00 | - | - | 1,00 | 3,00 |
| Cetiol^{®} CC (Dicaprylyl Carbonate) | 3,50 | - | 4,00 | 4,00 | 2,50 | 3,00 | 4,00 |
| Cetiol^{®} PGL (Hexyldecanol (and) Hexyldecyl Laurate) | - | 2,00 | - | 4,00 | 5,00 | 5,00 | 1,00 |
| Tegosoft^{®} DEC (Diethylhexyl Carbonate) | - | - | - | 2,50 | - | - | 3,00 |
| DC^{®} 345 (Cyclopentasiloxane) | 4,00 | - | - | - | 2,00 | - | - |
| Cetiol^{®} B (Dibutyl Adipate) | 5,00 | 3,5 | 5,00 | | | 2,00 | |
| Myritol^{®} 331 (Cocoglycerides) | 3,00 | - | - | - | - | 4,00 | 4,50 |
| Undecan oder Tridecan | 3,00- | 3,00 | 5,00 | 2,00 | 7,00 | 5,00 | 3,00- |
| DHA (Dihydroxyaceton) | 2,00 | 1,00 | 2,50 | 1,00 | 2,00 | 2,00 | 3,50 |
| Uvinul^{®} T 150 (Ethylhexyl Triazone) | - | | 1,00 | - | - | - | - |
| Tinosorb^{®} M (Methylene Bis- | - | 1,00 | 1,00 | - | - | - | - |
| Benzotriazoyl Tetramethylbutylphenol) | | | | | | | |
| Tinosorb^{®} S (Bis-Ethylhexyloxyphenol Methoxyphenyl Triazine) | - | 2,00 | - | - | 0,50 | - | - |
| Uvasorb^{®} HEB (Diethylhexyl Butamino Triazone) | - | - | - | - | 1,00 | - | - |
| Neo Heliopan^{®} AV (Ethylhexyl Methoxycinnamate | - | 5.00 | 5,00 | - | 3,00 | - | - |
| Parsol^{®} 1789 (Butyl Methoxydibenzoylmethane) | - | 3.00 | 3,00 | - | 2,00 | - | - |
| Neo Heliopan^{®} 303 (Octocrylene) | - | 3,50 | 2,00 | - | - | - | - |

**Sprühbare W/O Emulsion (SPF 20)**

| Phase | Bestandteil | INCI | Gew.-% |
|---|---|---|---|
| I | Dehymuls® LE | PEG-30 Dipolyhydroxystearate | 3.00 |
| | DC 245 | Cyclopentasiloxane | 5.00 |
| | Cetiol® A | Hexyl Laurate | 5.00 |
| | Cetiol® CC | Dicaprylyl Carbonate | 1,50 |
| | | Undecan oder Tridecan | 1,50 |
| | Cosmedia ® Gel CC | Dicaprylyl Carbonate (and) Stearalkonium Hectorite (and) Propylene Carbonate | 4.00 |
| | Cetiol® Sun | Titanium Dioxide Dispersion | 20.00 |
| II | Deionized Water | Aqua | qs 100 |
| | Butylene Glycol | Butylene Glycol | 3.00 |
| | Ajidew N50 | Sodium PCA | 2.00 |
| | Magnesium Sulphate | Magnesium Sulphate | 0.50 |
| | EDTA 4 Na | Tetrasodium EDTA | 0.05 |
| | Preservative | | q.s. |
| III | Parfum | | q.s. |
| Viscosity, Brook. RVT, 20°C, Sp 4, 5 rpm 2700 mPas | | | |

**Sprühbare W/O Emulsion (SPF 20)**

| Phase | Bestandteil | INCI | Gew.-% |
|---|---|---|---|
| I | Dehymuls® LE | PEG-30 Dipolyhydroxystearate | 4.00 |
| | Dehymuls ® PGPH | Polyglyceryl-2 Dipolyhydroxystearate | 4.00 |
| | Cetiol® 868 | Ethylhexyl Stearate | 7.00 |
| | Cetiol® CC | Dicaprylyl Carbonate | 2,70 |
| | | Undecan oder Tridecan | **2,70** |
| | Cosmedia ® Gel CC | Dicaprylyl Carbonate (and) Stearalkonium Hectorite (and) Propylene Carbonate | 3.00 |
| | Cetiol ® OE | Dicaprylyl Ether | 5.00 |
| | Zincum N 29 | Zinc Oxide | 2.00 |
| II | Cetiol® Sun | Titanium Dioxide Dispersion | 20.00 |
| III | Deionized Water | Aqua | qs 100.00 |
| | Glycerine | Glycerine | 5.00 |
| | Sodium Chloride | Sodium Chloride | 0.50 |
| | Magnesium Sulphate | Magnesium Sulphate | 0.50 |
| | Preservative | | q.s. |
| IV | Parfum | | q.s. |
| Viscosität: Brookfield. RVT, 20°C, Sp 5, 10 rpm 10400mPas | | | |

**Sonnenschutz Spray (SPF 6)**

| Phase | Bestandteil | INCI | Gew.-% |
|---|---|---|---|
| I | Deionized Water | Aqua | qs 100 |
| | Butylene Glycol | Butylene Glycol | 3.00 |
| II | Veegum Ultra | Magnesium Aluminum Silicate | 0.50 |
| | Keltrol T | Xanthan Gum | 0.30 |
| III | Eumulgin ® VL 75 | Lauryl Glucoside (and) Polyglyceryl-2- Dipolyhydroxystearate (and) Glycerin | 6.00 |
| | Cetiol® CC | Dicaprylyl Carbonate | 3,50 |
| | | Undecan oder Tridecan | **3,50** |
| | Cetiol® AB | C12-15 Alkyl Benzoate | 7.00 |
| | Cetiol® Sun | Titanium Dioxide Dispersion | 10.00 |
| | Preservative | | q.s. |
| | Parfum | | q.s. |
| Viscosität: Brook. RVT, 20°C, Sp 4, 5 rpm 3500 mPas pH= 6.5 | | | |

**Sonnenlotion (SPF 20)**

| | Bestandteil | INCI | Gew.-% |
|---|---|---|---|
| I | Myritol® 331 | Cocoglycerides | 2.00 |
| | Cetiol® SB 45 | Shea Butter | 1.00 |
| | Cosmedia Gel CC | Dicaprylyl Carbonate (and) Stearalkonium Hectorite | 2.00 |
| | Uvasorb® HEB | Dioctyl Butamido Triazone | 3.00 |
| | | Undecan oder Tridecan | 2,00 |
| | Neo Heliopan® AV | Ethylhexyl Methoxycinnamate | 4.00 |
| | Neo Heliopan® E1000 | Isoamyl p-Methoxycinnamate | 4.00 |
| | Neo Heliopan® 303 | Octocrylene | 3.00 |
| | Copherol® 1250 C | Tocopheryl Acetate | 0.25 |
| | Cosmedia® DC | Hydrogenated Dimer Dilinoleyl/Dimethylcarbonate Copolymer | 2.00 |
| II | Cetiol® Sun | Titanium Dioxide Dispersion | 6.00 |
| | Satiaxane CX 91 | Xanthan gum | 0,30 |
| | Eumulgin® VL75 | Lauryl Glucoside (and) Polyglyceryl-2-Dipolyhydroxystearate (and) Glycerin | 3.13 |
| III | Deionized Water | Water | 58.22 |
| | 2 NaEDTA | Disodium EDTA | 0.10 |
| | Liponic EG-1 | Glycereth-26 | 3.00 |
| IV | Tinosorb M | Methylene bis-benzotriazolyltetramethylbutylphenol | 3.00 |
| | Water | Water | 3.00 |
| | Preservative | | q.s. |
| | Parfum | | q.s |
| Viscosität Brook. RVT, 20°C, spindle 5,10 rpm 2560 mPaspH= 6.70 | | | |

**Sonnenlotion (SPF 50+)**

| | Bestandteil | INCI | Gew.-% |
|---|---|---|---|
| I | Cetiol® B | Bibutyl Adipate | 2.00 |
| | Cosmedia® DC | Hydrogenated Dimer Dilinoleyl/Dimethylcarbonate Copolymer | 1.00 |
| | | Undecan oder Tridecan | 2.00 |
| II | Neo Heliopan® OS | Ethylhexyl Salicylate | 3.00 |
| | Tinosorb® S | Bis-ethylhexyloxyphenol Methoxyphenyl Triazine | 2.50 |
| | Neo Heliopan® AV | Ethylhexyl Methoxycinnamate | 6.00 |
| | Neo Heliopan® 303 | Octocrylene | 8.00 |
| | Neo Heliopan® 357 | Butyl Methoxydibenzoylmethane | 2.50 |
| III | Cosmedia® Gel CC | Dicaprylyl Carbonate (and) Steralkonium Hectorite (and) Propylene Carbonate | 2.00 |
| IV | Emulgade® PL 6850 | Cetearyl Glucoside (and) Cetearyl Alcohol | 2.50 |
| | Eumulgin ® VL 75 | Lauryl Glucoside (and) Polyglyceryl-2- Dipolyhydroxystearate (and) Glycerin | 3.00 |
| V | Cetiol® Sun | Titanium Dioxide Dispersion | 10.00 |
| VI | Deionized Water | Aqua | qs 100 |
| | 2 NaEDTA | Disodium EDTA | 0.10 |
| | Preservative | | q.s. |
| | Liponic EG-1 | Glycereth-26 | 3.00 |
| | Keltrol® T | Xanthan gum | 0.60 |
| | Veegum Ultra | Magnesium Aluminum Silicate | 2.00 |
| VII | Dry Flo Plus | Aluminum Starch Octenylsuccinate | 2.00 |
| VIII | Tinosorb® M | Methylene Bis-Benzotriazolyl Tetramethylbutylphenol | 5.00 |
| IX | Parfum | | q.s. |
| Viskosität Brookfield Helipath 20°C, TB, 10 rpm 16200 mPas pH= 6.7 | | | |

**Anti-Ageing Sonnenschutz (SPF 30)**

| | Bestandteil | INCI | Gew.-% |
|---|---|---|---|
| I | Myritol® 331 | Cocoglycerides | 2.00 |
| | Cetiol® B | Bibutyl Adipate | 3.00 |
| | | Undecan oder Tridecan | 2.00 |
| | Uvinul A plus | Diethylamino Hydroxybenzoyl Hexyl Benzoate | 5.00 |
| | Uvinul T 150 | Ethylhexyl Triazone | 2.50 |
| | Cosmedia® Gel CC | Dicaprylyl Carbonate (and) Stearalkonium Hectorite | 2.00 |
| | Cosmedia® DC | Hydrogenated Dimer Dilinoleyl/Dimethylcarbonate Copolymer | 2.00 |
| II | Cetiol® Sun | Titanium Dioxide Dispersion | 10.00 |
| | Satiaxane CX 91 | Xanthan gum | 0.40 |
| | Eumulgin® VL75 | Lauryl Glucoside (and) Polyglyceryl-2- Dipolyhydroxystearate (and) Glycerin | 3.50 |
| III | Deionized Water | Aqua | 49.60 |
| | 2NaEDTA | Disodium EDTA | 0.10 |
| | Glycerin | Glycerin | 3.00 |
| | Preservative | | q.s. |
| IV | Photonyl® LS | Arginine (and) Disodium Adenosine Triphosphate (and) Mannitol (and) Pyridoxine HCl (and) RNA (and) Histidine HCl (and) Phenylalanine (and) Tyrosine | 2.00 |
| | Water | | 12.00 |
| V | Copherol® 1250 C | Tocopheryl Acetate | 0.50 |
| | Parfum | | q.s. |
| Viskosität: Brook. RVT, 20°C, Spindel 5, 10 rpm 3560 mPaspH= 6.50 | | | |

**Anti-Falten Creme**

| | Bestandteil | INCI | Gew.-% |
|---|---|---|---|
| I | Eumulgin ® BA 25 | Beheneth-25 | 3.30 |
| | Lameform® TGI | Polyglyceryl-3 Diisostearate | 0.70 |
| | | Undecan oder Tridecan | 2.00 |
| | Cutina ® PES | Pentaerythrityl Distearate | 2.00 |
| | Cetiol ®CC | Dicaprylyl Carbonate | 1.30 |
| | Cetiol ® AB | C12-C15 Alkyl Benzoate | 5.00 |
| | Cetiol ® LC | Coco-Caprylate/Caprate | 3.00 |
| | Cetiol® Sun | Titanium Dioxide Dispersion | 6.00 |
| | Phenonip | Phenoxyethanol (and) parabens | 0.80 |
| II | Deionized Water | Aqua | 61.85 |
| | 4NaEDTA | Tetrasodium EDTA | 0.05 |
| | Glycerin | Glycerin | 3.00 |
| | Butylene Glycol | Butylene Glycol | 2.00 |
| III | Veegum Ultra | Magnessium Aluminum Silicate | 3.00 |
| | Keltrol T | Xanthan Gum | 0.30 |
| IV | | Perfume Waterfresh | 0.50 |
| | Coviox ® T70 C | Tocopherol | 0.10 |
| V | Deionized Water | Aqua | 1.00 |
| | Plantactiv ® Aesculus | Escin | 0.10 |
| Viskosität, Brookfield, RVT, 20°C, Spindel 5, 5 rpm 11500 mPas pH= 6.2-6.7 | | | |

**Sonnenschutzlotion SPF 30**

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | | Undecan oder Tridecan | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia ® DC | Hydrogenated Dimer Dilinoleyl/Dimethylcarbonate Copolymer | 1.00 |
| II | Cetiol® Sun | Titanium Dioxide Dispersion | 14.00 |
| | Satiaxane CX 91 | Xanthan gum | 0.10 |
| | Cosmedia ® SP | Sodium Polyacrylate | 0.40 |
| | Eumulgin® VL75 | Lauryl Glucoside (and) Polyglyceryl-2-Dipolyhydroxystearate (and) Glycerin | 3.75 |
| III | Deionized Water | Aqua | qs 100.00 |
| | Neo Heliopan® Hydro | Phenylbenzimidazole Sulfonic acid | 2.00 |
| | Trometamine | Trometamine | 1.60 |
| | Phenonip | | qs |
| Viscosität: Brookfield, RVT, 20°C, Spindel 5,10 rpm 2240 mPas pH= 7.35 | | | |

**Sonnencreme SPF 50+**

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | | Undecan oder Tridecan | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia® DC | Hydrogenated Dimer Dilinoleyl/Dimethylcarbonate Copolymer | 2.00 |
| | Emulgade® PL 6850 | Cetearyl Glucoside (and) Cetearyl Alcohol | 4.50 |
| | Lanette® E | Sodium Cetearyl Sulfate | 1.0 |
| II | Cetiol® Sun | Titanium Dioxide Dispersion | 14.00 |
| III | Deionized Water | Aqua | qs 100.00 |
| | Neo Heliopan® Hydro | Phenylbenzimidazole Sulfonic Acid | 2.00 |
| | NaOH 50% | Sodium Hydroxide | 0.6 |
| | | Preservative | q.s. |
| | | Parfum | q.s. |
| Viskosität: Brookfield, Helipath 20°C, TE, 4 rpm , 195000 mPas pH= 7.5 | | | |

**AP/Deo in Aerosol Form (Gew.-%)**

| Bestandteil | INCI | A | B |
|---|---|---|---|
| IPM | Isopropyl Myristate | 10,8 | 10,8 |
| Cetiol® CC | Dicaprylyl Carbonate | 5.00 | 5.00 |
| Cosmedia® Gel CC | Dicaprylyl Carbonate (and) Stearalkonium Hectorite (and) Propylene Carbonate | - | 2.00 |
| | Caprylic capric triglyceride(and)Stearalkonium Hectorite (and) Propylene Carbonate | 2.00 | - |
| | Undecan oder Tridecan | 2.00 | 2.00 |
| Locron® P | Aluminium chlorhydrate | 3.00 | 3.00 |
| Hydagen® CAT | Triethyl Citrate | 1.00 | 1.00 |
| Perfume | Perfume | 1.20 | 1.20 |
| Propane | | 75.00 | 75.00 |

**Foundation oder Sonnenschutz enthaltend Titanium Dioxid**

| Ingredients | INCI Name | Gew.-% |
|---|---|---|
| Cetiol® CC | Dicaprylyl Carbonate | 95.00 |
| Cosmedia® Gel CC | Dicaprylyl Carbonate (and) Stearalkonium Hectorite (and) Propylene Carbonate | 1.00 |
| | Undecan oder Tridecan | 1.00 |
| Titanium dioxide | | 5.00 |

**Sonnenschutz Spray SPF 40 (Gew.-%)**

| | Ingredient | INCI | A | B |
|---|---|---|---|---|
| I | Eumulgin ® VL 75 | Lauryl Glucoside (and) Polyglyceryl-2 Dipolyhydroxystearate (and) Glycerin | 6.0 | 6.0 |
| | Generol ® R | Brassica Campestris (Rapeseed) | 1.0 | 1.0 |
| | Cegesoft ® SH Cosmedia ® Gel CC | Sterols Shorea Stenoptera Dicaprylyl Carbonate(and) Stearalkonium Hectorite (and) Propylene Carbonate | 1.0 2.0 | 1.0 |
| | Eusolex 2292 | Ethylhexylmethoxycinnamate | 7.5 | 7.5 |
| | Tinosorb® S | Bis-ethylhexyloxyphenol methoxyphenyltriazine | 2.0 | 2.0 |
| | Cetiol ® CC | Dicaprylyl carbonate | 1.5 | |
| | | Undecan oder Tridecan | 1.5 | 1.5 |
| | Cetiol ® AB | C12-15 Alkyl Benzoate | 4.0 | 4.0 |
| | Cosmedia ® DC | Hydrogenated Dimer Dilinoleyl / Dimethylcarbonate Copolymer | 1.00 | 1.00 |
| II | Water | | 61.45 | 63.45 |
| | EDTA 4Na (Prolabo) | Tetrasodium EDTA | 0,05 | 0.05 |
| | Elestab 50J | Chlorphenesin & Methylparaben | 0.60 | |
| III | Cosmedia®SP | Sodium Polyacrylate | 0.40 | 0.40 |
| I V | Tinsorb® M | Methylenebis benzotriazolyl tetramethylbutylphenol | 10.0 | 10.0 |
| Viskosität, Brookfield. RVT, 23° C, spindle 2,5 rpm, ph:6 3500mPas 700mPas | | | | |

**Spray SPF 40**

| | Bestandteil | INCI | A | B |
|---|---|---|---|---|
| I | Eumulgin® VL75 | Lauryl Glucoside (and) Polyglyceryl-2 Dipolyhydroxystearate (and) Glycerin | 6.0 | 6.0 |
| | Generol® | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 3.0 | - |
| | | Undecan oder Tridecan | **2.0** | **2.0** |
| | Eusolex 2292 | Ethylhexylmethoxycinnamate | 7.5 | 7.5 |
| | Tinosorb S | Bis-ethylhexyloxyphenol methoxyphenyltriazine | 2.13 | 4.74 |
| | Cetiol® CC | Dicaprylyl Carbonate | 2.0 | 2.0 |
| | Cetiol® AB | C12-15 Alkyl Benzoate | 4.0 | 4.0 |
| II | Water | | 62.32 | 62.71 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.00 | 10.00 |
| Viskosität Book. RVT, 23° C, spindle 2,5 rpm, ph:6 A: 4020 mPas B: 730 mPas | | | | |

**Feuchtigkeitsspendende Sonnenschutz Lotion W/O SPF 20**

| | Bestandteil | INCI | A | B |
|---|---|---|---|---|
| I | Dehymuls® | PEG - 30 Dipolyhydroxystearate | 3.00 | 3.00 |
| | DC 245 | Cyclopentasiloxane | 5.00 | 5.00 |
| | Cetiol® OE | Dicaprylyl Ether | 4.00 | 4.00 |
| | Cetiol® A | Hexyl Laurate | 5.00 | 5.00 |
| | Cetiol® CC | Dicaprylyl Carbonate | 1.50 | 1.50 |
| | | Undecan oder Tridecan | **1.50** | **1.50** |
| II | Cosmedia® Gel CC | Dicaprylyl Carbonate(and) Stearalkonium Hectorite (and) Propylene Carbonate | 4.00 | |
| III | Cetiol® SUN | Titanium dioxide (and) Alumina (and) Dimethicone and) Stearic acid ( and) Dicaprylyl Carbonate (and) Polyhydroxystearic acid | 20.00 | 20.00 |
| IV | Deionized water | Aqua | 49.55 | 53.55 |
| | Butylene Glycol | Butylene Glycol | 3.00 | 3.00 |
| | Ajidew N 50 | Sodium PCA | 2.00 | 2.00 |
| | Mg SO4 | Magnesium Sulfate | 0.50 | 0.50 |
| | EDTA, 4 Na | Tetrasodium EDTA | 0.05 | 0.05 |
| | Phenonip | Phenoxyethanol (and) Parabens | 0.50 | 0.50 |
| V | PerfumeSoleil/L27 8319/ C | Floressence | 0.40 | 0.40 |
| Viskosität, Brook. RVT, spindle 4, speed 5 [mPas] | | | 2000 | 5000 |

**Spray SPF 40 (Angaben in Gew.-%)**

| | Bestandteil | INCI | A | B |
|---|---|---|---|---|
| I | Eumulgin® VL 75 | Lauryl Glucoside (and) Polyglyceryl-2 Dipolyhydroxystearate (and) Glycerin | 6.0 | 6.0 |
| | Generol® R | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 2.0 | |
| | Eusolex 2292 | Ethylhexylmethoxycinnamate | 7.5 | 7.5 |
| | Tinosorb S | Bis-ethylhexyloxyphenol methoxyphenyltriazine | 2.0 | 2.0 |
| | Cetiol® CC | Dicaprylyl carbonate | 1.5 | 1.5 |
| | | Undecan oder Tridecan | 1.5 | 1.5 |
| | Cetiol® AB | C12-15 Alkyl Benzoate | 4.0 | 4.0 |
| | Cosmedia ® DC | Hydrogenated Dimer Dilinoleyl/Dimethylcarbonate Copolymer | 1.0 | 1.00 |
| II | Water | | 61.45 | 63.45 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.0 | 10.0 |
| Viskosität, Brook. RVT, 23° C, spindle 2, 5 rpm, pH: 6 [mPas] A: 3500, B: 700 | | | | |

**Mat finish fluid foundation**

| | Bestandteil | INCI | Gew.-% |
|---|---|---|---|
| I | Lameform® TGI | Polyglyceryl-3 Diisostearate | 1.50 |
| | Eumulgin® BA25 | Beheneth-25 | 3.50 |
| | Cetiol® CC | Dicaprylyl Carbonate | 4.00 |
| | Myritol® 331 | Cocoglycerides | 2.00 |
| | | Undecan oder Tridecan | 2.00 |
| | Fitoderm® | Squalane | 3.00 |
| | Cutina® PES | Pentaerythiryl Distearate | 1.00 |
| | Cegesoft® SH | Shorea Stenoptera | 1.00 |
| | Phenonip | Phenoxyethanol (and) Parabens | 0.80 |
| | KF 96, 100cs | Dimethicone | 3.00 |
| | Cetiol® OE | Dicaprylyl Ether | 3.00 |
| II | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33- 8001 | Iron Oxide CI 77491 | 0.60 |
| | SunPuro Black Iron Oxide C33-7001 | Iron Oxide CI 77499 | 0.20 |
| III | Deionized Water | Aqua | 52.50 |
| | Glycerine | Glycerin | 3.00 |
| | Butylene Glycol | Butylene Glycol | 2.00 |
| IV | Veegum Ultra | Magnesium Aluminium Silicate | 1.00 |
| | Keltrol T | Xanthan Gum | 0.40 |
| V | Cosmedia® Gel CC | Dicaprylyl Carbonate (and) Stearalkonium Hectorite (and) Propylene Carbonate | 3.00 |
| VI | Cosmedia® PMMA V12 | Polymethylmethacrylate | 2.00 |
| VII | Mirasil CDPDM | Cyclomethicone (and) Diphenyldimethicone | 3.00 |
| VIII | Perfume Caresse | | 0.20 |
| | Viskosiät, Brook. RVT, spindle 3, speed 5, 8400mPas pH:6.7 | | |

**Ultra protective spray SPF 40 0/W**

| | Bestandteil | INCI | Gew.-% |
|---|---|---|---|
| I | Eumulgin® VL 75 | Lauryl Glucoside (and) Polyglyceryl-2 Dipolyhydroxystearate (and) Glycerin | 7.00 |
| | Generol® R | Brassica Campestris (Rapeseed) Sterols | 1.00 |
| | Cosmedia® DC | Hydrogenated Dimer Dilinoleyl / Dimethylcarbonate Copolymer | 1.00 |
| | Eusolex 2292 | Ethylhexylmethoxycinnamate | 7.50 |
| | Tinosorb S | Bis-ethylhexyloxyphenol methoxyphenyltriazine | 2.00 |
| | Cetiol® CC | Dicaprylyl carbonate | 2.00 |
| | Cetiol® AB | C12-15 Alkyl Benzoate 4.00 | 4.00 |
| | | Undecan oder Tridecan | 2.00 |
| | Cegesoft® VP | Olus & Hydrogenated Vegetable Oil & Candelilla Cera | 2.50 |
| | Myritol® 331 | Cocoglycerides | 2.00 |
| | Cosmedia® Gel CC | Sodium polyacrylate Carbonate(and) Stearalkonium Hectorite (and) Propylene Carbonate | 4.00 |
| II | Water | qsp100 | 55.15 |
| | EDTA 4Na | Tetrasodium EDTA 0.05 | 0.05 |
| | Elestab 50J | Chlorphenesin & Methylparaben | 0.60 |
| III | Keltrol T | Xanthan Gum | 0.20 |
| IV | Tinosorb M | Methylenebis benzotriazolyl tetramethylbutylphenol | 10.00 |
| Viscosity, Brook. RVT, 23° C, spindle 2,5 rpm, 2500mPaspH:6.4 | | | |

**Soft moisture Gel Creme**

| | Bestandteil | INCI | Gew.-% |
|---|---|---|---|
| I | Deionized Water | Aqua | 65.35 |
| | Glycerine | Glycerin | 3.00 |
| | Phenonip | Phenoxyethanol (and) Parabens | 0.60 |
| II | Cosmedia® SP | Sodium Polyacrylate | 1.20 |
| III | Cegesoft® PFO | Passiflora Incarnata | 2.00 |
| | Eusolex 2292 | Ethylhexyl Methoxycinnamate | 2.00 |
| | KF 96,100cs | Dimethicone 2.00 | 2.00 |
| IV | Cetiol® CC | Dicaprylyl Carbonate | 2.50 |
| | Eutanol® G | Octyldodecanol | 4.0 |
| | | Undecan oder Tridecan | 2.50 |
| | Eusolex 4360 | Benzophenone-3 | 0.10 |
| | Cegesoft® SBE | Butyrospermum Parkii | 1.50 |
| V | Cosmedia® Gel CC | Dicaprylyl Carbonate (and) Stearalkonium Hectorite (and) Propylene Carbonate | 5.00 |
| VI | Deionized Water | Aqua | 5.00 |
| | Osmhydran® LS 8453 | Mannitol (and) Arginine (and) Serine (and) Sucrose (and) PCA (and) Citrulline (and) Glycogen (and) Histidine HCI (and) Alanine (and) Threonine (and) Glutamic Acid (and) Lysine HCI | 2.00 |
| VII | Vegeseryl® LP LS 9058 | Hydrolyzed Soy Protein | 1.00 |
| VIII | Coviox® T70C | Tocopherol | 0.10 |
| | Perfume Raphaelle | | 0.15 |
| Viscosity, Brook. RVT, Helipath TE, speed 4,101 000mPas pH:5.5 | | | |

**Samtige Reinigungsmaske**

| | Bestandteil | INCI | Gew.-% |
|---|---|---|---|
| I | Emulgade® SE-PF | Glyceryl Stearate (and) Ceteareth-20 (and) Ceteareth-12 (and) Cetearyl Alcohol (and) Cetyl Palmitate | 8.00 |
| | Lanette® O | Cetearyl Alcohol | 2.00 |
| | Cegesoft® PS6 | Olus | 5.00 |
| | Cetiol® CC | Dicaprylyl Carbonate | 2.00 |
| | Cutina® PES | Pentaerythrityl Distearate | 3.00 |
| | | Undecan oder Tridecan | 2.00 |
| II | Cosmedia® Gel CC | Dicaprylyl Carbonate (and) Stearalkonium Hectorite (and) Propylene Carbonate | 5.00 |
| III | Deionized Water | Aqua | 45.00 |
| | Glycerine | Glycerin | 10.00 |
| | EDTA, 4Na | Tetrasodium EDTA | 0.10 |
| | Elestab® 50J | Chlorphenesin (and) Methylparaben | 0.40 |
| IV | Dry Flo AF | Corn Starch Modified0 | 1.00 |
| | Propylene Glycol | Propylene Glycol | 3.00 |
| V | Kaolin | Kaolin | 10.00 |
| VI | Perfume | | 0.20 |
| | Concombre | | |
| | Coviox® T70C | Tocopherol | 0.10 |
| VII | Purisoft ® LS 9602 | Glycerin (and) Moringa Pterygosperma | 2.00 |
| VIII | Green Covasol W7035 (a.s.0.1%) | CI 42090 (and) CI19140 | 3.20 |
| Viscosity, Brook. RVT, Helipath TE, Speed 4, 254 000mPas pH:6.3 | | | |

**Luxuriöse Körper Butter**

| | Bestandteil | INCI | Gew.-% |
|---|---|---|---|
| I | Deionized water | Aqua | 61.50 |
| | Glycerine | Glycerin | 5.00 |
| | Butylene Glycol | Butylene Glycol | 2.00 |
| | EDTA, 4 Na | Tetrasodium EDTA | 0.10 |
| | Phenonip | Phenoxyethanol (and) Parabens | 0.80 |
| II | Cosmedia® SP | Sodium Polyacrylate | 1.50 |
| III | Eumulgin® BA 25 | Beheneth-25 3.00 | 3.00 |
| | Lanette® O | Cetearyl Alcohol | 4.00 |
| | Cetiol® SN | Cetearyl Isononanoate | 3.00 |
| | Cetiol® SB45 | Butyrospermum Parkii | 5.00 |
| | Cetiol® CC | Dicaprylyl Carbonate | 0.50 |
| | | Undecan oder Tridecan | 0.50 |
| | KF 96, 100cs | Dimethicone | 1.00 |
| | Generol® R | Brassica Campestris (Rapeseed) Sterols | 0.50 |
| IV | Cosmedia® Gel CC | Dicaprylyl Carbonate (and) Stearalkonium Hectorite (and) Propylene Carbonate | 5.00 |
| V | Cosmedia® PMMA V12 | Polymethylmethacrylate | 2.00 |
| | Deionized water | Aqua | 4.00 |
| VI | Coviox® T70C | Tocopherol | 0.10 |
| | Perfume Pétale 139012E | | 0.50 |
| Viscosity, Brook. RVT Helipath TE, speed4, 800 000mPas pH:7 | | | |

**Happy Deo Mist**

| Bestandteil | INCI | Gew.-% |
|---|---|---|
| IPM | Isopropyl Palmitate | 6.50 |
| Cetiol® Sensoft | Propylheptyl Caprylate | 6.50 |
| Cosmedia® Gel CC | Caprylic/Capric Triglyceride (and) Stearalkonium Hectorite (and) Propylene Carbonate | 2.00 |
| | Undecan oder Tridecan | 2.00 |
| Locron P | Aluminium Clorohydrate | 5.00 |
| Perfume Deo Talco | Perfume | 0.30 |
| Propellant | | 77.7 |

| | | |
|---|---|---|
| Ratio propellant/other (3.34:1); Valve: RK 150P RA 634/5/8 (Vapor Phase); Button: V04.776 | | |

**Rich Hair conditioner / Haar Conditioner**

| Phase | Handelsname | INCI Bezeichnung | Gew.-% |
|---|---|---|---|
| I. | Dehyquart®L80 | Dicocoylethyl Hydroxyethylmonium Methosulfat (und) Propylene Glycol | 2 |
| | Lanette®O | Cetearyl Alcohol | 5 |
| | Cutina®GMS | Glyceryl Stearate | 1 |
| | Undecan oder Tridecan | | **0,3** |
| | Cetiol®J 600 | Oleyl Erucate | 0,5 |
| | Plantacare®1200 UP | Lauryl Glucoside | 2 |
| II. | Glycerol | | 2 |
| | Wasser, entionisiert | | 50 |
| III. | Hydroxyethylcellulose | Hydroxyethylcellulose | 0,4 |
| | Wasser, deionisiert | | Ad 100 |
| IV. | Aloe vera Gel | | 0,1 |
| | Konservierungsmittel | | q.s. |
| | Parfum | | q.s. |
| >> | pH Wert | | 4 - 4,5 |
| >> | Viskosität mPas | | Ca. 8000 |

Herstellung: Phase I und II wurden auf 85°C erhitzt, Phase II wurde bei 85°C zugefügt und die Temperatur wurde beim Rühren gehalten bis zur vollständigen Homogenität. Die Emulsion wurde unter Rühren abgekühlt und das vorher in Wasser aufgelöste Polymer (Phase III) wurde zugefügt. Danach wurden die übrigen Additive (Phase IV) zugefügt und der pH-Wert eingestellt (z.B. mit Zitronensäure).

**Hair-Rinse & Colouring /Haar Spülung und Färbung**

| Phase | Handelsname | INCI Bezeichnung | Gew.-% |
|---|---|---|---|
| I. | Dehyquart® F75 | Distearoylethyl Hydroxyethylmonium Methosulfate (and) Cetearyl Alcohol | 2 |
| | Eumulgin® B2 | Ceteareth-20 | 0,5 |
| | Lanette® O | Cetearyl Alcohol | 4 |
| | Undecan oder Tridecan | | **1,5** |
| II. | Wasser, deionisiert | | Ad 100 |
| III. | Sol. 5% Arianor Mahogany | Colour | 10 |
| IV. | Konservierungsmittel | | q.s. |
| >> | pH Wert | | 3,76 |

Herstellung: Schmelzen von Phase I bei 80-85°C. Erhitzen von Phase II auf 80-85°C and einrühren in Phase I. Rühren bei dieser Temperatur bis zur kompletten Homogenität. Kühlen unter Rühren auf 40°C. Hinzufügen von Konservierungsmittel and wenn nötig, von hitzeempfindlichen Additiven. Kühlen beim Rühren auf 30°C.Anpassen des pH Wertes, z.B. mit Zitronensäure. Hinzufügen der Arianor Lösung bei unter 40°C.

**Styling Gel Typ "Out of bed"**

| Phase | Handelsname | INCI Bezeichnung | Gew.-% |
|---|---|---|---|
| I. | Alcool 96% (Prolabo) | Alcohol 96% | 12 |
| | Luviskol® VA 64 (BASF) | PVP/VA | 4,5 |
| | Polyox® WSR-301 | PEG-90M | 0,25 |
| | Methocel®E4M Premium EP (Dow) | Hydroxypropyl Methylcellulose | 0,6 |
| | Dehyquart® L80 | Dicocoylethyl Hydroxyethylmonium Methosulfate (and) Propylene Glycol | 0,6 |
| II. | Eumulgin® HRE 40 | PEG-40 Hydrogenated Castor Oil | 1 |
| | Undecan oder Tridecan | | **1,5** |
| III. | Wasser, entionisiert | | a.d. |
| IV | Hispagel® 200 | Glycerin (und) Glyceryl Polyacrylate | 36,7 |
| V | Konservierungsmittel, Parfum | | q.s. |
| >> | pH Wert | | 6,8 |
| >> | Viskosität (mPas) Brookfield RVT 23°C Spindel 5, 10rpm | | 220000 |

Herstellung: 1. Hinzufügen von Phase I bei Raumtemperatur nach und nach zum Alkohol. 2. Mischen von Phase II bis zur Homogenität und hinzufügen zu Phase I, 3. Hinzufügen von Phase III langsam zu Phase I und II, 4. Hinzufügen von Phase IV langsam zu Phase I-III, 5.wenn nötig hinzufügen von Phase V.

**Sprühbarer Hair-Care Conditioner mit Gluadin® WQTM**

| Phase | Handelsname | INCI | Gew.-% |
|---|---|---|---|
| I | Monomuls® 60-35C Powder | Hydrogenated Palm Glycerides | 1,24 |
| | Eumulgin® B1 | Ceteareth-12 | 2,76 |
| | Cetiol® OE | Dicaprylyl Ether | 5 |
| | Undecan oder Tridecan | | 4 |
| | Dow Corning® 345 | Cyclomethicone | 2 |
| II. | Glycerin (86%) | | 2 |
| | Wasser | | 16 |
| III. | Gluadin® WQTM | Hydroxypropyltrimonium hydrolyzed wheat protein | 2,85 |
| | Plantacare® 2000 UP | Decyl Glucoside | 1 |
| | Wasser | | Ad 100 |
| | Konservierungsmittel | | q.s. |
| | pH Wert | | 5,5 |
| | Viskosität mPas | | <100 |

Herstellung: 1. Erhitzen der Komponenten von Phase I auf 85°C, 2. Erhitzen der Komponenten von Phase II ebenfalls auf 85°C und hinzufügen von Phase II durch Einrühren in Phase I, 3. Hinzufügen von Phase III kalt und rühren bis es abkühlt auf Raumtemperatur.

**Conditioner mit Dehyquart® C 4046**

| Phase | Handelsname | INCI | Gew.- % |
|---|---|---|---|
| I. | Dehyquart® C 4046 | Cetearyl Alcohol (und) Dipalmitoylethyl Hydroxyethylmonium Methosulfat (und) Ceteareth-20 | 4 |
| | Eutanol® G | Octyldodecanol | 1 |
| | Undecan oder Tridecan | | **0,5** |
| II. | Wasser, entionisiert | | Ad 100 |
| | Parfum | | q.s. |
| | Konservierungsmittel | | q.s. |
| >> | pH Wert | | 3,5 |
| >> | Viskosität (mPas) Brookfield RVF, 23°C, Spindel 4, 10rpm | | 10300 |

Herstellung: Schmelzen von Phase I bei 80-85°C. Erhitzen von Phase II auf 80-85°C und Einrühren in Phase I. Fünf Minuten rühren bei dieser Temperatur. Abkühlen auf 40°C während des Rührens. Hinzufügen des Konservierungsmittels und wenn nötig, hitzeempfindlicher Additive. Abkühlen auf 30°C während des Rührens. Anpassen des pH Wertes, z.B. mit Zitronensäure.

**Conditioner mit Dehyquart® C 4046**

| Phase | Handelsname | INCI | Gew.-% |
|---|---|---|---|
| I. | Dehyquart® C 4046 | Cetearyl Alcohol (und) Dipalmitoylethyl Hydroxyethylmonium Methosulfat (und) Ceteareth-20 | 4 |
| | Undecan oder Tridecan | | **1** |
| II. | Wasser, entionisiert | | Ad 100 |
| III. | Parfum | | q.s. |
| | Konservierungsmittel | | q.s. |
| >> | pH Wert | | 3,5 |
| >> | Viskosität (mPas) Brookfield RVF, 23°C, Spindel 4, 10rpm | | 2060 |

Herstellung: Schmelzen von Phase I bei 80-85°C. Erhitzen von Phase II auf 80-85°C und Einrühren in Phase I. Fünf Minuten Rühren bei dieser Temperatur. Abkühlen auf 40°C während des Rührens. Hinzufügen des Konservierungsmittels und wenn nötig, hitzeempfindlicher Additive. Abkühlen auf 30°C während des Rührens. Anpassen des pH Wertes, z.B. mit Zitronensäure.

**Hair Polish Glossing Spray - Japan Hair Oil sprühbar**

| Handelsname | INCI | Gew.-% |
|---|---|---|
| Dow Corning® 345 Fluid (Dow Corning) | Cyclomethicone | 85 |
| Cetiol® CC | Dicaprylyl Carbonate | 4 |
| Cetiol® ISL | Isostearyl Lactate | 3 |
| Undecan oder Tridecan | | 5,2 |
| Neo Heliopan AV (Haarmann & Reimer) | Ethylhexyl Methoxycinnamate | 1,5 |
| Wacker PDM 1000 | Trimethylsiloxyphenyl Dimethicone | 0,5 |
| Konservierungsmittel | | q.s. |
| Parfum | | q.s. |
| Wasser, entionisiert | | Ad 100 |

| | | |
|---|---|---|
| Herstellung: Hinzufügen aller Zutaten in der Reihenfolge wie aufgeführt. Mischen bei Raumtemperatur. | | |

**Hair gloss finishing serum**

| Handelsname | INCI | Gew.-% |
|---|---|---|
| DC®1501 (Dow Corning) | Cyclopentasiloxane (und) Dimethiconol | 70 |
| Undecan oder Tridecan | | 19,7 |
| Cetiol® CC | Dicaprylyl Carbonat | 8 |
| Myritol® 318 | Caprylic/Capric Triglycerid | 0,5 |
| Neo Heliopan E 1000 (Haarmann & Reimer) | Isoamyl p-Methoxycinnamat | 1 |
| Phenonip (SIPCA) | Phenoxyethanol (und) Parabens | 0,5 |
| Parfum | | 0,3 |
| Farbstoff | | q.s. |

Herstellung: Hinzufügen der Zutaten wie gezeigt. Mischen bei Raumtemperatur. Bemerkung: Der Farbstoff und das Parfum, die benutzt werden, müssen öllöslich sein (das Parfum außerdem verträglich mit Dimethiconol, um Trübheit zu vermeiden)

**Hair gloss Gelatine für sehr geschädigtes und stumpfes Haar**

| Handelsname | INCI | Gew.-% |
|---|---|---|
| Cetiol® CC | Dicaprylyl Carbonate | 33,6 |
| Undecan oder Tridecan | | 10 |
| Myritol® 318 | Caprylic/Capric Triglycerid | 43,6 |
| Wacker HDK H 20 (Wacker) | Silica Dimethyl Silylate | 12,5 |
| Parfum | | 0,3 |
| pH Wert | | N.A. |
| Viskosität bei 20°C, Helipath, spindel E, 5rpm (mPas) | | 500000 |

| | | |
|---|---|---|
| Herstellung: Vermischen der Zutaten in der angegebenen Reihenfolge. | | |

**Glossy light Hair Cream**

| Phase | Handelsname | INCI | Gew. % |
|---|---|---|---|
| I. | Dehyquart® F 75 | Distearoylethyl hydroxyethylmonium Methosulfat (und) Cetearyl Alkohol | 0,8 |
| | Dehyquart® L 80 | Dicocoylethyl Hydroxyethylmonium Methosulfat (und) Propylene Glycol | 1,8 |
| | Undecan oder Tridecan | | 1 |
| | Lanette® O | Cetearyl Alcohol | 5 |
| | Cetiol® CC | Dicaprylyl Carbonate | 4 |
| II. | Wasser, entionisiert | | 87,4 |
| III. | Konservierungsmitt el | | q.s. |
| | Parfum | | q.s. |
| >> | pH Wert | | 3,3 |
| | Viskosität bei 20°C, | Brookfield RVT, spindel 4, 10 rpm (mPas) | 8600 |

Herstellung: 1. Erhitzen von Phase I auf 80-85°C, 2. Erhitzen von Phase II auf 80-85°C und hinzufügen über Phase I. Ca. 30 Minuten Rühren bei derselben Temperatur. 3. Abkühlen lassen bei leichtem Rühren. 4. Wenn die Temperatur unter 40°C ist, hinzufügen von Phase III in angegebener Reihenfolge, 5. Anpassen des pH Wertes wenn nötig.

**Tiefen Haar Pflege Creme mit Wildmango Butter**

| Phase | Handelsname | INCI | Gew.-% |
|---|---|---|---|
| I. | Strukture XL (National Starch) Wasser, entionisiert | Hydroxypropyl Starch Phospat | 5 |
| | | | 86,3 |
| II. | Dehyquart L 80 | Dicocoylethyl Hydroxyethylmonium Methosulfat (und) Propylene Glycol | 2,6 |
| | Undecan oder Tridecan | | 1 |
| | Irwinol® LS 9319 | Octyldodecanol (und) Irvingia gabonensis (und) Hydrogenated Coco Glycerides | 0,1 |
| | Lamesoft®TM Benz | Glycol distearate (and) Coco Glucoside (and) Glyceryl Oleate (and) Glyceryl Stearate | 4 |
| | Gluadin ®WLM | Hydrolyzed Wheat Protein | 0,5 |
| | Nutrilan®MILK | Hydrolyzed Milk Protein | 0,5 |
| III. | Konservierungsmittel | | q.s. |
| | Parfum | | q.s. |
| >> | pH Wert | | 3,8 |
| >> | Viskosität (mPas), Brookfield RVT, 20°C, Spindel 4,10 rpm | | 10000 |

Herstellung: 1.Auflösen von Struktur XL in Wasser und Rühren bis zur vollständigen Homogenität, 2. Anpassen des pH Wertes auf 3,5 - 4,0 mit Zitronensäure, verdünnt, 3. Hinzufügen der restlichen Zutaten in der aufgeführten Reihenfolge während des Rührens (Irwinol muss vor der Hinzugabe geschmolzen werden)

**Hair Cream gloss & Conditioning**

| Phase | Handelsname | INCI | Gew.-% |
|---|---|---|---|
| I. | Dehyquart® F 75 | Distearoylethyl Hydroxyethylmonium Methosulfat (und) Cetearyl Alcohol | 2 |
| | Lanette®O | Cetearyl Alcohol | 3 |
| | Undecan oder Tridecan | | 1 |
| | Dehyquart® L 80 | Dicocoylethyl Hydroxyethylmonium Methosulfat (und) Propylene Glycol | 2 |
| | Cacao Butter (Nederland) | Theobroma cacao Seed Butter | 0,5 |
| | Structure XL (National Starch) | Hydroxypropyl Starch Phosphat | 5 |
| II. | Wasser, entionisiert | | 86,02 |
| | Glycerin | | 0,48 |
| III. | Konservierungsmittel | | q.s. |
| | Parfum | | q.s. |
| >> | pH Wert | | 4,3 |
| >> | Viskosität (mPas) Brookfield RVT, 20°C Spindel 5, 10 rpm | | 47600 |

Herstellung: 1. Schmelzen der Zutaten von Phase I bei 80-85°C, 2. Hinzufügen des Wassers bei derselben Temperatur während des Rührens. Diese Temperatur halten und für 30 Minuten rühren, 3. Abkühlen unter langsamen Rühren, 4. Anpassen des pH Wertes, wenn nötig mit Zitronensäure, 5. Hinzufügen des Konservierungsmittels und des Parfums bei unter 40°C.

**Deep penetrating Hair Reconstructor**

| Phase | Handelsname | INCI | Gew.- % |
|---|---|---|---|
| I. | Dehyquart® L 80 | Dicocoylethyl Hydroxyethylmonium Methosulfat (und) Propylene Glycol | 2 |
| | Undecan oder Tridecan | | 1 |
| | DC 949 (Dow Corning) | Amodimethicone (und) Cetrimonium Chloride (und) Trideceth-12 | 1 |
| | Gluadin® WLM | Hydrolyzed Wheat Protein | 2 |
| | Parfum | | q.s. |
| | Konservierungsmittel | | q.s. |
| II. | Lamesoft® PW 45 | Cetyl palmitate (and) Beheneth-10 (and) Hydrogenated Castor Oil (and) Glyceryl Stearate | 4 |
| | Wasser, entionisiert | | 37,75 |
| III. | Rheocare®CTH(E) (Cosmetic Rheologies) | Polyquaternium-37 (und) Propylene Glycol Dicaprylate/Dicaprate (and) PPG-1 Trideceth-6 | 2,25 |
| | Wasser, entionisiert | | 50 |
| >> | pH Wert | | 4 |
| | Viskosität (mPas) Brookfield RVT, 20°C Spindel 4,10 rpm | | 10500 |

Herstellung: 1. Mischen der Komponenten von Phase I bis zur vollständigen Homogenität, 2. Auflösen von Lamesoft® PW 45 in Wasser und hinzufügen über Phase 1, 3. Auflösen von Rheocare Polymer in entionisiertem Wasser bis man eine homogene Creme erhält, 4. Hinzufügen von Phase I + II über Phase III, 5. Anpassen des pH Wertes wenn nötig.

**Hair Conditioner Classic Care**

| Phase | Handelsname | INCI | Gew.-% |
|---|---|---|---|
| I. | Lanette® O | Cetearyl Alcohol | 4,5 |
| | Dehyquart® F 75 | Distearoylethyl Hydroxyethylmonium Methosulfat (und) Cetearyl Alcohol | 1,4 |
| | Cutina® GMS V | Glyceryl Stearate | 0,5 |
| | Undecan oder Tridecan | | 0,2 |
| | DC 200-1000 (Dow Corning) | Dimethicone | 0,2 |
| II. | Dehyquart® A-CA | Cetrimonium Chloride | 2 |
| | Wasser, entionisiert | | 91,2 |
| III. | Konservierungsmittel | | q.s. |
| | Parfum | | q.s. |
| | pH Wert | | 3,9 |
| | Viskosität (mPas) Brookfield RVT, 20°C Spindel 4,10 rpm | | 19700 |

Herstellung: 1. Mischen von Phase I bei 80-85°C bis zur Homogenität, 2. Hinzufügen von Phase II über Phase I bei derselben Temperatur (80-85°C) und Rühren. Weiterrühren während 30 Minuten, 3. Abkühlen bei langsamen Rühren und hinzufügen von Phase III bei einer Temperatur von weniger als 40°C, 4. Anpassen des pH Wertes wenn nötig (pH Wert ist = 3,5 - 4,5).

**Haar Glanz Nebel ("Mist")**

| Handelsname | INCI | Gew.-% |
|---|---|---|
| Undecan oder Tridecan | | **80** |
| Cetiol® CC | Dicaprylyl Carbonat | 15 |
| Dow Corning® 200 Fluid (Dow Corning) | Dimethicone | 2,7 |
| Neo® Heliopan AV (Haarmann & Reimer) | Ethylhexyl Methoxycinnamate | 1,5 |
| Wacker PDM 1000 | Trimethylsiloxyphenyl Dimethicone | 0,5 |
| Parfum | | 0,3 |
| pH Wert | | N.A. |
| Viskosität bei 20 °C (mPas) | | < 100 |

| | | |
|---|---|---|
| Herstellung: Mischen der Zutaten in der angegebenen Reihenfolge | | |

**Sprühbarer Hair Conditioner, leave-on**

| Phase | Handelsname | INCI | Gew.-% |
|---|---|---|---|
| I. | Monomuls® 60 35 C | Hydrogenated Palm Glycerides | 1,24 |
| | Eumulgin® B1 | Ceteareth-12 | 2,76 |
| | Cetiol® S | Dioctylcyclohexane | 9 |
| | Cetiol® OE | Dicaprylyl Ether | 9 |
| | Undecan oder Tridecan | | 2 |
| II. | Wasser | | 56,2 |
| | Gluadin® WQ | Laurdimonium Hydroxypropyl hydrolyzed wheat protein | 2,85 |
| | Plantacare® 1200 UP | Lauryl Glucoside | 1 |
| | Konservierungsmittel | | q.s. |
| | Parfum | | q.s. |
| | Viskosität mPas | | |

Herstellung: 1.Erhitzen von Phase I auf 85°C und Rühren bis es homogen ist, 2. Erhitzen von Phase II auf 85°C und langsam hinzufügen zu Phase I während des Rührens, 3. Hinzufügen von Phase III (kalt) bei 78°C zu PhaseI/II während des Rührens, 4. Der Emulsion erlauben Abzukühlen während des Rührens, welches die Emulsion ständig in Bewegung hält. Vermeiden von Lufteinschluss. Hinzufügen von Phase IV bei 30°C.

**Cream to Powder Foundation**

| | Komponente INCI Bezeichung (Handelsname, Hersteller) | Gew.% |
|---|---|---|
| A | Diisostearyl malate (Corum 5015, Corum) | 7,7 |
| | Titanium dioxide (HD-0748, Lubrizol/Noveon) | 12,6 |
| | Iron oxides (HD-3170, Lubrizol/Noveon) | 1,25 |
| | Iron oxides (HD-3511, Lubrizol/Noveon) | 0,2 |
| B | Dimethicone (and) dimethicone crosspolymer (DC 9041 Sillcone Elastomer Blend, Dow Corning) | 8 |
| | Undecan oder Tridecan | 5 |
| | Ethylhexyl Isononanoate (Corum 5021, Corum) | 6 |
| | Pentaerythryl tetraisostearate (Corum 5041, Corum) | 7 |
| | Polyisobutane (and) C₁₅₋₁₉ alkane (and) C₁₂₋₁₄ isoparaffin (Permethyl 284C, Presperse) | 3 |
| | Cholesteryl hydroxystearate (Corum 5-89, Corum) | 0,5 |
| | Carnauba Wachs | 4 |
| | Cetyl Palmitate (Corum 5000, Corum) | 0,6 |
| | Myristyl myristate (Corum 5028, Corum) | 0,6 |
| | Tribehenine (Corum 5094, Corum) | 0,5 |
| | Mikrocristallines Wachs | 1,5 |
| | Methyl methacrylate crosspolymer (Glanzpearl GMX 0610, ISP) | 5,5 |
| | Boron nilinde (Soltouch CC6059, Momantive) | 1 |
| | Talk (und) methicone (Corum 5901, Corum) | 11,67 |
| | Mica (und) methicone (und) Parffinium liquidum (mineral) oil (Sericite SL012P, Nikko)10 | |
| | Lauroyl lysine (Corum 5106, Corum) | 6 |
| C | Pentaerythryl tetraisostearate (und) Paraffinium liquidum (mineral) oil | 3 |
| | (und) disteardimonium hectorite (und) propylene carbonate (und) palmitoyl tripeptide (Corum 9913, Corum) | |
| | Pentaerythryl tetraisostearate (und) Paraffinium liquidum (mineral) oil (und) disteardimonium hectorite (und) propylene carbonate (und) palmitoyl oligopeptide (Corum 8616, Corum) | 3 |
| | Phenoxyethenol (und) methylparaben (und) butylparaben (und) ethylparaben (und) propylparaben (Paragon MEPB, Molmyre) | 1 |
| | Parfum | 0,06 |

Herstellung: Vormischung von A herstellen, B zu A geben und auf 90 °C erhitzen, gut mischen, Hitzezufuhr einstellen und C zugeben.

**Makeup foundation**

| Komponente INCI Bezeichung (Handelsname, Hersteller) | Gew.-% |
|---|---|
| Wasser | 78,2 |
| Glycerin | 1,89 |
| Triethanolamine 99% | 0,75 |
| Methylparaben (Nipagin M. Nipa) | 0,19 |
| Xanthan gum (Keltrol SP, CP Kelco) | 0,28 |
| Titanium dioxid (Softex Titanium dioxide, C47-7756, Sun Chemical) | 8,75 |
| Brown iron oxid (SunChroma Brown iron oxide, C33-315 Sun Chroma, Sun Chemical) | 1,08 |
| Red iron oxide (SunChroma red iron oxide, C33-124 Sun Chroma, Sun Chemical) | 0,17 |
| Phenyl trimethicone (DC 556 cosmetic Fluid, Dow Corning) | 3,41 |
| Undecan oder Tridecan | 1 |
| Stearic acid, 94% | 1,33 |
| Cetyl stearyl alcohol (Lanette O, Cognis) | 2,84 |
| Propylparaben (Nipasol, M. Nipa) | 0,1 |

**"Skin Corrector"**

| Komponente INCI Bezeichung (Handelsname, Hersteller) | Gew.-% |
|---|---|
| Synthetisches Wachs (Cirebelle 108, Cirebella) | 2,9 |
| Undecan oder Tridecan | 19,51 |
| Synthetisches Wachs (Cirebelle 303, Cirebella) | 2,33 |
| Titanium dioxid Wachs dispersion (CWD-8906, Sun Chemical) | 65,12 |
| Yellow iron oxide wax dispersion (CWD-8942, Sun Chemical) | 4,18 |
| Red iron oxide wax dispersion (CWD-8801, Sun Chemical) | 1,91 |
| Black iron oxide wax dispersion (CWD-9921, Sun Chemical) | 0,05 |
| Alumina (und) titanium dioxid (SpectraFlex Focus White C88-1001, Sun Chemical) | 4 |

**Matte Antiaging Foundation**

| Phase | Komponente INCI Bezeichung (Handelsname, Hersteller) | Gew.-% |
|---|---|---|
| A | Cyclopentasiloxane (und) dimethicone copolyol (DC 5225C, Dow Corning) | 12 |
| | Undecan oder Tridecan | 10 |
| | Titanium dioxid (BTD-401, Kobo) | 9 |
| | Yellow iron oxide (BYO-12, Kobo) | 0,8 |
| | Red iron oxide (BRO-12, Kobo) | 0,07 |
| | Black iron oxide (BRO-12, Kobo) | 0,034 |
| | Methylmethacrylate crosspolymer (Sepimat SB, Seppic) | 1 |
| | Methylmethacrylate crosspolymer (Sepimat HB V, Seppic) | 1 |
| | Phenoxyethanol (und) methylparaben (und) ethylparaben (und) propylparaben (und) butylparaben (Sepicide HB, Seppic) | 17,296 |
| | Parfum | 0,3 |
| B | Dipalmitoyl hydroxyproline (Sepilift DPHP, Seppic) | 0,5 |
| | Palmitoyl praline (und) magnesium palmitoyl-glutamate (und) sodium palmitoyl sarcosinate (Sepifeel One, Seppic) | 05, |
| | Undecylenoyl phenylalanine (Sepiwhite MSH, Seppic) | 0,1 |
| C | Sodium hydroxide, 48% Lösung | 0,2 |
| | Glycerin | 5 |
| | Sodium chloride | 2 |
| | Imidazolidinyl urea (Sepicide Cl, Seppic) | 0,2 |
| D | Wasser | 40 |

Herstellung: Phase A wird hergestellt durch Dispersion der Puder in den Silikonen und den n-Undecan/n-Tridecan unter Rühren, in Kugelmühle zerkleinern. Phasen B, C und D werden separat hergestellt und jeweils auf 80 °C erhitzt. Phase D wird zu Phase C hinzugefügt, dieses Gemisch wird dann zu B gegeben. Homogenisierung für einige Minuten, Abkühlen unter mäßigem Rühren. Diese Mischung wird dann unter heftigem Rühren zu Phase A gegeben und die entstehende Emulsion wird homogenisiert.

**Super Matte Foundation**

| Phase | Komponente INCI Bezeichung (Handelsname, Hersteller) | Gew.-% |
|---|---|---|
| A | Wasser | 54,72 |
| | Disodium EDTA | 0,1 |
| | Wasser (und) montmorillonite (und) cetyl alcohol (und) glycerin (und) pentylene glycol (und) sucrose stearate (und) aderoglucan (Matte Lite Concentrate 269, MMP) | 20 |
| B | Sucrose distearate (Sistearna SP30-C, MMP/Sisterna) | 0,75 |
| C | Titanium dioxide | 6 |
| | Red iron oxide | 0,5 |
| | Yellow iron oxide | 1,25 |
| | Black iron oxide | 0,13 |
| | Nylon 12 | 0,25 |
| D | Dimethicone (und) dimethicone/vinyl dimethicone crosspolymer (Clearocast 200, MMP) | 5 |
| | Undecan oder Tridecan | 1 |
| | Isododecane (und) Isononyl Isononanoate (Clearocast 550, MMP) | 10 |
| E | Konservierungsmittel | 0,3 |

**"Chocolate Mousse"**

| Phase | Komponente INCI Bezeichung (Handelsname, Hersteller) | Gew.-% |
|---|---|---|
| A | Cl 77891 dimethicone (SAT-T-47051, US cosmetics) | 0,528 |
| | Cl 77491 dimethicone (SAT-R-33128, US cosmetics) | 0,456 |
| | Cl 77492 dimethicone (SAT-Y-33873, US cosmetics) | 0,732 |
| | Cl 77499 dimethicone (SAT-B-33134, US cosmetics) | 0,284 |
| B | Dimethicone/vinyl dimethicone crosspolymer (und) silica (Dow Corning 9701 Cosmetic Powder, Dow Corning) | 23 |
| | Zink oxid (und) dimethicone (Z-cote HP-1, BASF) | 6 |
| C | Wasser | 4 |
| | Wasser (und) propylene glycol (und) Helianthus annuus (Sonnnenblume) seed oil (und) Theobroma cacao extract (und) sclarolum gum (Cocoa Phytolat, Alban Muller) | 1 |
| | Dimethicone (Dow Corning 200 Fluid, 5cSt, Dow Corning) | 22,75 |
| | Undecan oder Tridecan | 22,75 |
| | Cyclopentasiloxane (und) dimethiconol (Dow Corning 1501 Fluid, Dow Corning) | 5 |
| | Cyclopentasiloxane (und) dimethicone crosspolymer (und) dimethiconol (Dow Corning 9546 Silicone Elastomer Blend, Dow Corning) | 5 |
| | Theobroma cacao seed butter (Cocoa Butter, Alban Muller) | 1 |
| | Cocos nucifera (Kokosnuss) Öl (und) Gardenia tahitensis Blüten Extrakt (Monoi de Tahip Butter frangaced, Pacific Sud Cosmetique) | 1 |
| | Tocopherol acetate (di-alpha-Tocopherol Acetate, DSM) | 0,5 |
| | Polysilicone-15 (Parsol-SLX, DSM) | 5 |
| D | Parfum (Chocolat Creme 0310585, Expressions Perfumées) | 1 |

Herstellung: A wird vermahlen, und B wird zugefügt und gerührt. Phase C wird vorgelegt und unter Rühren und auf 45 °C erhitzt. Dann wird AB in kleinen Portionen zugefügt und zum Schluss wird D zugegeben.

**Fresh Foundation Emulsion Mousse**

| Phase | Komponente INCI Bezeichung (Handelsname, Hersteller) | Gew.-% |
|---|---|---|
| A | Ceteareth-25 (und) PEG-2 stearate (und) Paraffinium liquidum (mineral) oil (und) hydrogenated coconut oil (und) cetyl alcohol (und) sodium stearate (Base O/W 097, LCW) | 5 |
| | Caprylic/capric triglyceride | 15 |
| | Undecan oder Tridecan | 1 |
| B | Wasser | 59 |
| | Konservierungsmittel | 0,3 |
| | Glycerin | 2 |
| | Algas extract (und) sorbitol (Fucosorb, LCW) | 2 |
| C | Cl 77499 (und) silica (Unipure Black LC 989 EM, LCW) | 0,1 |
| | Cl 77492 (und) silica (Unipure Yellow LC 162 EM, LCW) | 0,75 |
| | Cl 77491 (und) silica (Unipure Red LC 361 EM, LCW) | 0,18 |
| | Cl 77891 (und) silica (Unipure white LC 981 EM, LCW) | 2,95 |
| | Mica (Submica FL, LCW) | 1 |
| | Methylmethacrylate crosspolymer (und) silica (Covabeed PMMA 2MUSL, LCW) | 1 |
| | Mica (Mica 8 R2041, LCW) | 4 |
| | Talk (Talc LCW, LCW) | 4 |
| | Sodium plyacrylate (Covacryl MV50, LCW) | 1 |
| | Silica dimethyl silyata (Covasilic 15, LCW) | 0,5 |

Herstellung: A und B werden getrennt hergestellt und jeweils auf 70°C erhitzt. B wird zu A unter Rühren zugefügt. Phase C wurde separate hergestellt und mit einem Pulvermischer gut durchmischt und danach unter Rühren zur Mischung aus A und B gegeben.

**Mattifying Fluid Foundation**

| Phase | Komponente INCI Bezeichung (Handelsname, Hersteller) | Gew.-% |
|---|---|---|
| A | Wasser | 20 |
| | Butylene glycol | 4 |
| | PEG-400 | 4 |
| | Dimethicone copolyol PEG-7 phosphate (Pecostil PS 100, Pheonix) | 1 |
| B | Titanium dioxide | 10 |
| | Talk | 2 |
| | Iron oxide yellow | 0,9 |
| | Iron oxide red | 0,3 |
| | Iron oxide black | 0,05 |
| | Titanium dioxide (Cl 77891) (und) iron oxide (Cl 77491) (und) mica (und) triethoxy caprylylsilane | 3 |
| C | C₂₀₋₂₂ alkyl phosphate (und) C₂₀₋₂₂ alcohols (Sensanov WR, Seppic) | 1 |
| | C₁₄₋₂₂ alcohol (und) C₁₂₋₂₀ alkylglucoside (Montanov L, Seppic) | 2 |
| | Isostearyl isostearat | 8 |
| | Undecan oder Tridecan | 1 |
| | Ethylhexyl palmitate | 9 |
| | Vegetable oil (und) palm alcohol (und) lecithin (und) vegetable stearyl esters (Sensactive Veg. Chemyunion) | 2 |
| D | Wasser | q.s. to 100 |
| | Xanthan gum | 0,15 |
| E | Tromethamine | q.s. |
| | Tetrasodium EDTA | 0,05 |
| | Polymethyl methacrylate (Micropearl M100, Seppic) | 3 |
| F | Sodium acrylate/acryloyldimethyllaurate copolymer (und) isohexadecanle (und) polysorbane (Simulgel EG, Seppic) | 0,5 |
| G | Phenoxyethanol (und) methylparaben (und) ethylparaben (und) propylparaben (und) butylparaben (Chemynoi, Chemyunion) | 0,1 |
| | Parfum | qs |

**Ultra gloss Lippenstifte**

| Komponente INCI Bezeichung (Handelsname, Hersteller) | Gew.-% |
|---|---|
| Polyglyceryl-10 hydroxystearate/stearata/eicosadioate (und) dextrin palmitate (Nikkol Nikkowax LM, Nikko) | 5 |
| Copernicia cerifera (carnauba) Wachs | 10 |
| Cera alba (Bienenwachs) | 15 |
| Cetyl alcohol | 5 |
| Ricinus communis (castor) seed oil | 63 |
| Undecan oder Tridecan | 2 |

| | |
|---|---|
| Herstellung: Mischung erhitzen und bei 80°C schmelzen und in Gießform füllen. | |

**Maximum shine Lippenstifte**

| Phase | Komponente INCI Bezeichung (Handelsname, Hersteller) | Gew.-% |
|---|---|---|
| A | D&C Red No. 6 Barium Lake (Red 6 lake, Emerald Hilton Davis) | 0,7 |
| | Iron oxides (CC33-5136 Russet Iron Oxide, Sun Chemical) | 2,5 |
| | Iron oxides (CC33-135 Black iron oxide, Sun Chemical) | 0,25 |
| | Ricinus communis (castor) seed oil (Crystal O, CasChem) | 3 |
| B | Ricinus communis (castor) seed oil (Crystal O, CasChem) | 35,75 |
| | Copernica cerifera (carnauba) Wachs (Carnauba No. 1, Strahl & Pitsch) | 1,1 |
| | Euphorbia cerifera (candelilla) Wachs (Candelilla Pure, Strahl & Pitsch) | 5 |
| | Ozokerite (Ozokerite Wax 1700, Frank B, Ross Company) | 2,5 |
| | Undecan oder Tridecan | 1 |
| | Mikrocrystallines Wachs (Microwax SP 19, Strahl & Pitsch) | 2 |
| | Diisostearyl malata (Schercamol DISM Ester, Lubrizol/Noveon) | 24 |
| | Trisostearyl citrate (Schercomol TISC Ester, Lubrizol/Noveon) | 16,85 |
| | Methylparaben | 0,2 |
| | Propylparaben | 0,1 |
| C | Mica (und) iron oxides (Colorona Copper, EMD chemicals) | 5 |
| D | Ascorbyl palmitate | 0,05 |

**Ultra lasting lip color**

| Phase | Komponente INCI Bezeichung (Handelsname, Hersteller) | Gew.-% |
|---|---|---|
| A | D&C Red No. 7 Calcium Lake (31-DA-3707, Emerald Hilton Davis) | 0,6 |
| | D&C Red no. 6 Barium Lake (31-DA-3006, Emerald Hilton Davis) | 1,5 |
| | Iron oxides (CC33-5138 Russet iron oxide, Sun Chemical) | 4,5 |
| | Titanium dioxide | 0,5 |
| | Ricinus communis (castor) seed oil (Crystal O, CasChem) | 8,3 |
| B | Ricinus communis (castor) seed oil (Crystal O, CasChem) | 21,65 |
| | Copernica cerifera (carnauba) Wachs (Carnauba No. 1, Strahl & Pitsch) | 1,5 |
| | Euphorbia cerifera (candelilla) Wachs (Candelilla Pure, Strahl & Pitsch) | 6 |
| | Ozokerite (Ozokerite Wax 1700, Frank B. Ross Company) | 2,5 |
| | Mikrocrystallines Wachs (Microwax SP 19, Strahl & Pitsch) | 3,5 |
| | Triisostearyl polyglyceryl-3 dimer diinoleate (Schercemol PTTD Ester, Lubrizol/Noveon) | 10 |
| | Trisostearyl citrate (Schercomol TISC Ester, Lubrizol/Noveon) | 22 |
| | Isopropyl Isostearate (Schercamol 316 Ester, Lubrizol/Noveon) | 4 |
| | Undecan oder Tridecan | 1 |
| | Methylparaben | 0,2 |
| | Propylparaben | 0,1 |
| C | Mica (und) Titanium dioxide (Flamenco Red, Engelhard) | 12 |
| D | Ascorbyl palmitate (Ascorbyl palmitate, DSM) | 0,05 |

**Long lasting Shine Lip Gloss**

| Phase | Komponente INCI Bezeichung (Handelsname, Hersteller) | Gew.-% |
|---|---|---|
| A | Octyldodecanol | 47,93 |
| B | Ethylcellulose (Ethocel 100FP, Amerchol) | 5 |
| C | Undecan oder Tridecan | 18 |
| | bis-Biglyceryl polyacladipate-2 | 15 |
| | Octyl methoxycinnamate | 7,5 |
| | Polyethylene | 2 |
| D | Red No. 7 Aluminium lake (Sun Chemical) | 0,58 |
| | Iron oxide (Cl 77491, Sun Chemical) | 0,52 |
| | Iron oxide (Cl 77492, Sun Chemical) | 0,48 |
| | Iron oxide (Cl 77499, Sun Chemical) | 0,09 |
| E | Mica (und) titanium dioxide (Prepearis Shining Salin, Prespense) | 1,9 |
| | Dimethicone crosspolymer/silica (DC 7901 Powder, Dow Corning) | 1 |

**High-shine Lippenstift**

| | Komponente | Gew.-% |
|---|---|---|
| A | Hydrogenated Polydecene (und) ethylene propylene/styrene copolymer (und) butylene/ethylene/styrene copolymer (Versagel MG 750, Penreco) | 25,89 |
| | Petrolatum (und) ethylene/propylene/styrene copolymer (und) butylene/ethylene/styrene copolymer (Versagel P100, Penreco) | 10,38 |
| | Euphorbia cerifera (candelilla) wachs (Candelilla wax refined, Ross) | 8,8 |
| | Cera alba (Bienenwachs) (White Bleached Beeswax, Ross) | 5,18 |
| | Lanolin Wachs | 4,14 |
| | Lanolin Öl | 4,14 |
| | Octyldodecanol (Eutanol® G, Cognis) | 4,14 |
| | Undecan oder Tridecan | 3,63 |
| | Copernicia cerifera (carnauba) Wachs (Carnauba wax, Ross) | 3,11 |
| | Octyldodecyl stearoyl stearate (Ceraphyl 647, ISP) | 3,11 |
| | Tridecyl trimellitate (Liponate TDTM, Lipo) | 3,11 |
| | Propylparaben | 0,1 |
| | Butylated hydroxytoluene | 0,1 |
| B | Butyrospermum parkii (shea butter) (Shea Butter, Ultra refined, Biochemica) | 5,18 |
| | Jojoba esters (Floraesters 30, Floratech) | 2,07 |
| | Theobroma grandiflorum seed butter (Cupuacu Butter, Beraca/Ross) | 2,07 |
| C | Iron oxides (und) synthetic fluorphlogopite (Sunshine Super Russet, Sun Chemical) | 8,29 |
| | Iron oxides (Soft-Tex Russet Iron Oxide, Sun Chemical) | 5,18 |
| | Red No. 7 Lake | 1,04 |
| | Yellow No. 6 Lake | 0,38 |

**Eleganter Lippenstift**

| Komponente INCI Bezeichnung (Hersteller) | Gew.-% |
|---|---|
| Dipentaerythrityl hexacaprylate/hexacaprate (und) tridecyl trimellitate (und) tri-decyl stearate (und) neopentyl glycol dicaprylate/dicaprate (Lipovol MOS-350, Lipo) | 72,2 |
| Synthetisches Bienenwachs (Lipobee 102, Lipo) | 8 |
| Copernicia cerifera (carnauba) Wachs | 4 |
| Euphorbia cerifera (candelilla) wachs | 4 |
| Undecan oder Tridecan | 1 |
| Propylparaben | 0,2 |
| Mica (und) boron nitride (Lipomic 501 BN, Lipo) | |
| Tocopherol (Vitamin E Acetate, Ruger Chemical) | 0,5 |
| Titanium dioxide (Cl 77891) (und) dipentaerythrityl hexacaprylate/hexacaprate (und) tridecyl trimellitate (und) tridecyl stearate (und) neopentyl glycol dicaprylate/dicaprate (Titanium dioxide, 60% in Lipovol MOS-350, Lipo) | 2,8 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Red, 60% in Lipovol MOS-350, Lipo) | 4,7 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Yellow, 60% in Lipovol MOS-350, Lipo) | 0,8 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Black, 60% in Lipovol MOS-350, Lipo) | 0,8 |

Herstellung: Alle Bestandteile werden unter Rühren auf 80-85 °C erhitzt. Der Ansatz wird auf 70-75°C abgekühlt und in eine Gießform gefüllt.

**Lip Gloss**

| | Komponente INCI Bezeichnung (Hersteller) | Gew.-% |
|---|---|---|
| A | Undecan oder Tridecan | 2 |
| | C₄₋₂₄ alkyl dimethicone/divinyldimethicone crosspolymer dimethicone (NuLastic Silk MA, DM, Alzo) | 15 |
| | Methylheptyldiisostearate (Beantree, Alzo) | 10 |
| | C₄₋₂₄ alkyl dimethicone/divinyldimethicone crosspolymer dimethicone (NuLastic Silk MA, DM-6, Alzo) | q.s to 100 |
| | Tocopherol (Vitamin E, BASF) | 0,2 |
| | Konservierungsmittel | qs |
| | Parfum (Peach Nectar 6104085, Bell Flavors & Fragances) | 0,2 |
| B | Calcium Sodium borosilicate (und) Iron oxides (Reflecks Dimensions Shiny Bronze G25000000, BASF) | 1 |
| | Mica (und) Titanium dioxide (Flamenco Winter Sparide 1300000 | 5 |
| | Hydrogenated polyisobutene (Luvitol Lite, BASF) | 10 |

Herstellung: Phasen A und B werden in getrennten Gefäßen jeweils homogenisiert, dann wird Phase B zu Phase A gefügt und gemischt, bis das Produkt homogen vorliegt.

**Creamy Sheer Lip Colour**

| | Komponente INCI | Gew.-% |
|---|---|---|
| A | Sesamum indicum (Sesam) Samen Öl | 21,28 |
| | Octyldodecanol (Jarcol I-20, Jarchem) | 15,47 |
| | Euphorbia cerifera (candelilla) wachs (SP75, Strahl & Pitsch) | 12 |
| | Isohexyl Caprate | 10,07 |
| | Bis-Diglyceryl polyaryladipate-2 (Softisan 649, Sasol) | 9,95 |
| | Polyisobutene (Permethyl 104A, Presperse) | 9,67 |
| | Undecan oder Tridecan | 2,89 |
| | Polyethylene (Performalene PL Polyethylene, New Phrase) | 2,5 |
| | Mikrocrystallines Wachs (Be Square 195, New Phrase) | 2,5 |
| B | Synthetic wax and titanium dioxide (und) Isopropyl Titanium triisostearate (SW65U, Kobo) | 4,24 |
| | Synthetic wax and iron oxide (und) Isopropyl Titanium triisostearate (SW60ER, Kobo) | 1,41 |
| | Synthetic wax and D&C Red No. 6 Barium Lake (und) Isopropyl Titanium triisostearate (SW40R6E, Kobo) | 1,41 |
| | Synthetic wax and Blue No.1 Lake (und) Isopropyl Titanium triisostearate (SW30B1A, Kobo) | 0,71 |
| | Mica (und) titanium dioxide (KTZ Classic White, Kobo) | 0,96 |
| C | Vitis vinifera (grape) seed oil | 4,84 |
| | Vitamin E acetata | 0,1 |

**Lip volume booster**

| | INCI Bezeichnung (Handelsname, Hersteller) | Gew.-% |
|---|---|---|
| A | Ricinus communis (castor) oil | 39,7 |
| | Hydrogenated coco-glycerides (Softisan 100, Sasol) | 8 |
| | Candelilla cera (und) Paraffin (Cenilla G, Barlocher) | 9 |
| | Bis-Diglyceryl polyacyladipate-1 (Softisan 645, Sasol) | 8 |
| | Cera alba (Cerabell White, Barlocher) | 3 |
| | Cera carnauba Wachs (Cernauba T1, Barlocher) | 2 |
| | Tricaprylin (Trivant OC-G, Trivant Chemical) | 20 |
| | Tocopheryl acetate (D-alpha-Tocopheryl Acetate, DSM/Roche) | 0,5 |
| | Undecan oder Tridecan | 1 |
| | Propylparaben (Nipasol M. Nipa) | 0,1 |
| | BHT (Butylhydroxytoluol) | 0,05 |
| B | Ricinus communis (castor) oil (und) Cl 15850 (und) BHT (COD 8001, Sun Chemical) | 0,8 |
| | Calcium aluminum borosilicate (und) Cl 77891 (und) silica (und) tin oxide (Fionastar Noble Sparks, Merck) | 1 |
| | Mica (und) Cl 77891 (und) Cl 77491 (Timiron MP29 Sun Gold Sparkle, Merck) | 2 |
| | Mica (und) Cl 77891 (Timiron MP 149 Diamond Cluster, Merck) | 2 |
| C | Glycerin (und) palmitoyl tripeptide-3 (Syn-Coll, Pentapharm) | 2,5 |
| | Alkyl methacrylate crosspolymer (Poly-Pore E200, Amcol HBS) | 0,35 |

Herstellung: alle Bestandteile der Phase A auf 80°C erhitzen, Phase B zu A hinzufügen, Phase C mischen und zu AB zugeben kurz vor der Homogenisierung.

**Plumping effect Lippenstift**

| | Komponenten INCI Bezeichnung (Handelsname, Hersteller) | Gew.% |
|---|---|---|
| A | D&C Red No. 7 Ca Lake (Cl 15850:1) (HD-3007, Noveon) | 0,4 |
| | Red iron oxide (Cl 77491) (HD-3511, Noveon) | 0,4 |
| | Titanium dioxide Cl 77891 (HD-0748, Noveon) | 1,2 |
| | Paraffinum liquidum (mineral) oil (und) disteardimonium hectorite (und) propylene carbonate (Bentone Gel MO V, Elementis Specialities) | 1,5 |
| | Diisostearyl maleate (Corum 5015, Corum) | 10,5 |
| | Mica (und) titanium dioxide (und) tin oxide (Prestige Sparkling blue, Eckart) | 1,5 |
| B | Pentaerythrityl tetraisostearate (Corum 5041, Corum) | 15 |
| | Cholesteryl hydroxystearate (Corum 5069, corum) | 1 |
| | Hydrogenated polydecene (Nexbase 2006FG, Fortum) | 15 |
| | Undecan oder Tridecan | 10 |
| | Isostearyl isostearate (corum 5088, Corum) | 19,9 |
| | Phenyltrimethicone (DC 556, Dow Corning) | 2 |
| | PEG-8 Bienenwachs (Corum 2680, Corum) | 2 |
| | Ceresin | 9 |
| | Euphorbia cerifera (candelilla) wachs | 2 |
| | Cera alba (Bienenwachs) | 2 |
| | Copernicia cerifera (carnauba) Wachs | 3 |
| | Jojoba ester (Floraester-70, Floratech) | 1 |
| | Pentaerythrityl tetraisostearate (und) Paraffinum liquidum (mineral) oil (und) disteardimonium hectorite (und) propyl carbonate (und) palmitoyl oligopeptide (Corum 8813, Corum) | 2 |
| C | Vanilyl butyl ether (Corum 9235, Corum) | 0,4 |
| | Flavor | 0,2 |

Herstellung: Phase A wird zur Homogenität gemischt, die Phase B wird hinzugefügt und auf 90°C erhitzt und gut gemischt. Die Hitzezufuhr wird gestoppt und Phase C hinzugefügt, danach gut gemischt.

**Lippenstift**

| | Komponenten INCI Bezeichnung (Handelsname, Hersteller) | Gew.-% |
|---|---|---|
| A | Propylene glycol dicaprylate/dicaprate (und) bis-diglyceryl polyacyladipate-1 (und) bis-diglyceryl polyadipate-2 (Softisan Gel, Sasol) | 9 |
| | Stearalkonium hectorite (und) propylene carbonate (Softisan 649, Sasol) | 6 |
| | Hydrogenated coco-glycerides (Softisan 100, Sasol) | 22,5 |
| | Hydrogenated palm oil (Softisan 154, Sasol) | 3 |
| | C₁₂₋₁₀ alkyl octanoate (Cosmacol EDI, Sasol) | 5 |
| | Petrolsturm (Merkur Vaseline773, Merkur) | 11 |
| | Cera alba (Bienenwachs) | 7 |
| | Cyclomethicone (Beisli CM 040, Wacker Chemie) | 3 |
| | Undecan oder Tridecan | 4 |
| | Ricinus communis (castor) oil | 6 |
| | Paraffin | 14 |
| | Antioxidans | qs |
| B | Titanium dioxide (Cl 77891) (Timiron Splendid Red, Merck) | 3,5 |
| | Mica (und) Titanium dioxide (Cl 77891) (Timiron Starlusler MP-115, Merck) | 2,5 |
| | Mica (und) Titanium dioxide (Cl 77891) (Timiron Super Silver Fine, Merck) | 3 |
| C | Parfum | qs |
| | Tocopheryl acetate | 0,5 |

**Volumizing Mascara**

| | Komponenten INCI Bezeichnung (Handelsname, Hersteller) | Gew.-% |
|---|---|---|
| A | Cera alba (Bienenwachs) (White Beeswax SP422P, Strahl & Pitsch) | 2,76 |
| | Glyceryl stearate | 2,3 |
| | Copernicia cerifera (carnauba) Wachs (Carnauba wax SP63, Strahl & Pitsch) | 1,98 |
| | Stearic acid | 3 |
| | Ethylcellulose (Ethocel Standard 100FP Premium, Amerchol) | 3 |
| | Undecan oder Tridecan | 1 |
| | Octyldodecanol | 7 |
| | Premised Chitosan PCA, 2,5% (Kytamer PCA Polymer, Amerchol) | 15 |
| B | Iron oxide (Black Iron Oxide, Sun Chemical) | 6 |
| | Wasser | 55,45 |
| C | Triethanolamine 99% | 1,5 |
| D | Propylene glycol (und) diazolidinyl ures (und) methylparaben (und) propylparaben (NiPaguard, Clariant) | 1 |

**Lidschatten Stift (Eye shadow stick)**

| | Komponenten INCI Bezeichnung (Handelsname, Hersteller) | Gew.-% |
|---|---|---|
| A | Undecan oder Tridecan | 1 |
| | Ocryldodacyl neopentanoate (Elefac I-205, Alzo) | qs. to 100 |
| | Calcium sodium borosilicate (und) silica (und) titanium dioxide (Reflecks MultiDimensions Varying Violet G58000000, BASF) | 14 |
| | Mica (und) Titanium dioxide (und) iron oxides (Gemtone Moonstone G004, BASF) | 1 |
| B | Butylene glycol | 3,13 |
| | Acetylated glycol stearate (Unitwax, Guardian, ISP) | 22 |
| | Silica (Cab-o-Sil M5, Cabot) | 1,5 |
| C | Simmondsia chinensis (jojoba) seed oil (Lipovol J. Lipo) | 7,5 |
| | Stearic acid (Emersol 132, Cognis) | 3,6 |
| | Konservierungsmittel | qs |
| | Antioxidans | qs |

Phase B wird geschmolzen und gemischt, und dann zur vorgemischten Phase A gegeben. AB werden unter Rühren auf 80-05°C erhitzt und dann wird langsam Phase C zugegeben.

**Metallic Silver shine Mascara**

| | Komponenten INCI Bezeichnung (Handelsname, Hersteller) | Gew.-% |
|---|---|---|
| A | Wasser | 56,25 |
| | Xanthan gum (Keltrol T, CP Kelco) | 0,15 |
| | Magnesium aluminium silicate (Veegum HV, RT Vandervilt) | 0,55 |
| | Carbon black (und) wasser (MBD 201 20% Dispersion, Geotech) | 5 |
| B | Disodium EDTA (Edeta BD, BASF) | 0,05 |
| | Methylparaben (Methyl-4-hydroxybenzoate H 5501, Sigma Aldrich) | 0,3 |
| | Triethanolamine | 0,5 |
| | Propylene glycol | 2 |
| C | Aluminium powder (und) silica (Visionaire Bright Silver sea, Eckart) | 3 |
| D | Undecan oder Tridecan | 5 |
| | Cyclopentasiloxane (und) PEG/PPG-18/18 dimethicone (Dow Corning 5225C Formulation Aid, Dow Corning) | 1 |
| | Cera alba (Bienenwachs) (Ewacers 12, Wagner) | 10 |
| | Stearic acid (Kortacid 1695, Akzo Nobel) | 2 |
| | Glyceryl stearate (Imwitor 960K, Sasol Wax) | 2 |
| | Stearyl alcohol (Lanette 18, Cognis) | 2 |
| | Polyisobutene (Permethyl-104A, Chesham Chemicals) | 1 |
| | Phenoxyethanol (und) methylparaben (und) butylparaben (und) ethylparaben (und) propylparaben (und) isobutylparaben (Phenonip, Clariant) | 1,2 |
| | PVP polyvinylpyrrolidone (Luviskol K30 Powder, BASF) | 4 |
| | Carnauba Wachs (Ewacera 34, Wagner) | 4 |

Herstellung: A wird gemischt, B wird gemischt und zu A zugefügt, C wird zu AB gegeben und auf 75°C erhitzt. D wird separat vorgemischt und auf 75°C erhitzt und zu ABC gegeben, während des Abkühlens wird gerührt.

**Creamy Shadow Stick**

| | Komponenten INCI Bezeichnung (Handelsname, Hersteller) | Gew.-% |
|---|---|---|
| A | Undecan oder Tridecan | 38,5 |
| | PPG-3 myristyl ether (Varonic APM, Evonik, Degussa) | 7,0 |
| | Polyglyceryl-4- isostearate (and) cetyl PEG-PPG-10/1 dimethicone (and) hexyl laurate (Abil WE 09, Evonik, Degussa) | 1,0 |
| | Dimethicone, 10 mPas | 2,5 |
| | Cera alba (bees wax) | 4,5 |
| | Copernicia cerifera (carnauba) wax | 4,0 |
| | Ethylene/VA copolymer (A-C copolymer 400, Honeywell) | 2,5 |
| | Ozokerite | 5,8 |
| | C16-C36 acid triglyceride | 2,0 |
| | Isobutylparaben (and) isopropylparaben (and) butylparaben (Liquipar Oil, From Nature with Love) | 0,5 |
| B | Nylon-12 (egolon 12-10, Evonik, Degussa | 2,0 |
| | Titanium dioxide | 5,0 |
| | Chromium oxide green | 10,00 |
| | CI 77491 (and) aluminium powder (and) silica | 5,0 |
| | CI 77891 (and) CI 77288 (and)mica | 10,0 |

**Wasserfeste Mascara**

| | Komponenten INCI Bezeichnung (Handelsname, Hersteller) | Gew.-% |
|---|---|---|
| A | Wasser | 32,84 |
| | Magnesium aluminium silicate (Veegum HV, RT Vanderbilt) | 0,10 |
| | Xanthan gum (Keltrol 1000, Kelco Corp.) | 0,20 |
| | Methylparaben | 0,25 |
| | Trisodium EDTA (Protacide Na3 EDTA, Protameen) | 0,01 |
| | Premixed sodium acrylates/acrylnitrogens copolymer (Hydrilien 9, 2,5% gel. Lio - siehe unten) | 15,00 |
| B | Glycerin (and) polyester 5 (Lipo PE Base G-55, Lipo) | 12,00 |
| | Talc (Rose Talc, Prespersa) | 3,00 |
| | Iron oxide (CI 77499) (Iron Oxide Black, Ultra Chemical) | 9,00 |
| C | Triethanolamine, 99% | 1,00 |
| D | Synthetic bees wax (Lipobee 102, Lipo) | 10,00 |
| | Copernicia cerifera (carnauba) wax | 4,50 |
| | Undecan oder Tridecan | 5,50 |
| | C12-C16 alkyl benzoate (Liponate NEB, Lipo) | 1,00 |
| | Stearic acid (Lipo Stearic Acid, Lipo) | 3,00 |
| | Sorbitan sesquioleate (Liposorb SQO, Lipo) | 1,30 |
| | Propylparaben | 0,20 |
| E | Water (aqua) | 1,00 |
| | Imidazolidinyl urea (Leposerve IU, Lipo) | 0,10 |

Herstellung: Die Bestandteile der Phase A werden gemischt und auf 70 °C erhitzt, Phase A wird in einer Kolloidmühle gemahlen, die Bestandteile der Phase B werden in der angegebenen Reihenfolge zugefügt unter beständigem Mahlen, bis das Pigment gleichmäßig dispergiert ist, danach wird Phase C zugefügt. Die Bestandteile von C werden gemischt und bei 80-84°C gemischt (und dabei Entlüftet). Phase D wird zum Ansatz gegeben und emulgiert, der Ansatz wird auf 35 °C abgekühlt und die vorgemischte Phase E wird zugefügt. Bei 30°C wird der Ansatz in geeignete Gefäße abgefüllt.

Sodium acrylates/acrylnitrogens copolymer Premix: 97,50 Gew.- % Wasser und 2,5 Gew.- % Sodium acrylates/acrylnitrogens copolymer werden gemischt und auf 78-80°C erhitzt unter Rühren. Es wird für 15 bis 20 Minuten geführt bis die Lösung klar und homogen ist. Danach wird auf Raumtemperatur abgekühlt.

**Sebum resistenter Lidschatten**

| | Komponenten INCI Bezeichnung (Handelsname, Hersteller) | Gew.-% |
|---|---|---|
| A | Stearyl dimethicone (DC 2503 Cosmetic Wax, Dow Corning) | 10,00 |
| | C30-C45 alkyl methicone (and) C30-C45 olefin (DC AMS-C30 Cosmetic Wax, Dow Corning) | 5,00 |
| | Isododecane (and) arcylates/polytrimethylsiloxymethacrylate (DC FA 4002 ID Silicaone Acrylate, Dow Corning) | 10,00 |
| | Cyclopentasiloxane (and) triemthylsiloxymethacrylate (DC 749 Fluid, Dow Corning) | 51,00 |
| B | Cyclopentasiloxane (and) dimethicone crosspolymere (DC 9040 Silcone Elastomer Blend, Dow Corning) | 5,00 |
| | Undecan oder Tridecan | 3 |
| C | Silica silylate (DC VM-2270 Aerogel Fine Particles, Dow Corning) | 1,00 |
| | Mica | 15,00 |

Herstellung: Die Bestandteile der Phase A werden gemischt und auf 80 °C erhitzt, unter Rühren wird abgekühlt. Die Phase B wird gemischt und unter Rühren zur Phase A gegeben, C wird unter langsamem Rühren zugegeben.

**Lippenstift**

| | Inhaltsstoff | INCI Bezeichnung | Gew.-% |
|---|---|---|---|
| I | Cerilla | Candellila Wax | 9.00 |
| | Cerewax | Microcristallina Wax | 3.50 |
| | Cerozo | Ozokerite Wax | 2.50 |
| | Cerauba | Carnauba Wax | 2.50 |
| | Cetiol MM | Myristyl Mysristate | 5.00 |
| | Eutanol® G | Octyldodecanol | 10.00 |
| | Myritol® 331 | Cocoglycerides | 5.00 |
| | Cetiol® CC | Dicaprylyl Carbonate | 4.00 |
| | | Undecan oder Tridecan | 4.00 |
| | Cetiol® J600 | Oleyl Erucate | 1.00 |
| | Nipasol M | Propyl Paraben | 0.50 |
| | Lamesoft® TGI | Polyglyceryl-3 Diisostearate | 3.00 |
| | Cetiol® Sensoft | Propylheptyl Caprylate | 6.00 |
| II | COD 8008 | CI 77981 | 2.70 |
| | COD 8001 | CI 15850 | 12.90 |
| | COD 8010 | CI 15985 | 2.80 |
| | COD 8002 | CI 15850 | 1.40 |
| | COD 8007 | CI 42090 | 0.20 |
| | Ricinus Oil Codex | Ricinus Communis | 20.60 |
| III | | Fragance Astral 112 445 B | 0.40 |
| | Irwinol® LS 9319 | Octyldodecanol & Irvingia Gabonesis & Hydrogenated Coco-Glycerides | 2.50 |
| | Coviox® T70C | Tocopherol | 0.50 |

**Lippenpflege**

| | Inhaltsstoff | INCI Bezeichnung | Gew.-% |
|---|---|---|---|
| I | Polybutene M100 | Polybutene | 53.50 |
| | Cetiol® Sensoft | Propylheptyl Caprylate | 10.00 |
| | | Undecan oder Tridecan | 5.00 |
| | Isopropyl palmitate | Isopropyl Palmitate | 20.00 |
| | Cosmedia® DC | Hydrogenated Dimer Dilinoleyl Dimethylcarbonate Copolymer | 5.00 |
| | Red 28 Lake | Red 28 Lake | 0.05 |
| | | Perfume Fruits Rouges | 0.30 |
| | Coviox® T70C | Tocopherol | 0.10 |
| | Nipasol M | Propylparaben | 0.50 |
| II | Aerosil R972 | Silica Dimethyl Silylate | 5.30 |
| III | Ronastar Noble Spars | Calcium Aluminium Borosilicate (and) Silica (and) Titanium Dioxide | 0.25 |
| | Viskosität (mPa.s): Brookfield RVT, Spindle 5, Speed 10 11 000 | | |

**O/W Flüssig Foundation**

| | Inhaltsstoff | INCI Bezeichnung | Gew.-% |
|---|---|---|---|
| I | Deionized Water | Aqua | 50.50 |
| | Butylene Glycol | Butylene Glycol | 2.00 |
| | Glycerine | Glycerin | 3.00 |
| II | Veegum Ultra | Magnesium Aluminium Silicate | 1.00 |
| | Keltrol T | Xanthan Gum | 0.40 |
| III | Lameform® TGI | Polyglyceryl-3 Diisostearate | 1.50 |
| | Eumulgin® B2 | Ceteareth-20 | 3.50 |
| | Cutina® PES | Pentaerythrityl Distearate | 1.00 |
| | Cegesoft® SH | Shorea Stenoptera | 1.00 |
| | Cetiol® CC | Dicaprylyl Carbonate | 4.00 |
| | | Undecan oder Tridecan | 2.00 |
| | Myritol® 331 | Cocoglycerides | 4.00 |
| | Fitoderm® | Squalane | 3.00 |
| | Cetiol® Sensoft | Propylheptyl Caprylate | 5.00 |
| | Phenonip | Phenoxyethanol (and) Parabens | 0.80 |
| IV | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33-8001 | Iron Oxide CI 77491 | 0.60 |
| | SunPuro Black Iron Oxide C33-7001 | Iron Oxide CI 77499 | 0.20 |
| V | Cosmedia® Gel CC | Dicaprylyl Carbonate (and) Stearalkonium Hectorite (and) PropyleneCarbonate | 3.00 |
| VI | Micropearl M100 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosität (mPa.s): Brk. RVT spindle 5, speed 10 5 500, pH 7.3 | | | |

### Anhang

1) Abil^{®} EM 90
   INCI: Cetyl Dimethicone Copolyol
   Hersteller: Tego Cosmetics (Goldschmidt)
2) Allianz^{®} OPT
   INCI: Acrylates/C12-22 Alkyl
   Methacrylate Copolymer
   Hersteller: Rohm und Haas
3) Amphisol^{®} K
   INCI: Potassium Cetyl Phosphate
   Hersteller: Hoffmann La Roche
4) Antaron^{®} V 220
   INCI: PVP/Eicosene Copolymer
   Hersteller: GAF General Aniline Firm
   Corp. (IPS-Global)
5) Antaron^{®} V 216
   INCI: PVP/Hexadecene Copolymer
   Hersteller: GAF General Aniline Firm
   Corp. (IPS-Global)
6) Arlacel^{®} 83
   INCI: Sorbitan Sesquioleate
   Hersteller: Uniqema (ICI Surfacants)
7) Arlacel^{®} P 135
   INCI: PEG-30 Dipolyhydroxystearate
   Hersteller: Uniqema (ICI Surfacants)
8) Bentone^{®} 38
   INCI: Quaternium-18 Hectorite
   Hersteller: Rheox (Elementis Specialties)
9) Carbopol^{®} 980
   INCI: Carbomer
   Hersteller: Goodrich
10) Carbopol^{®} 2984
   INCI: Carbomer
   Hersteller: Noveon, Inc.
11) Carbopol^{®} ETD 2001
   INCI: Carbomer
   Hersteller: Noveon, Inc.
12) Carbopol^{®} Ultrez 10
   INCI: Carbomer
   Hersteller: Noveon, Inc.
13) Cegesoft^{®} C 17
   INCI: Myristyl Lactate
   Hersteller: Cognis GmbH, Grünau
14) Cegesoft^{®} PFO
   INCI: Passiflora Incarnata (EU)
   Hersteller: Cognis GmbH
15) Cegesoft^{®} PS 6
   INCI: Olus
   Hersteller: Cognis GmbH
16) Ceraphyl^{®} 45
   INCI: Diethylhexyl Malate
   Hersteller: International Specialty Products
17) Cetiol^{®} 868
   INCI: Ethylhexyl Stearate
   Hersteller: Cognis GmbH
18) Cetiol^{®} A
   INCI: Hexyl Laurate
   Hersteller: Cognis GmbH
19) Cetiol^{®} B
   INCI: Dibutyl Adipate
   Hersteller: Cognis GmbH
20) Cetiol^{®} CC
   INCI: Dicaprylyl Carbonate
   Hersteller: Cognis GmbH
21) Cetiol^{®} J 600
   INCI: Oleyl Erucate
   Hersteller: Cognis GmbH
22) Cetiol^{®} LC
   INCI: Coco-Caprylate/Caprate
   Hersteller: Cognis GmbH
23) Cetiol^{®} OE
   INCI: Dicaprylyl Ether
   Hersteller: Cognis GmbH
24) Cetiol^{®} PGL
   INCI: Hexyldecanol, Hexyldecyl Laurate
   Hersteller: Cognis GmbH
25) Cetiol^{®} S
   INCI: Diethylhexylcyclohexane
   Hersteller: Cognis GmbH
26) Cetiol^{®} SB 45
   INCI: Shea Butter Butyrospermum Parkii (Linne)
   Hersteller: Cognis GmbH
27) Cetiol^{®} SN
   INCI: Cetearyl Isononanoate
   Hersteller: Cognis GmbH
28) Copherol^{®} F 1300 C
   INCI: Tocopherol
   Hersteller: Cognis GmbH
29) Copherol 1250 C
   INCI: Tocopheryl Acetate
   Hersteller: Cognis GmbH
30) Cosmedia^{®} DC
   INCI: Hydrogenated Dimer Dilinoleyl /Dimethylcarbonate Copolymer
   Hersteller: Cognis GmbH
31) Cosmedia^{®} SP
   INCI: Sodium Polyacrylate
   Hersteller: Cognis GmbH
32) Cutina^{®} E 24
   INCI: PEG-20 Glyceryl Stearate
   Hersteller: Cognis GmbH
33) Cutina^{®} HR
   INCI: Hydrogenated Castor Oil
   Hersteller: Cognis GmbH
34) Cutina^{®} MD
   INCI: Glyceryl Stearate
   Hersteller: Cognis GmbH
35) Cuitina^{®} PES
   INCI: Pentaerythrityl Distearate
   Hersteller: Cognis GmbH
36) Dehymuls^{®} FCE
   INCI: Dicocoyl Pentaerythrityl Distearyl Citrate
   Hersteller: Cognis GmbH
37) Dehymuls^{®} HRE 7
   INCI: PEG-7 Hydrogenated Castor Oil Hersteller: Cognis GmbH
38) Dehymuls^{®} PGPH
   INCI: Polyglyceryl-2 Dipolyhydroxystearate
   Hersteller: Cognis GmbH
39) Dow Corning^{®} 244 Fluid
   INCI: Cyclomethicone
   Hersteller: Dow Corning
40) Dow Corning^{®} 246 Fluid
   INCI: Cyclopentasiloxane
   Hersteller: Dow Corning
41) Dow Corning^{®} 2502
   INCI: Cetyl Dimethicone
   Hersteller: Dow Corning
42) Dry^{®}Flo Plus
   INCI: Aluminium Starch Octenylsuccinate
   Hersteller: National Starch
43) Elfacos^{®}ST 37
   INCI: PEG-22 Dodecyl Glycol Copolymer
   Hersteller: Akzo-Nobel
44) Elfacos^{®}ST 9
   INCI: PEG-45 Dodecyl Glycol Copolymer
   Hersteller: Akzo-Nobel
45) Emery^{®} 1780
   INCI: Lanolin Alcohol
   Hersteller: Cognis Corporation (Emery)
46) Emulgade^{®} CM
   INCI: Cetearyl Isononanoate and Ceteareth-20 and Cetearyl Alcohol and Glyceryl Stearate and Glycerin and Ceteareth-12 and Cetyl Palmitate
   Hersteller: Cognis GmbH
47) Emulgade^{®}PL 68/50
   INCI: Cetearyl Glucoside, Cetearyl Alcohol
   Hersteller: Cognis GmbH
48) Emulgade^{®} SE - PF
   INCI: Glyceryl Stearate (and) Ceteareth-20 (and) Ceteareth-12 (and) Cetearyl Alcohol (and) Cetyl Palmitate
   Hersteller: Cognis GmbH
49) Emulgade^{®} SUCRO
   INCI: Sucrose Polystearate (and) Hydrogenated Polyisobutene Hersteller: Cognis GmbH
50) Eumulgin^{®} B1
   INCI: Ceteareth-12
   Hersteller: Cognis Deutschland G mbH
51) Eumulgin^{®} B 2
   INCI: Ceteareth- 20
   Hersteller: Cognis GmbH
52) Eumulgin^{®} HRE 40
   INCI: PEG-40 Hydrogenated Castor Oil
   Hersteller: Cognis GmbH
53) Eumulgin^{®} SG
   INCI: Sodium Stearoyl Glutamate
   Hersteller: Cognis GmbH
54) Eumulgin^{®} VL 75
   INCI: Lauryl Glucoside (and) Polyglyceryl-2 Dipolyhydroxystearate (and) Glycerin
   Hersteller: Cognis GmbH
55) Eusolex^{®} OCR
   INCI: Octocrylene
   Hersteller: Merck
56) Eusolex^{®} T 2000
   INCI: Titanium Dioxide, Alumina, Simethicone
   Hersteller: Merck
57) Eusolex^{®} T AQUA
   INCI: Water and Titanium Dioxide and
      Alumina and Sodium
      Metaphosphate and
      Phenoxyethanol and Sodium
      Methylparaben
   Hersteller: Merck
58) Eutanol^{®} G
   INCI: Octyldodecanol
   Hersteller: Cognis GmbH
59) Eutanol^{®}G 16
   INCI: Hexyldecanol
   Hersteller: Cognis GmbH
60) Eutanol^{®}G 16 S
   INCI: Hexyldecyl Stearate
   Hersteller: Cognis GmbH
61) Finsolv^{®} TN
   INCI: C 12/15 Alkyl Benzoate Hersteller: Findex (Nordmann/Rassmann)
62) Generol^{®} R
   INCI: Brassica Campestris (Rapseed) Sterols
   Hersteller: Cognis GmbH
63) Glucate^{®} DO
   INCI: Methyl Glucose Dioleate
   Hersteller: NRC Nordmann/Rassmann
64) Hispagel^{®} 200
   INCI: Glycerin, Glyceryl Polyacrylate
   Hersteller: Cognis GmbH
65) Hostaphat^{®} KL 340 N
   INCI: Trilaureth-4 Phosphate
   Hersteller: Clariant
66) Hydagen^{®} C.A.T.
   INCI Triethyl Citrate
   Hersteller: Cognis GmbH
67) Hydagen^{®} DCMF
   INCI : Chitosan
   Hersteller: Cognis GmbH
68) Insect Repellent^{®} 3535
   INCI : Ethyl Butylacetylaminopropionate
   Hersteller : EMD Chemicals Inc
69) Isolan^{®} PDI
   INCI: Diisostearoyl Polyglyceryl-3 Diisostearate
   Hersteller: Goldschmidt AG
70) Keltrol^{®} T
   INCI: Xanthan Gum
   Hersteller: CP Kelco
71) Lameform^{®} TGI
   INCI: Polyglyceryl-3 Diisostearate
   Hersteller: Cognis GmbH
72) Lanette^{®} 14
   INCI: Myristyl Alcohol
   Hersteller: Cognis GmbH
73) Lanette 18
   INCI: Stearyl Alcohol
   Hersteller: Cognis GmbH
74) Lanette^{®} 22
   INCI: Behenyl Alcohol
   Hersteller: Cognis GmbH
75) Lanette^{®} E
   INCI: Sodium Cetearyl Sulfate
   Hersteller: Cognis GmbH
76) Lanette^{®} O
   INCI: Cetearyl Alcohol
   Hersteller: Cognis GmbH
77) Locron^{®} L
   INCI: Aluminium Chlorhydrate
   Hersteller: Clariant
78) Lucentite^{®} SAN
   INCI: Quaternium-18 Hectoritr
   Hersteller: Co-Op Chemical Co., Ltd.
79) Monomuls^{®} 90-O 18
   INCI: Glyceryl Oleate
   Hersteller: Cognis GmbH
80) Myrj^{®} 51
   INCI: PEG-30-Sterate
   Hersteller: Uniqema
81) Myritol^{®} 312
   INCI: Caprylic/Capric Triglyceride
   Hersteller: Cognis GmbH
82) Myritol^{®} 331
   INCI: Cocoglycerides
   Hersteller: Cognis GmbH
83) Myritol^{®} PC
   INCI: Propylene Glycol Dicaprylate/Dicaprate
   Hersteller: Cognis GmbH
84) Neo Heliopan^{®} 303
   INCI: Octocrylene
   Hersteller: Symrise
85) Neo Heliopan^{®} AP
   INCI: Disodium Phenyl Dibenzimidazole Tetrasulfonate
   Hersteller: Symrise
86) Neo Heliopa^{®} AV
   INCI: Ethylhexyl Methoxycinnamate
   Hersteller: Symrise
87) Neo Heliopan^{®} BB
   INCI: Benzophenone-3
   Hersteller: Symrise
88) Neo Heliopan^{®} E 1000
   INCI: Isoamyl-p-Methoxycinnamate
   Hersteller: Symrise
88) Neo Heliopan^{®} Hydro
   INCI: Phenylbenzimidazole Sulfonic Acid
   Hersteller: Symrise
89) Neo Heliopan^{®} MBC
   INCI: 4-Methylbenzylidene Camphor
   Hersteller: Symrise
90) Neo Heliopan^{®} OS
   INCI: Ethylhexyl Salicylate
   Hersteller: Symrise
91) Novata^{®} AB
   INCI: Cocoglycerides
   Hersteller: Cognis GmbH
92) Parsol^{®} 1789
   INCI: Butyl Methoxydibenzoylmethane
   Hersteller: Hoffmann-La Roche (Givaudan)
93) Pemulen^{®} TR-2 Polymer
   INCI: Acrylates / C10-30 Alkylacrylate Crosspolymer
   Hersteller: Noveon, Inc.
94) Photonyl^{®} LS
   INCI: Arginine, Disodium Adenosine Triphosphate, Mannitol, Pyridoxine HCL, Phenylalanine, Tyrosine
   Hersteller: Laboratoires Serobiologiques (Cognis)
95) Prisorine^{®} 3505
   INCI: Isostearic Acid
   Hersteller: Uniqema
96) Prisorine^{®} 3758
   INCI: Hydrogenated Polyisobutene
   Hersteller: Uniqema
98) Rezal 36G
   INCI: Aluminum Zirconium Tetrachlorohydrex GLY
   Hersteller: Reheis, Inc
99) SFE^{®} 839
   INCI: Cyclopentasiloxane and
   Dimethicone/Vinyl Dimethicone Crosspolymer
   Hersteller: GE Silicones
100) Silikonöl Wacker AK^{®} 350
   INCI: Dimethicone
   Hersteller: Wacker
101) Tego^{®} Care 450
   INCI: Polyglyceryl-3 Methylglucose Distearate
   Hersteller: Tego Cosmetics (Goldschmidt)
102) Tego^{®} Care CG 90
   INCI: Cetearyl Glucoside
   Hersteller: Goldschmidt
103) Tegosoft^{®} DEC
   INCI: Diethylhexyl Carbonate
   Hersteller: Goldschmidt
104) Tinosorb^{®} S
   INCI: Bis-Ethylhexyloxyphenol Methoxyphenyl Triazine
   Hersteller: Ciba Specialty Chemicals Corporation
105) Tinosorb^{®} M
   INCI: Methylene Bis-Benzotriazolyl Tetramethylbutylphenol
   Herstelller: Ciba Specialty Chemicals Corporation
106) Tween^{®} 60
   INCI: Polysorbate 60
   Hersteller: Uniqema (ICI Surfactants)
107) Uvasorb^{®} HEB
   INCI: Diethylhexyl Butamido Triazone
   Hersteller: 3V Inc.
108) Unirep^{®} U-18
   INCI: Dimethyl Phthalate and Diethyl Toluamide and Ethyl Hexanediol
   Hersteller: Induchem AG
109) Uvinul^{®} T 150
   INCI: Ethylhexyl Triazone
   Hersteller: BASF
110) Uvinul^{®} A plus
   INCI: Diethylamino Hydroxybenzoyl Hexyl Benzoate
   Hersteller: BASF
111) Veegum^{®} Ultra
   INCI: Magnesium Aluminium Silicate
   Hersteller: R. T. Vanderbilt Company, Inc
112) Veegum^{®} Plus
   INCI: Magnesium Aluminum Silicate and Cellulose Gum
   Hersteller: R. T. Vanderbilt Company, Inc
113) Z-Cote^{®} HP 1
   INCI: Zinc Oxide and Triethoxycaprylylsilane
   Hersteller: BASF
114) Zinc Oxide NDM
   INCI: Zinc Oxide
   Hersteler: Symrise

## Patentansprüche

1. Kosmetische und/oder pharmazeutische Zubereitungen, enthaltend 0,1 bis 80 Gew.% eines Kohlenwasserstoffs, ausgewählt aus der Gruppe bestehend aus C7, C9, C11, C13, C15, C17, C19, C21 und C23 Kohlenwasserstoffen.

2. Zubereitungen nach Anspruch 1, **dadurch gekennzeichnet, dass** es sich um lineare Kohlenwasserstoffe handelt.

3. Zubereitungen nach Anspruch 1 und/oder 2, **dadurch gekennzeichnet, dass** es sich um gesättigte Kohlenwasserstoffe handelt.

4. Zuberereitungen nach mindestens einem der Ansprüche 1 bis 3, **dadurch gekennzeichnet, dass** die Kohlenwasserstoffe ausgewählt sind aus der Gruppe bestehend aus n-Heptan, n-Nonan, n-Undecan, n-Tridecan, n-Pentadecan, n-Heptadecan, n-Nonadecan, n-Henicosan und n-Tricosan.

5. Kosmetische und/oder pharmazeutische Zubereitung nach mindestens einem der Ansprüche 1 bis 4, enthaltend mindestens **einen Antiperspirant / Desodorant Wirkstoff.**

6. Kosmetische und/oder pharmazeutische Zubereitung nach nach mindestens einem der Ansprüche 1 bis 5, enthaltend **mindestens einen UV-Lichtschutzfilter.**

7. Kosmetische und/oder pharmazeutische Zubereitungen nach mindestens einem der Ansprüche 1 bis 6, enthaltend mindestens einen **Selbstbräuner.**

8. Kosmetische und/oder pharmazeutische Zubereitungen nach mindestens einem der Ansprüche 1 bis 7, enthaltend mindestens **ein Pigment und/oder Farbstoff.**

9. Kosmetische und/oder pharmazeutische Zubereitungen nach einem mindestens einem der Ansprüche 1 bis 8, enthaltend mindestens einen **Emulgator und/oder ein Tensid und/oder eine Wachskomponente und/oder ein Polymer und/oder einen weiteren Ölkörper.**

10. Verwendung von Kohlenwasserstoffen, ausgewählt aus der Gruppe bestehend aus bestehend aus C7, C9, C11, C13, C15, C17, C19, C21 und C23 Kohlenwasserstoffen in kosmetischen und/oder pharmazeutischen Zubereitungen, insbesondere als Ölkörper und/oder Dispergiermittel.
